# EUROPEAN PATENT APPLICATION

(11) **EP 3 922 627 A1**
(43) Date of publication of application: **15.12.2021**
(21) Application number: 20752612.0
(22) Date of filing: 21.01.2020
(51) Int. Cl.: C07C 65/26, C07C 69/92, C07C 229/52, C07D 333/76, C07F 5/02, C09K 11/06, C07C 49/697, C07D 209/86, H01L 51/50

(54) **COMPOUND AND LIGHT EMITTING ELEMENT USING SAME**

(30) Priority: 08.02.2019 JP 2019022156
(71) Applicant: Sumitomo Chemical Company, Ltd., Chuo-ku Tokyo 104-8260 (JP)
(72) Inventor: KOBAYASHI, Satoshi, Tsukuba-shi, Ibaraki 300-3294 (JP); TANAKA, Masanobu, Tsukuba-shi, Ibaraki 300-3294 (JP); OHUCHI, Kazuei, Tsukuba-shi, Ibaraki 300-3294 (JP); ISHIKAWA, Rui, Tsukuba-shi, Ibaraki 300-3294 (JP); USUI, Motoaki, Tsukuba-shi, Ibaraki 300-3294 (JP); SATO, Takahiro, Tsukuba-shi, Ibaraki 300-3294 (JP)
(74) Representative: J A Kemp LLP
(86) International application number: PCT/JP2020/001828
(87) International publication number: WO 2020/162156

(57) **Abstract**

To provide a compound with which the luminance life of a light emitting device is excellent.

A compound represented by the formula (1): 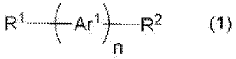 [wherein,
n represents an integer of 2 or more and 20 or less.
R¹ and R² each independently represent a hydrogen atom, an alkyl group, a cycloalkyl group, an alkoxy group, a cycloalkoxy group or a halogen atom.
Ar¹ represents a mono-cyclic or condensed-cyclic arylene group, a mono-cyclic or condensed-cyclic divalent heterocyclic group or a group represented by - N(R^{X1})-. R^{X1} represents a hydrogen atom, an alkyl group, a cycloalkyl group, an aryl group or a monovalent heterocyclic group.

At least two of Ar¹ represent prescribed groups.].

## Description

### Technical Field

The present invention relates to a compound or a light emitting device using the same.

### Background Art

Organic electroluminescent devices (hereinafter, referred to also as "light emitting device") can be suitably used for applications of display and illumination because of high external quantum yield and low driving voltage. The light emitting device usually has organic layers such as a light emitting layer, an electron injection layer, an electron transporting layer, a charge transporting layer and the like.
Hence, studies on compounds that improve the properties of the light emitting device, as the compound to be used in each of these layers, are underway (Patent Documents 1 to 4).

For example, Patent Document 1 describes a compound having a structure in which three fluorenes having a substituent only at the 9-position are bound. Further, Patent Document 2 describes a compound in which two fluorenes having substituents only at the 2-position and the 9-position are bonded to a fluorene having a substituent only at the 9-position. Additionally, Patent Documents 3 and 4 describe polymer compounds containing in the constitutional unit a divalent group obtained by removing two hydrogen atoms at the 2-position and the 7-position from a fluorene having a substituent only at the 9-position.

### [Prior Art Document]

### [Patent Document]

Patent Document 1: International Publication WO2017/103610
Patent Document 2: Japanese Unexamined Patent Application Publication (JP-A) No. 2012-33845
Patent Document 3: International Publication WO2013/058160
Patent Document 4: International Publication WO2015/159932

### Summary of the Invention

### Problem to be Solved by the Invention

However, when these compounds and polymer compounds are used for fabrication of a light emitting device, the luminance life of the resulting light emitting device is not necessarily sufficient.

Then, the present invention has an object of providing a compound which gives a light emitting device excellent in luminance life.

### Means for Solving the Problem

The present invention provides the following articles.
[Article 1] A compound represented by the formula (1) : 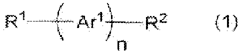 [wherein,
n represents an integer of 2 or more and 20 or less.
R¹ and R² each independently represent a hydrogen atom, an alkyl group, a cycloalkyl group, an alkoxy group, a cycloalkoxy group or a halogen atom, and these groups optionally have a substituent.
Ar¹ represents a mono-cyclic or condensed-cyclic arylene group, a mono-cyclic or condensed-cyclic divalent heterocyclic group or a group represented by - N(R^{X1})-, and these groups optionally have a substituent. A plurality of Ar¹ may be the same or different. R^{X1} represents a hydrogen atom, an alkyl group, a cycloalkyl group, an aryl group or a monovalent heterocyclic group, and these groups optionally have a substituent.

At least two of Ar¹ represent groups represented by the formula (2). A plurality of the groups represented by the formula (2) may be the same or different.] 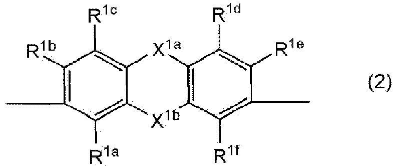 [wherein,
X^{1a} and X^{1b} each independently represent a single bond, an oxygen atom, a sulfur atom, a group represented by -S(=O)-, a group represented by -S(=O)₂-, a group represented by -C(=O)-, a group represented by -C(R^{1g})₂-, a group represented by -Si(R^{1g})₂-, a group represented by -NR^{1g}- or a group represented by C(R^{1g})₂-C(R^{1g})₂-. At least one of X^{1a} and X^{1b} represents a group represented by -C(R^{1g})₂- or a group represented by -NR^{1g}-.
R^{1g} represents a hydrogen atom, an alkyl group, a cycloalkyl group, an aryl group or a monovalent heterocyclic group, and these groups optionally have a substituent. When a plurality of R^{1g} are present, they may be the same or different and may be combined together to form a ring. When a plurality of R^{1g} are present and these are combined together to form a ring, they typically form a ring together with atoms to which these groups are attached.
R^{1a}, R^{1b}, R^{1c}, R^{1d}, R^{1e} and R^{1f} each independently represent a hydrogen atom, an alkyl group, a cycloalkyl group, an alkoxy group, a cycloalkoxy group, an aryl group, an aryloxy group, a monovalent heterocyclic group, an amino group or a halogen atom, and these groups optionally have a substituent. R^{1a} and R^{1g}, R^{1b} and R^{1c}, R^{1c} and R^{1g}, R^{1g} and R^{1d}, R^{1d} and R^{1e}, and R^{1f} and R^{1g} each may be combined together to form a ring together with atoms to which they are attached.

In at least two groups represented by the above-described formula (2) in the above-described formula (1), at least one of R^{1g} is a group represented by the formula (2-1) or a group represented by the formula (2-2) .

In the above-described formula (1), if all Ar¹ are groups represented by the above-described formula (2) and all X^{1a} in all the groups represented by the formula (2) are single bonds and all X^{1b} are groups represented by -C(R^{1g})₂-, then, at least one of R^{1a}, R^{1b}, R^{1e} and R^{1f} represents at least one group selected from the group consisting of an alkyl group, a cycloalkyl group, an alkoxy group, a cycloalkoxy group, an aryl group, an aryloxy group, a monovalent heterocyclic group, an amino group and a halogen atom (the alkyl group, the cycloalkyl group, the alkoxy group, the cycloalkoxy group, the aryl group, the aryloxy group, the monovalent heterocyclic group or the amino group optionally has a substituent), in at least one of all the groups represented by the formula (2).]

―R³―{(Q¹)ₙ₁-Y¹(M¹)ₐ₁(Z¹)_{b1}}ₘ₂ (2-1)

[wherein,
R³ represents an aromatic hydrocarbon group or a heterocyclic group, and these groups optionally have a substituent.
n1, a1 and b1 each independently represent an integer of 0 or more, m2 represents an integer of 1 or more, and a1 and b1 are selected so that the charge of the group represented by the above-described formula (2-1) is 0. When a plurality of n1, a1 and b1 are present, they may be the same or different at each occurrence.
Q¹ represents an alkylene group, a cycloalkylene group, an arylene group, an oxygen atom or a sulfur atom, and these groups optionally have a substituent. When a plurality of Q¹ are present, they may be the same or different.
Y¹ represents -CO₂⁻, -SO₃⁻, -SO₂⁻, -PO₃²⁻, -CO₂Y¹', -SO₃Y¹', -SO₂Y¹', -P(=O) (-OY¹')(-O⁻) or -P(=O) (-OY¹')₂. When a plurality of Y¹ are present, they may be the same or different. Y¹' represents a hydrocarbon group optionally having a substituent, a heterocyclic group optionally having a substituent, or a hydrogen atom. When Y¹ is -CO₂Y¹', -SO₃Y¹', -SO₂Y¹' or -P(=O)(-OY¹')₂, the subscript a1 for M¹ directly bonded to the Y¹ is 0 and the subscript b1 for Z¹ directly bonded to the M¹ is 0. When Y¹ is -CO₂⁻, -SO3⁻, -SO₂⁻, -PO₃²⁻ or - P(=O)(-OY¹')(-O⁻), the subscript a1 for M¹ directly bonded to the Y¹ is an integer of 1 or more. When a plurality of Y¹' are present, they may be the same or different.
M¹ represents an alkali metal cation, an alkaline earth metal cation or an ammonium cation, and this ammonium cation optionally has substituent. When a plurality of M¹ are present, they may be the same or different.
Z¹ represents F⁻, Cl⁻, Br⁻, I⁻, OH⁻, B (R^{a})₄⁻, R^{a}SO₃⁻, R^{a}COO⁻, NO₃⁻, SO₄²⁻, HSO₄⁻, PO₄³⁻, HPO₄²⁻, H₂PO₄⁻, BF₄⁻ or PF₆⁻. R^{a} represents an alkyl group, a cycloalkyl group or an aryl group, and these groups optionally have a substituent. When a plurality of Z¹ are present, they may be the same or different.]

   — R⁴—{(Q²)ₙ₂-Y²(M²)ₐ₂(Z²)_{b2}}m₃ (2-2)

   [wherein,
   n2 and b2 each independently represent an integer of 0 or more and a2 and m3 each independently represent an integer of 1 or more, but a2 and b2 are selected so that the charge of the group represented by the above-described formula (2-2) is 0. When a plurality of n2, a2 and b2 are present, they may be the same or different at each occurrence.
   R⁴ represents an aromatic hydrocarbon group or a heterocyclic group, and these groups optionally have a substituent.
   Q² represents an alkylene group, a cycloalkylene group, an arylene group, an oxygen atom or a sulfur atom, and these groups optionally have a substituent. When a plurality of Q² are present, they may be the same or different.
   Y² represents -C⁺R^{c}₂, -N⁺R^{c}₃, -P⁺R^{c}₃, -S⁺R^{c}₂ or - I⁺R^{C}₂. R^{c} represents an alkyl group, a cycloalkyl group or an aryl group, and these groups optionally have a substituent. A plurality of R^{c} may be the same or different. When a plurality of Y² are present, they may be the same or different.
   M² represents F⁻, Cl⁻, Br⁻, I⁻, OH⁻, B (R^{b})₄⁻, R^{b}SO₃⁻ R^{b}COO⁻, BF₄⁻, SbCl₆⁻ or SbF₆⁻. R^{b} represents an alkyl group, a cycloalkyl group or an aryl group, and these groups optionally have a substituent. When a plurality of R^{b} are present, they may be the same or different. When a plurality of M² are present, they may be the same or different.
   Z² represents an alkali metal cation or an alkaline earth metal cation. When a plurality of Z² are present, they may be the same or different.]

[Article 2] The compound according to Article 1, wherein at least one of the above-described Ar¹ is a mono-cyclic or condensed-cyclic arylene group other than a group represented by the above-described formula (2), a mono-cyclic or condensed-cyclic divalent heterocyclic group other than a group represented by the above-described formula (2) or a group represented by -N(R^{X1})- (R^{X1} represents the same meaning as described above).

[Article 3] The compound according to Article 1 or 2, wherein the compound represented by the above-described formula (1) is a compound represented by the formula (1-1): 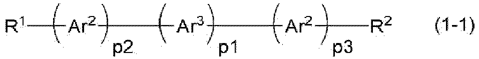 [wherein,
p1 represents an integer of 2 or more. p2 and p3 each independently represent an integer of 1 or more. The sum of p1, p2 and p3 is 4 or more and 20 or less.
Ar² represents a mono-cyclic or condensed-cyclic arylene group other than a group represented by the above-described formula (2) or a mono-cyclic or condensed-cyclic divalent heterocyclic group other than a group represented by the above-described formula (2), and these groups optionally have a substituent. A plurality of Ar² may be the same or different.
Ar³ represents a mono-cyclic or condensed-cyclic arylene group, a mono-cyclic or condensed-cyclic divalent heterocyclic group or a group represented by - N(R^{X1})-, and these groups optionally have a substituent. A plurality of Ar³ may be the same or different.

At least two of Ar³ are groups represented by the above-described formula (2).
R¹, R² and R^{X1} represent the same meaning as described above.].

[Article 4] The compound according to Article 3, wherein the compound represented by the above-described formula (1-1) is a compound represented by the formula (1A): 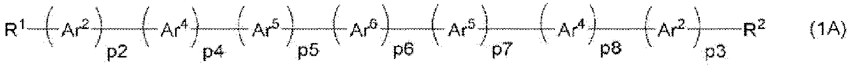 [wherein,
Ar², p2 and p3 represent the same meaning as described above.
p4 and p8 each independently represent an integer of 1 or more. p5, p6 and p7 each independently represent an integer of 0 or more. The sum of p2, p3, p4, p5, p6, p7 and p8 is an integer of 4 or more and 20 or less.
Ar⁴ represents a group represented by the formula (2A). A plurality of Ar⁴ may be the same or different.
Ar⁶ represents a group represented by the above-described formula (2). When a plurality of Ar⁶ are present, they may be the same or different.
Ar⁵ represents a mono-cyclic or condensed-cyclic arylene group other than a group represented by the above-described formula (2), a mono-cyclic or condensed-cyclic divalent heterocyclic group other than a group represented by the above-described formula (2) or a group represented by -N(R^{X1})-, and these groups optionally have a substituent. When a plurality of Ar⁶ are present, they may be the same or different. R¹, R² and R^{X1} represent the same meaning as described above. 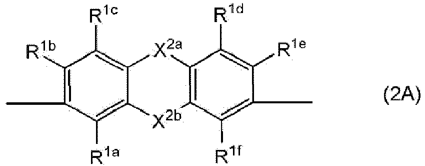
[wherein,
X^{2a} and X^{2b} each independently represent a single bond, a group represented by -C(R^{1g})₂- or a group represented by -NR^{1g}-. One of X^{2a} and X^{2b} is a single bond and the other of X^{2a} and X^{2b} is a group represented by -C(R^{1g})₂- or a group represented by - NR^{1g}-.
R^{1a}, R^{1b}, R^{1c}, R^{1d}, R^{1e}, R^{1f} and R^{1g} represent the same meaning as described above.]].

[Article 5] The compound according to Article 4, wherein p6 is an integer of 1 or more, and in at least one Ar⁶, one of X^{1a} and X^{1b} in the above-described formula (2) is a single bond.

[Article 6] The compound according to any one of Articles 1 to 5, wherein at least two groups represented by the above-described formula (2) are groups represented by the formula (2A): 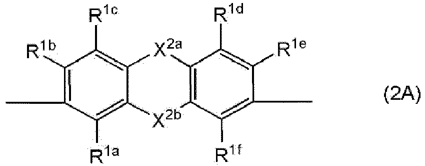 [wherein,
X^{2a} and X^{2b} each independently represent a single bond, a group represented by -C(R^{1g})₂- or a group represented by -NR^{1g}-. One of X^{2a} and X^{2b} is a single bond and the other of X^{2a} and X^{2b} is a group represented by -C(R^{1g})₂- or a group represented by - NR^{1g}-.
R^{1a}, R^{1b}, R^{1c}, R^{1d}, R^{1e}, R^{1f} and R^{1g} represent the same meaning as described above.].

[Article 7] The compound according to Article 6, wherein at least two groups represented by the above-described formula (2A) are groups represented by the formula (2A'): 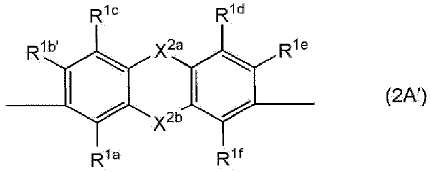 [wherein,
R^{1a}, R^{1c}, R^{1d}, R^{1e}, R^{1f}, X^{2a} and X^{2b} represent the same meaning as described above.
R^{1b}' represents an alkyl group, a cycloalkyl group, an alkoxy group, a cycloalkoxy group, an aryl group, an aryloxy group, a monovalent heterocyclic group, an amino group or a halogen atom, and these groups optionally have a substituent. R^{1b}' and R^{1c} may be combined together to form a ring together with carbon atoms to which they are attached.].

[Article 8] The compound according to Article 6 or 7, wherein at least two of X^{2a} are single bonds.

[Article 9] The compound according to any one of Articles 1 to 8, wherein at least one of R^{1g} is a group represented by the above-described formula (2-1).

[Article 10] The compound according to Article 9, wherein the group represented by the above-described formula (2-1) is a group represented by the formula (2-3): 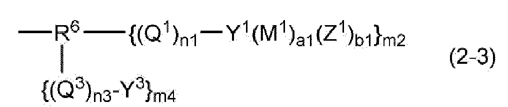 [wherein,
n1, a1, b1, m2, Q¹, Y¹, M¹ and Z¹ represent the same meaning as described above.
n3 represents an integer of 0 or more, and m4 represents an integer of 1 or more. When a plurality of n3 are present, they may be the same or different.
R⁶ represents an aromatic hydrocarbon group or a heterocyclic group, and these groups optionally have a substituent.
Q³ represents an alkylene group, a cycloalkylene group, an arylene group, an oxygen atom or a sulfur atom, and these groups optionally have a substituent. When a plurality of Q³ are present, they may be the same or different.
Y³ represents a group represented by the formula (5) or the formula (6). When a plurality of Y³ are present, they may be the same or different.]

   ―O― (R'O)ₐ₃-R" (5) 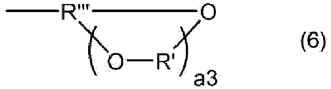
[wherein,
a3 represents an integer of 1 or more.
R' represents an alkylene group, a cycloalkylene group or an arylene group, and these groups optionally have a substituent. When a plurality of R' are present, they may be the same or different.
R" represents a hydrogen atom, an alkyl group, a cycloalkyl group or an aryl group, and these groups optionally have a substituent.
R‴ represents a hydrocarbon group, and this hydrocarbon group optionally has a substituent.].

[Article 11] The compound according to any one of Articles 1 to 10, wherein at least one of Ar¹ is a mono-cyclic or condensed-cyclic arylene group other than a group represented by the above-described formula (2) or a mono-cyclic or condensed-cyclic divalent heterocyclic group other than a group represented by the above-described formula (2), which is a group obtained by removing 2 hydrogen atoms constituting the ring from a benzene ring or an aromatic hydrocarbon ring in which only 2 or more and 10 or less benzene rings are condensed (the group optionally has a substituent) or a group represented by the formula (4): 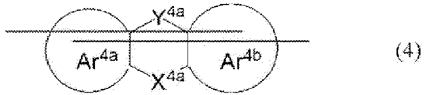 [wherein,
Ar^{4a} and Ar^{4b} each independently represent an aromatic hydrocarbon group or a heterocyclic group, and these groups optionally have a substituent. When a plurality of the above-described substituents are present, they may be combined together to form a ring together with atoms to which they are attached.
X^{4a} and Y^{4a} each independently represent a single bond, an oxygen atom, a sulfur atom, a group represented by -S(=O)-, a group represented by -S(=O)₂-, a group represented by -C(=O)-, a group represented by -SiR₂- or a group represented by -CR₂-CR₂-. R represents a hydrogen atom, an alkyl group, a cycloalkyl group, an aryl group or a monovalent heterocyclic group, and these groups optionally have a substituent. A plurality of R may be the same or different and may be combined together to form a ring. When a plurality of R are combined together to form a ring, they typically form a ring together with atoms to which they are attached.

The substituent which Ar^{4a} optionally has and R, and the substituent which Ar^{4b} optionally has and R each may be combined together to form a ring together with atoms to which they are attached.].

[Article 12] The compound according to Article 11, wherein the group represented by the above-described formula (4) is a group represented by the formula (4A): 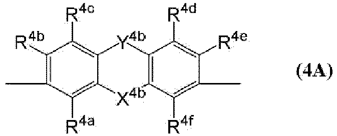 [wherein,
X^{4b} and Y^{4b} each independently represent a single bond, an oxygen atom, a sulfur atom or a group represented by -CR₂-CR₂-. One of X^{4b} and Y^{4b} represents a single bond. R represents the same meaning as described above.
R^{4a}, R^{4b}, R^{4c}, R^{4d}, R^{4e} and R^{4f} each independently represent a hydrogen atom, an alkyl group, a cycloalkyl group, an alkoxy group, a cycloalkoxy group, an aryl group, an aryloxy group, a monovalent heterocyclic group, an amino group or a halogen atom, and these groups optionally have a substituent. R^{4b} and R^{4c}, R^{4c} and R, R^{4d} and R, R^{4a} and R, R^{4f} and R, and R^{4d} and R^{4e} each may be combined together to form a ring together with carbon atoms to which they are attached.].

[Article 13] The compound according to any one of Articles 1 to 12, wherein in the above-described formula (1), Ar¹ is composed only of groups selected from the group consisting of a group represented by the above-described formula (2), a group obtained by removing 2 hydrogen atoms constituting the ring from a benzene ring or an aromatic hydrocarbon ring in which only 2 or more and 10 or less benzene rings are condensed (the group optionally has a substituent), a group represented by the formula (4), and a group represented by -N(R^{X1})- (R^{X1} represents the same meaning as described above): 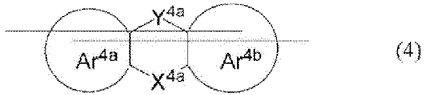 [wherein,
Ar^{4a} and Ar^{4b} each independently represent an aromatic hydrocarbon group or a heterocyclic group, and these groups optionally have a substituent. When a plurality of the above-described substituents are present, they may be combined together to form a ring together with atoms to which they are attached.
X^{4a} and Y^{4a} each independently represent a single bond, an oxygen atom, a sulfur atom, a group represented by -S(=O)-, a group represented by -S(=O)₂-, a group represented by -C(=O)-, a group represented by -SiR₂- or a group represented by -CR₂-CR₂-.
R represents a hydrogen atom, an alkyl group, a cycloalkyl group, an aryl group or a monovalent heterocyclic group, and these groups optionally have a substituent. A plurality of R may be the same or different and may be combined together to form a ring. When a plurality of R are combined together to form a ring, they typically form a ring together with atoms to whichy they are attached.

The substituent which Ar^{4a} optionally has and R, and the substituent which Ar^{4b} optionally has and R each may be combined together to form a ring together with atoms to which they are attached.].

[Article 14] A composition comprising at least one compound selected from the group consisting of a hole transporting material, a hole injection material, an electron transporting material, an electron injection material, a light emitting material, an antioxidant and a solvent and the compound according to any one of Articles 1 to 13.

[Article 15] A light emitting device comprising the compound according to any one of Articles 1 to 13.

[Article 16] A compound represented by the formula (11), the formula (12) or the formula (13): 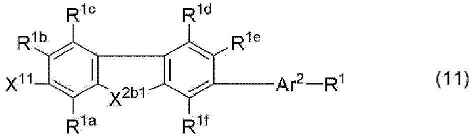 [wherein,
R^{1a}, R^{1b}, R^{1c}, R^{1d}, R^{1e} and R^{1f} each independently represent a hydrogen atom, an alkyl group, a cycloalkyl group, an alkoxy group, a cycloalkoxy group, an aryl group, an aryloxy group, a monovalent heterocyclic group, an amino group or a halogen atom, and these groups optionally have a substituent. R^{1a} and R^{1g}, R^{1b} and R^{1c}, R^{1d} and R^{1e}, and R^{1f} and R^{1g} each may be combined together to form a ring structure together with atoms to which they are attached.
X^{2b1} represents a group represented by -C(R^{1g})₂- or a group represented by -NR^{1g}-.
R^{1g} represents a hydrogen atom, an alkyl group, a cycloalkyl group, an aryl group or a monovalent heterocyclic group, and these groups optionally have a substituent. When a plurality of R^{1g} are present, they may be the same or different and may be combined together to form a ring (When a plurality of R^{1g} are present and these are combined together to form a ring, they typically form a ring together with atoms to which these groups are attached.). At least one of R^{1g} is a group represented by the formula (2-1) or a group represented by the formula (2-2).
X¹¹ represents a halogen atom or B(OR^{C2})₂ (wherein, R^{C2} represents a hydrogen atom, an alkyl group, a cycloalkyl group or an aryl group, and these groups optionally have a substituent. A plurality of R^{C2} may be the same or different and may be combined together to form a ring structure together with oxygen atoms to which they are attached.).
R¹ represents a hydrogen atom, an alkyl group, a cycloalkyl group, an alkoxy group, a cycloalkoxy group or a halogen atom, and these groups optionally have a substituent.
Ar² represents a mono-cyclic or condensed-cyclic arylene group other than the group represented by the formula (2) or a mono-cyclic or condensed-cyclic divalent heterocyclic group other than the group represented by the formula (2), and these groups optionally have a substituent.]

   **—** R³**—** {(Q¹)ₙ₁-Y¹(M¹)ₐ₁(Z¹)_{b1}}ₘ₂ (2-1)
[wherein,
R³ represents an aromatic hydrocarbon group or a heterocyclic group, and these groups optionally have a substituent.
n1, a1 and b1 each independently represent an integer of 0 or more, and m2 represents an integer of 1 or more, but a1 and b1 are selected so that the charge of the group represented by the above-described formula (2-1) is 0. When a plurality of n1, a1 and b1 are present, they may be the same or different at each occurrence.
Q¹ represents an alkylene group, a cycloalkylene group, an arylene group, an oxygen atom or a sulfur atom, and these groups optionally have a substituent. When a plurality of Q¹ are present, they may be the same or different.
Y¹ represents -CO₂⁻, -SO3⁻, -SO₂⁻, -PO₃²⁻, -CO₂Y¹', -SO₃Y¹', -SO₂Y¹', -P(=O)(-OY¹')(-O⁻) or -P(=O)(-OY¹')₂. When a plurality of Y¹ are present, they may be the same or different. Y¹' represents a hydrocarbon group optionally having a substituent, a heterocyclic group optionally having a substituent, or a hydrogen atom. When Y¹ is -CO₂Y¹', -SO₃Y¹', -SO₂Y¹' or -P(=O) (-OY¹')₂, the subscript a1 for M¹ directly bonded to the Y¹ is 0 and the subscript b1 for Z¹ directly bonded to the M¹ is 0. When Y¹ is -CO₂⁻, -SO3⁻, -SO₂⁻, -PO₃²⁻ or - P(=O)(-OY¹)(-O⁻), the subscript a1 for M¹ directly bonded to the Y¹ is an integer of 1 or more.

When a plurality of Y¹' are present, they may be the same or different.
M¹ represents an alkali metal cation, an alkaline earth metal cation or an ammonium cation, and this ammonium cation optionally has substituent. When a plurality of M¹ are present, they may be the same or different.
Z¹ represents F⁻, Cl⁻, Br⁻, I⁻, OH⁻, B (R^{a})₄⁻, R^{a}SO₃⁻ R^{a}COO⁻, NO₃⁻, SO₄²⁻, HSO₄⁻, PO₄³, HPO₄²⁻, H₂PO₄⁻, BF₄⁻ or PF₆⁻. R^{a} represents an alkyl group, a cycloalkyl group or an aryl group, and these groups optionally have a substituent. When a plurality of Z¹ are present, they may be the same or different.]

   ―R⁴―{(Q²)ₙ₂-Y²(M²)ₐ₂(Z²)_{b2}}ₘ₃ (2-2)

   [wherein,
   n2 and b2 each independently represent an integer of 0 or more, and a2 and m3 each independently represent an integer of 1 or more, but a2 and b2 are selected so that the charge of the group represented by the above-described formula (2-2) is 0. When a plurality of n2, a2 and b2 are present, they may be the same or different at each occurrence.
   R⁴ represents an aromatic hydrocarbon group or a heterocyclic group, and these groups optionally have a substituent.
   Q² represents an alkylene group, a cycloalkylene group, an arylene group, an oxygen atom or a sulfur atom, and these groups optionally have a substituent. When a plurality of Q² are present, they may be the same or different.
   Y² represents -C⁺R^{c}₂, -N⁺R^{c}₃, -P⁺R^{c}₃, -S⁺R^{c}₂ or - I⁺R^{c}₂. R^{c} represents an alkyl group, a cycloalkyl group or an aryl group, and these groups optionally have a substituent. A plurality of R^{c} may be the same or different. When a plurality of Y² are present, they may be the same or different.
   M² represents F⁻, Cl⁻, Br⁻, I⁻, OH⁻, B (R^{b})₄⁻, R^{b}SO₃⁻, R^{b}COO⁻, BF₄⁻, SbCl₆⁻ or SbF₆⁻. R^{b} represents an alkyl group, a cycloalkyl group or an aryl group, and these groups optionally have a substituent. When a plurality of R^{b} are present, they may be the same or different. When a plurality of M² are present, they may be the same or different.
   Z² represents an alkali metal cation or an alkaline earth metal cation. When a plurality of Z² are present, they may be the same or different.] 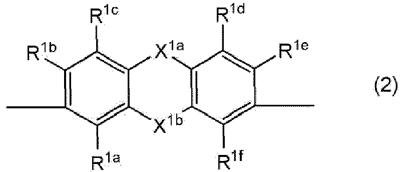
[wherein,
X^{1a} and X^{1b} each independently represent a single bond, an oxygen atom, a sulfur atom, a group represented by -S(=O)-, a group represented by -S(=O)₂-, a group represented by -C(=O)-, a group represented by -C(R^{1g})₂-, a group represented by -Si(R^{1g})₂-, a group represented by -NR^{1g}- or a group represented by C(R^{1g})₂-C(R^{1g})₂-. At least one of X^{1a} and X^{1b} represents a group represented by -C(R^{1g})₂- or a group represented by -NR^{1g}-.
R^{1a}, R^{1b}, R^{1c}, R^{1d}, R^{1e}, R^{1f} and R^{1g} represent the same meaning as described above.] 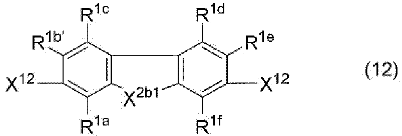
[wherein,
R^{1a}, R^{1c}, R^{1d}, R^{1e}, R^{1f} and X^{2b1} represent the same meaning as described above.
R^{1b}' represents an alkyl group, a cycloalkyl group, an alkoxy group, a cycloalkoxy group, an aryl group, an aryloxy group, a monovalent heterocyclic group, an amino group or a halogen atom, and these groups optionally have a substituent. R^{1b}' and R^{1c} each may be combined together to form a ring structure together with atoms to which they are attached.
X¹² represents a halogen atom or a group represented by B(OR^{C2})₂ (wherein, R^{C2} represents the same meaning as described above.). A plurality of X¹² may be the same or different.] 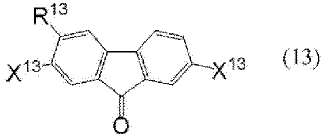
[wherein,
R¹³ represents an alkyl group, a cycloalkyl group, an aryl group or a monovalent heterocyclic group, and these groups optionally have a substituent.
X¹³ represents a chlorine atom, a bromine atom, an iodine atom, or a group represented by B(OR^{C2})₂ (wherein, R^{C2} represents the same meaning as described above.). A plurality of X¹³ may be the same or different.].

### Effect of the Invention

The present invention can provide a compound which gives a light emitting device excellent in luminance life. In addition, according to the present invention, it is possible to provide a composition comprising the compound and a light emitting device comprising the same. Further, the present invention can provide an intermediate compound which is useful for producing the compound.

### Modes for Carrying Out the Invention

Suitable embodiments of the present invention will be illustrated in detail below.

### <Explanation of common terms>

Terms commonly used in the present specification have the following meanings unless otherwise stated.

Me represents a methyl group, Et represents an ethyl group, Bu represents a butyl group, i-Pr represents an isopropyl group and t-Bu represents a tert-butyl group.

A hydrogen atom may be a heavy hydrogen atom or a light hydrogen atom.

In the formula representing a metal complex, the solid line representing a bond with the central metal means a covalent bond or a coordination bond.

"The polymer compound" means a polymer having molecular weight distribution and having a polystyrene-equivalent number-average molecular weight of 1×10³ to 1×10⁸.

The polymer compound may be any of a block copolymer, a random copolymer, an alternating copolymer and a graft copolymer, and may also be another form.

The end group of the polymer compound is preferably a stable group since if a polymerization active group remains intact there, there is a possibility of a decrease in a light emitting property or luminance life when the polymer compound is used for fabrication of a light emitting device. The end group is preferably a group conjugatively bonded to the main chain and includes, for example, groups bonding to an aryl group or a monovalent heterocyclic group via a carbon-carbon bond.

"The low molecular compound" means a compound having no molecular weight distribution and having a molecular weight of 1×10⁴ or less.

"The constitutional unit" means a unit occurring once or more times in the polymer compound.

"The alkyl group" may be any of linear and branched. The number of carbon atoms of the linear alkyl group, not including the number of carbon atoms of the substituent, is usually 1 to 50, preferably 1 to 30, and more preferably 1 to 20. The number of carbon atoms of the branched alkyl group, not including the number of carbon atoms of the substituent, is usually 3 to 50, preferably 3 to 30, and more preferably 4 to 20.

The alkyl group optionally has a substituent and examples thereof include a methyl group, an ethyl group, a propyl group, an isopropyl group, a butyl group, a 2-butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group, and groups obtained by substituting a hydrogen atom in these groups with a cycloalkyl group, an alkoxy group, a cycloalkoxy group, an aryl group, a fluorine atom and the like (for example, a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-hexylphenyl)propyl group and a 6-ethyloxyhexyl group).

The number of carbon atoms of "the cycloalkyl group", not including the number of carbon atoms of the substituent, is usually 3 to 50, preferably 3 to 30, and more preferably 4 to 20.

The cycloalkyl group optionally has a substituent and examples thereof include a cyclohexyl group, a methylcyclohexyl group and an ethylcyclohexyl group.

"The aryl group" means an atomic group remaining after removing from an aromatic hydrocarbon one hydrogen atom bonding directly to a carbon atom constituting the ring. The number of carbon atoms of the aryl group, not including the number of carbon atoms of the substituent, is usually 6 to 60, preferably 6 to 30, and more preferably 6 to 18.

The aryl group optionally has a substituent and examples thereof include a phenyl group, a 1-naphthyl group, a 2-naphthyl group, a 1-anthracenyl group, a 2-anthracenyl group, a 9-anthracenyl group, a 1-pyrenyl group, a 2-pyrenyl group, a 4-pyrenyl group, a 2-fluorenyl group, a 3-fluorenyl group, a 4-fluorenyl group, a 2-phenylphenyl group, a 3-phenylphenyl group and a 4-phenylphenyl group, and groups obtained by substituting a hydrogen atom in these groups with an alkyl group, a cycloalkyl group, an alkoxy group, a cycloalkoxy group, an aryl group, a fluorine atom and the like.

"The alkoxy group" may be any of linear and branched. The number of carbon atoms of the linear alkoxy group, not including the number of carbon atoms of the substituent, is usually 1 to 40, and preferably 4 to 10. The number of carbon atoms of the branched alkoxy group, not including the number of carbon atoms of the substituent, is usually 3 to 40, and preferably 4 to 10.

The alkoxy group optionally has a substituent and examples thereof include a methoxy group, an ethoxy group, a propyloxy group, an isopropyloxy group, a butyloxy group, an isobutyloxy group, a tert-butyloxy group, a pentyloxy group, a hexyloxy group, a heptyloxy group, an octyloxy group, a 2-ethylhexyloxy group, a nonyloxy group, a decyloxy group, a 3,7-dimethyloctyloxy group and a lauryloxy group, and groups obtained by substituting a hydrogen atom in these groups with a cycloalkyl group, an alkoxy group, a cycloalkoxy group, an aryl group, a fluorine atom or the like.

The number of carbon atoms of "the cycloalkoxy group", not including the number of carbon atoms of the substituent, is usually 3 to 40, and preferably 4 to 10.

The cycloalkoxy group optionally has a substituent and examples thereof include a cyclohexyloxy group.

The number of carbon atoms of the "the aryloxy group", not including the number of carbon atoms of the substituent, is usually 6 to 60, and preferably 6 to 48.

The aryloxy group optionally has a substituent and examples thereof include a phenoxy group, a 1-naphthyloxy group, a 2-naphthyloxy group, a 1-anthracenyloxy group, a 9-anthracenyloxy group and a 1-pyrenyloxy group, and groups obtained by substituting a hydrogen atom in these groups with an alkyl group, a cycloalkyl group, an alkoxy group, a cycloalkoxy group, a fluorine atom or the like.

"The heterocyclic group" means an atomic group remaining after removing one or more hydrogen atoms bonding directly to carbon atoms or hetero atoms constituting the ring of a heterocylic compound. The heterocyclic group optionally has a substituent, and includes groups obtained by substituting a hydrogen atom in the heterocyclic compound with an alkyl group, a cycloalkyl group, an alkoxy group, a cycloalkoxy group and the like. Of the heterocyclic groups, "aromatic heterocyclic group" which is an atomic group remaining after removing from an aromatic heterocyclic compound a hydrogen atom bonding directly to a carbon atom or a hetero atom constituting the ring is preferable.

"The p-valent heterocyclic group" (p represents an integer of 1 or more) means an atomic group remaining after removing from a heterocyclic compound p hydrogen atoms among hydrogen atoms bonding directly to carbon atoms or hetero atoms constituting the ring. Of the p-valent heterocyclic groups, "p-valent aromatic heterocyclic group" which is an atomic group remaining after removing from an aromatic heterocyclic compound p hydrogen atoms among hydrogen atoms bonding directly to carbon atoms or hetero atoms constituting the ring is preferable.

The heterocyclic compound used herein optionally has a substituent, and the number of carbon atoms of the heterocyclic compound, not including the number of carbon atoms of the substituent, is usually 2 to 60, and preferably 4 to 20. The hetero atom in the heterocyclic compound includes, for example, at least one selected from the group consisting of a nitrogen atom, an oxygen atom and a sulfur atom. The number of the hetero atom in the heterocyclic compound is usually 1 to 20, and preferably 1 to 10. As the heterocyclic compound used herein, for example, pyridine, diazabenzene, triazine, azanaphthalene, diazanaphthalene, carbazole, dibenzofuran, dibenzothiophene, dibenzosilole, phenoxazine, phenothiazine, acridine, dihydroacridine, furan, thiophene, azole, diazole, triazole and julolidine are exmplified.

"The aromatic heterocyclic compound " means a compound in which the heterocyclic itself shows aromaticity such as oxadiazole, thiadiazole, thiazole, oxazole, thiophene, pyrrole, phosphole, furan, pyridine, pyrazine, pyrimidine, triazine, pyridazine, quinoline, isoquinoline, carbazole, dibenzophosphole and the like, and a compound in which an aromatic ring is condensed to the heterocyclic even if the heterocyclic itself shows no aromaticity such as phenoxazine, phenothiazine, dibenzoborole, dibenzosilole, benzopyran and the like.

The number of carbon atoms of the monovalent heterocyclic group, not including the number of carbon atoms of the substituent, is usually 2 to 60, and preferably 4 to 20.

The monovalent heterocyclic group optionally has a substituent and examples thereof include a thienyl group, a pyrrolyl group, a furyl group, a pyridinyl group, a piperidinyl group, a quinolinyl group, an isoquinolinyl group, a pyrimidinyl group, a triazinyl group, a julolidinyl group, and groups obtained by substituting a hydrogen atom in these groups with an alkyl group, a cycloalkyl group, an alkoxy group, a cycloalkoxy group or the like.

"The halogen atom" denotes a fluorine atom, a chlorine atom, a bromine atom or an iodine atom.

"The amino group" optionally has a substituent, and a substituted amino group is preferred. The substituent which the amino group has is preferably an alkyl group, a cycloalkyl group, an aryl group or a monovalent heterocyclic group.

The substituted amino group includes, for example, a dialkylamino group, a dicycloalkylamino group and a diarylamino group.

The amino group includes, for example, a dimethylamino group, a diethylamino group, a diphenylamino group, a bis(4-methylphenyl)amino group, a bis(4-tert-butylphenyl)amino group and a bis(3,5-di-tert-butylphenyl)amino group.

"The alkenyl group" may be any of linear and branched. The number of carbon atoms of the linear alkenyl group, not including the number of carbon atoms of the substituent, is usually 2 to 30, and preferably 3 to 20. The number of carbon atoms of the branched alkenyl group, not including the number of carbon atoms of the substituent, is usually 3 to 30, and preferably 4 to 20.

The number of carbon atoms of the "cycloalkenyl group", not including the number of carbon atoms of the substituent, is usually 3 to 30, and preferably 4 to 20.

The alkenyl group and the cycloalkenyl group optionally have a substituent and examples thereof include a vinyl group, a 1-propenyl group, a 2-propenyl group, a 2-butenyl group, a 3-butenyl group, a 3-pentenyl group, a 4-pentenyl group, a 1-hexenyl group, a 5-hexenyl group and a 7-octenyl group, and these groups having a substituent.

"The alkynyl group" may be any of linear and branched. The number of carbon atoms of the alkynyl group, not including the number of carbon atoms of the substituent, is usually 2 to 20, and preferably 3 to 20. The number of carbon atoms of the branched alkynyl group, not including the number of carbon atoms of the substituent, is usually 4 to 30, and preferably 4 to 20.

The number of carbon atoms of the "cycloalkynyl group", not including the number of carbon atoms of the substituent, is usually 4 to 30, and preferably 4 to 20.

The alkynyl group and the cycloalkynyl group optionally have a substituent and examples thereof include an ethynyl group, a 1-propynyl group, a 2-propynyl group, a 2-butynyl group, a 3-butynyl group, a 3-pentynyl group, a 4-pentynyl group, a 1-hexynyl group and a 5-hexynyl group, and these groups having a substituent.

"The arylene group" means an atomic group remaining after removing from an aromatic hydrocarbon two hydrogen atoms bonding directly to carbon atoms (preferably sp2 carbon atoms) constituting the ring. The number of carbon atoms of the arylene group, not including the number of carbon atoms of the substituent, is usually 6 to 60, preferably 6 to 30, and more preferably 6 to 18.

The arylene group optionally has a substituent and examples thereof include a phenylene group, a naphthalenediyl group, an anthracenediyl group, a phenanthrenedilyl group, a dihydrophenanthrenedilyl group, a naphthacenediyl group, a fluorenediyl group, a pyrenediyl group, a perylenediyl group and a chrysenediyl group, and these groups having a substituent, and groups represented by the formula (A-1) to the formula (A-20) are preferable. The arylene group includes groups obtained by bonding a plurality of these groups.

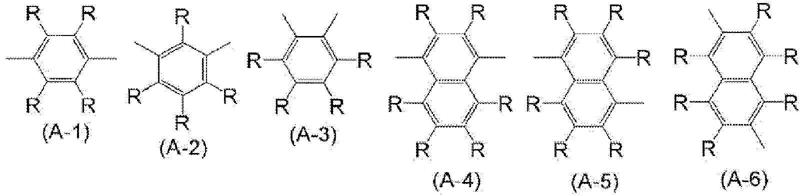

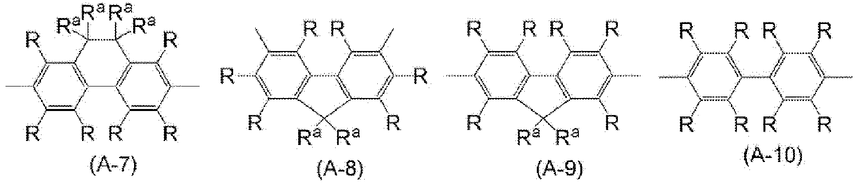

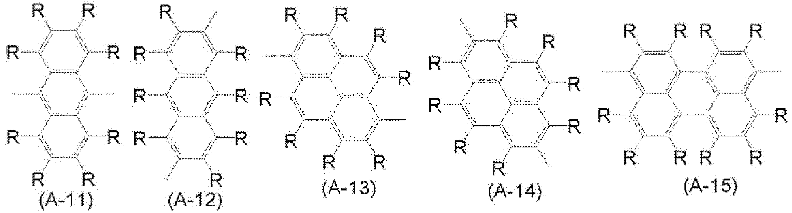

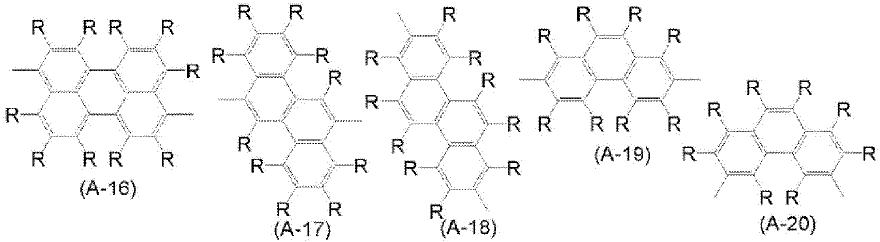

[wherein, R and R^{a} each independently represent a hydrogen atom, an alkyl group, a cycloalkyl group, an aryl group or a monovalent heterocyclic group. A plurality of R and R^{a} may be the same or different at each occurrence, and R^{a} may be combined together to form a ring together with atoms to which they are attached.]

In the present invention, the arylene group represented by Ar¹ means a mono-cyclic or condensed-cyclic arylene group.

The number of carbon atoms of the divalent heterocyclic group, not including the number of carbon atoms of the substituent, is usually 2 to 60, preferably 3 to 20, and more preferably 4 to 15.

The divalent heterocyclic group optionally has a substituent, and examples thereof include divalent groups obtained by removing from pyridine, diazabenzene, triazine, azanaphthalene, diazanaphthalene, carbazole, dibenzofuran, dibenzothiophene, dibenzosilole, phenoxazine, phenothiazine, acridine, dihydroacridine, furan, thiophene, azole, diazole or triazole two hydrogen atoms among hydrogen atoms bonding directly to carbon atoms or hetero atoms (preferably carbon atoms, more preferably sp2 carbon atoms) constituting the ring, and groups represented by the formula (AA-1) to the formula (AA-34) are preferable and groups represented by the formula (AA-1) to the formula (AA-32) are more preferable. The divalent heterocyclic group includes groups obtained by bonding a plurality of these groups. 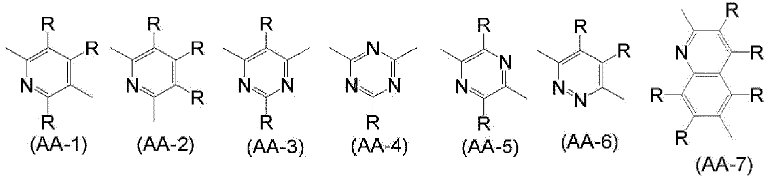 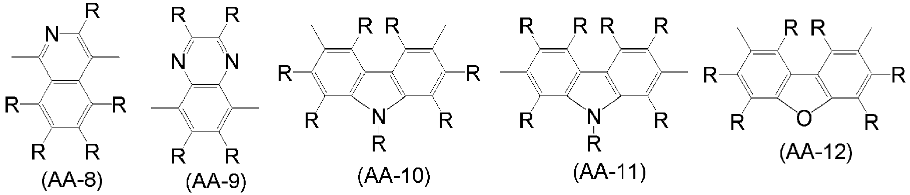 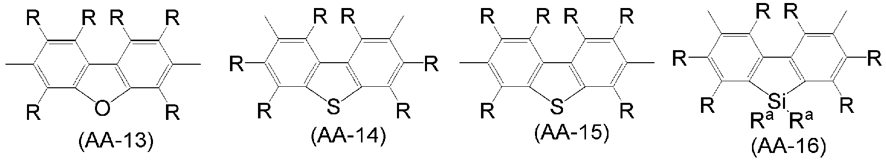 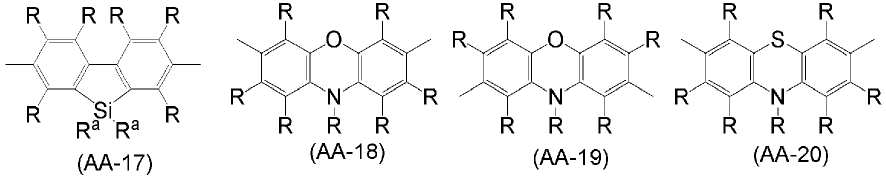 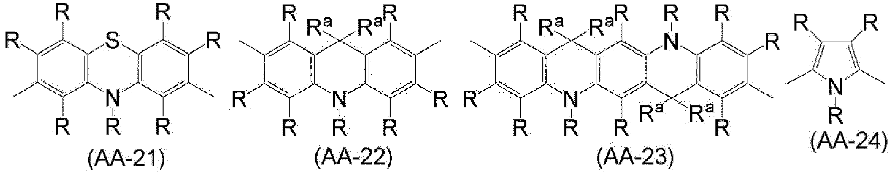 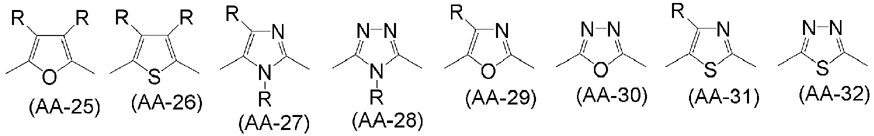 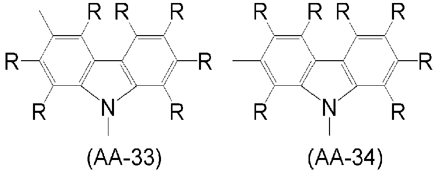 [wherein, R and R^{a} represent the same meaning as described above.].

In the present invention, the divalent heterocyclic group represented by Ar¹ means a mono-cyclic or condensed ring.

"The crosslinkable group" refers to a group capable of generating a new bond by being subjected to a heating treatment, an ultraviolet irradiation treatment, a near-ultraviolet irradiation treatment, a visible light irradiation treatment, an infrared irradiation treatment, a radical reaction and the like, and groups represented by any of the formulae (B-1) to (B-17) are preferable. These groups optionally have a substituent.

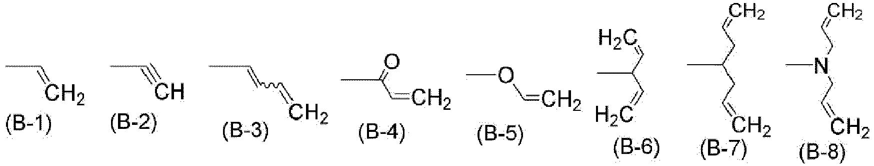

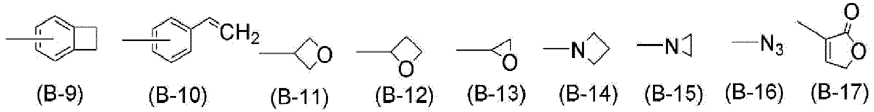

"The substituent" denotes a halogen atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group or a cycloalkynyl group, unless otherwise stated herein. The substituent may also be a crosslinkable group.

"The alkylene group" may be any of linear and branched. The number of carbon atoms of the linear alkylene group, not including the number of carbon atoms of the substituent, is usually 1 to 50, preferably 1 to 30, and more preferably 1 to 20. The number of carbon atoms of the branched alkylene group, not including the number of carbon atoms of the substituent, is usually 3 to 50, preferably 3 to 30, and more preferably 4 to 20.

The alkylene group optionally has a substituent and examples thereof include a methylene group, a 1,1-ethylene group, a 1,2-ethylene group, a 1,2-propylene group and a 1,3-propylene group, and groups obtained by substituting a hydrogen atom in these groups with a cycloalkyl group, an alkoxy group, a cycloalkoxy group, an aryl group, a fluorine atom and the like, for example, a difluoromethylene group, a tetrafluoroethylene group and the like.

The number of carbon atoms of "the cycloalkylene group", not including the number of carbon atoms of the substituent, is usually 3 to 50, preferably 3 to 30, and more preferably 4 to 20.

The cycloalkylene group optionally has a substituent and examples thereof include a 1,1-cyclohexylene group, a 1,2-cyclohexylene group, a 1,4-cyclohexylene group and a 3-methyl-1,2-cyclohexylene group.

"The hydrocarbon group" means an atomic group remaining after removing from a hydrocarbon one or more hydrogen atoms. The hydrocarbon used herein has a number of carbon atoms of usually 1 to 50, and preferably 1 to 20. As the hydrocarbon herein used, for example, methane, ethane, propane, n-butane, 2-methylpropane, n-pentane, 2-methylbutane, 2,2-dimethylpropane, n-hexane, n-heptane, n-octane, cyclopropane, cyclobutane, cyclopentane, cyclohexane, cycloheptane, cyclooctane, ethylene, propylene, 1-butene, 2-butene, 2-methylpropene, 1,3-butadiene, acetylene, propyne, butyne, benzene, naphthalene, anthracene, phenanthrene, pyrene, perylene, fluorene and dihydrophenanthrene are exemplified.

"The aromatic hydrocarbon group" means an atomic group remaining after removing from an aromatic compound one or more hydrogen atoms, among "hydrocarbon groups". The number of carbon atoms of the aromatic hydrocarbon, not including the number of carbon atoms of the substituent, is usually 6 to 60, preferably 6 to 30, and more preferably 6 to 18.

### <Compound represented by the formula (1)>

The present invention provides a compound represented by the above-described formula (1).

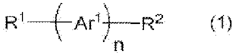

In the above-described formula (1), n is preferably an integer of 4 or more and 20 or less, more preferably an integer of 4 or more and 15 or less, and further preferably an integer of 4 or more and 12 or less. As the compound represented by the above-described formula (1), compounds having no molecular weight distribution are more preferable than polymer compounds having molecular weight distribution, since detection and purification of impurities are easy.

Ar¹ may have a group represented by the above-described formula (2-1) or the above-described formula (2-2) , in addition to substituents described in the explanation of common terms.

When an alkyl group, a cycloalkyl group, an aryl group and a monovalent heterocyclic group represented by R^{X1} has a substituent, the substituent includes also groups represented by the formula (2-1A) or the formula (2-2A), in addition to substituents described in the explanation of common terms.

―(Q¹)ₙ₁-Y¹(M¹)ₐ₁(Z¹)_{b1} (2-1A)

[wherein, n1, a1, b1, Q¹, Y¹, M¹ and Z¹ represent the same meaning as described above.]

―(Q²)ₙ₂-Y²(M²)ₐ₂(Z²)_{b2} (2-2A)

[wherein, n2, a2, b2, Q², Y², M² and Z² represent the same meaning as described above.]
R^{X1} is preferably an alkyl group, a cycloalkyl group, an aryl group or a monovalent heterocyclic group, more preferably an aryl group or a monovalent heterocyclic group, and further preferably an aryl group.

Regarding the group represented by the above-described formula (2):

An alkyl group, a cycloalkyl group, an aryl group and a monovalent heterocyclic group represented by R^{1g} may have a hydroxyl group, a mercapto group, or a group represented by the formula (2-1A) or the formula (2-2A), in addition to substituents described in the explanation of common terms.

R^{1g} is preferably an alkyl group, a cycloalkyl group, an aryl group or a monovalent heterocyclic group.

An alkyl group, a cycloalkyl group, an alkoxy group, a cycloalkoxy group, an aryl group, an aryloxy group and a monovalent heterocyclic group represented by R^{1a}, R^{1b}, R^{1c}, R^{1d}, R^{1e} and R^{1f} may have a group represented by the above-described formula (2-1A) or the above-described formula (2-2A), in addition to substituents described in the explanation of common terms.

It is preferable that at least one of R^{1a}, R^{1b}, R^{1e} and R^{1f} is an alkyl group, a cycloalkyl group, an alkoxy group, a cycloalkoxy group, an aryl group, an aryloxy group, a monovalent heterocyclic group, an amino group or a halogen atom, it is more preferable that at least one of R^{1b} and R^{1e} is an alkyl group, a cycloalkyl group, an alkoxy group, a cycloalkoxy group, an aryl group, an aryloxy group, a monovalent heterocyclic group, an amino group or a halogen atom, and it is further preferable that R^{1b} and R^{1e} are each an alkyl group, a cycloalkyl group, an alkoxy group, a cycloalkoxy group, an aryl group, an aryloxy group, a monovalent heterocyclic group, an amino group or a halogen atom.

It is preferable that at least one of R^{1a}, R^{1b}, R^{1e} and R^{1f} is an alkyl group, it is more preferable that at least one of R^{1b} and R^{1e} is an alkyl group, and it is further preferable that R^{1b} and R^{1e} are each an alkyl group.

The alkyl group represented by R^{1a}, R^{1b}, R^{1c}, R^{1d}, R^{1e} and R^{1f} is preferably a primary alkyl group, and the number of carbon atoms thereof, not including the number of carbon atoms of the substituent, is preferably 1 to 20, and more preferably 1 to 10.

If existing all Ar¹ are groups represented by the formula (2) and existing all X^{1a} are single bonds and existing all X^{1b} are groups represented by -C(R^{1g})₂- in the above-described existing all groups represented by the formula (2), then, at least one of R^{1a}, R^{1b}, R^{1e} and R^{1f} represents an alkyl group, a cycloalkyl group, an alkoxy group, a cycloalkoxy group, an aryl group, an aryloxy group, a monovalent heterocyclic group, an amino group or a halogen atom and these groups optionally have a substituent, in at least one of the above-described existing all groups represented by the formula (2).

In the above-described formula (1), at least two groups represented by the formula (2) represented by Ar¹ are preferably groups represented by the formula (2). A case where X^{2a} is a single bond is preferred.

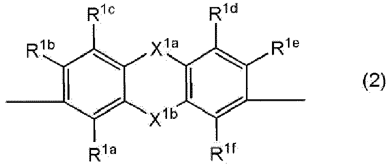

As the group represented by the above-described formula (2), for example, groups represented by the following formulae are exemplified. 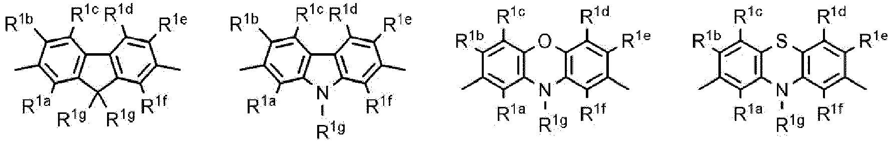 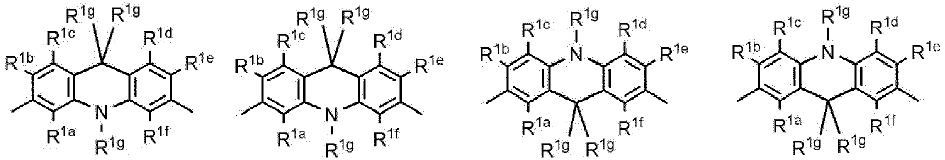 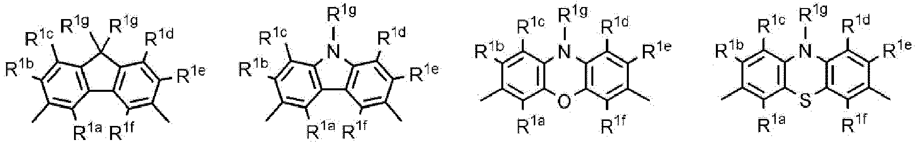 [wherein, R^{1a} to R^{1g} represent the same meaning as described above.]

Regarding the group represented by the formula (2-1) :

―R³―{(Q¹)ₙ₁―Y¹(M¹)ₐ₁(Z¹)_{b1}}ₘ₂ (2-1)

n1 is usually an integer of 0 to 10, and since synthesis of a compound represented by the formula (1) is easy, it is preferably an integer of 0 to 5, and more preferably 0.
a1 is usually an integer of 0 to 10, and since the stability and electron transportability of a compound represented by the formula (1) are excellent, it is preferably an integer of 0 to 5, and more preferably an integer of 0 to 2.
b1 is usually an integer of 0 to 10, and since the light emitting device of the present invention is excellent in luminance life, it is preferably an integer of 0 to 4, and more preferably 0 or 1.
m2 is usually an integer of 1 to 5, preferably 1 or 2, and more preferably 1.

The preferable ranges and examples of the aromatic hydrocarbon and the heterocyclic group represented by R³ are the same as those described in the explanation of common terms. R³ is preferably an aromatic hydrocarbon group optionally having a substituent. When the aromatic hydrocarbon group and the heterocyclic group have a substituent, the substituent includes not only groups mentioned above as the example of "substituent" but also groups having a chelating ability described later.

The preferable ranges and examples of the alkylene group, the cycloalkylene group and the arylene group represented by Q¹ are the same as those described in the explanation of common terms. Q¹ is preferably a single bond (n1 = 0), an alkylene group or an arylene group, and more preferably a single bond.

Y¹ is preferably -CO₂⁻, -CO₂Y¹', -SO₂⁻, -SO₂Y¹',-PO₃²⁻, -P(=O)(-OY¹')(-O⁻) or -P(=O)(-OY¹')₂, and more preferably -CO₂⁻ or -CO₂Y¹', since the light emitting device of the present invention is excellent in life.

The alkali metal cation represented by M¹ includes, for example, Li⁺, Na⁺, K⁺, Rb⁺ and Cs⁺, and since the driving voltage of the light emitting device of the present invention is reduced, it is preferably K⁺, Rb⁺ or Cs⁺, and more preferably Cs⁺.

The alkaline earth metal cation represented by M¹ includes, for example, Be²⁺, Mg²⁺, Ca²⁺, Sr²⁺ and Ba²⁺, and since the driving voltage of the light emitting device of the present invention is reduced, it is preferably Mg²⁺, Ca²⁺, Sr²⁺ or Ba²⁺, and more preferably Ba²⁺.

The substituent which the ammonium cation represented by M¹ optionally has is preferably an alkyl group, a cycloalkyl group or an aryl group, and more preferably an alkyl group.

M¹ is preferably an alkali metal cation or an alkaline earth metal cation, and more preferably an alkali metal cation, since the light emitting device of the present invention is excellent in life.

Y¹ is preferably -CO₂Y¹', -SO₃Y¹', -SO₂Y¹',-P(=O)(-OY¹')(-O⁻) or -P(=O)(-OY¹')₂, from the standpoint of the stability of a compound in synthesizing a compound represented by the above-described formula (1) and the stability of the substrate in the reaction. Particularly, Y¹' is preferably a hydrocarbon group optionally having a substituent or a heterocyclic group optionally having a substituent.

A reagent such as a metal hydroxide, a metal carbonate or an alkylammonium hydroxide is added to compounds represented by the above-described formula (1) in which Y¹' is a hydrocarbon group optionally having a substituent or a heterocyclic group optionally having a substituent, and if necessary, they are dissolved or suspended in water or an organic solvent, and they can be reacted to synthesize the corresponding metal salt or alkylammonium salt. Further, a strong acid such as hydrochloric acid, nitric acid, sulfuric acid and the like can be added to the resultant metal salt or alkylammonium salt to synthesize a compound in which Y¹' is a hydrogen atom.

The compound can also be synthesized by adding another metal hydroxide, metal carbonate or alkylammonium salt to compounds represented by the formula (1) in which M¹ is a metal salt or alkylammonium salt, and performing transmetalation thereof.

The metal hydroxide includes, for example, hydroxides of alkali metals and hydroxides of alkaline earth metals such as LiOH, NaOH, KOH, RbOH, CsOH, Mg(OH)₂, Ca(OH)₂ and the like. The metal carbonate includes, for example, carbonates of alkali metals such as Li₂CO₃, Na₂CO₃, K₂CO₃, Rb₂CO₃, Cs₂CO₃ and the like. As the alkylammonium salt compound, tetramethylammonium hydroxide, tetraethylammonium hydroxide, tetrabutylammonium hydroxide and the like are exmplified. The equivalent amount of the metal hydroxide, metal carbonate and alkylammonium hydroxide is usually 0.5 to 10 equivalent, and preferably 1.0 to 2.0 equivalent with respect to acids or esters represented by -CO₂Y¹', -SO₃Y¹', -SO₂Y¹', -P(=O)(-OY¹')(-O⁻) or -P(=O)(-OY¹')₂ in the formula (2-1). The amount of the solvent used for the reaction is usually 1-fold by weight to 10000-fold by weight with respect to a compound represented by the above-described formula (1). The reaction time is usually 1.0 to 50 hours, and the reaction temperature is usually 0 to 100°C . After the reaction, the object is dissolved in a solvent and washed with water, then, concentrated, alternatively, when the solubility of the object is low, the reaction product is concentrated as it is, and can be purified by means such as crystallization and chromatography.

R^{a} in B(R^{a})4⁻, R^{a}SO₃⁻ and R^{a}COO⁻ represented by Z¹ is preferably an alkyl group or an aryl group, and more preferably an alkyl group.

Z¹ is preferably F⁻, Cl⁻, Br⁻, I⁻, OH⁻, B (R^{a})₄⁻, R^{a}SO₃⁻, R^{a}COO⁻ or NO₃⁻, and more preferably F⁻, Cl⁻, Br⁻, I⁻, OH⁻, R^{a}SO₃⁻ or R^{a}COO⁻, since synthesis of a polymer compound containing a constitutional unit represented by the formula (1) is easy.

In the above-described formula (2-1), it is preferable that n1 is 0, and Y¹' is a hydrocarbon group optionally having a substituent, a heterocyclic group optionally having a substituent or a hydrogen atom, or M¹ is an alkali metal cation or an alkaline earth metal cation, it is more preferable that Y¹' is a hydrocarbon group optionally having a substituent, a heterocyclic group optionally having a substituent or a hydrogen atom, or M¹ is an alkali metal cation, it is further preferable that Y¹' is a hydrogen atom, or M¹ is an alkali metal cation, and it is particularly preferable that M¹ is an alkali metal cation.

The group represented by the above-described formula (2-1) is preferably a group represented by the formula (2-1B) or the formula (2-1C).

―R³―Co₂⁻(M²)⁺ (2-1B)

―R³―CO₂Y^{2'} (2-1C)

[wherein,
R³ represents the same meaning as described above. (M²)⁺ represents an alkali metal cation.
Y²' represents a hydrocarbon group having 1 to 8 carbon atoms optionally having a substituent, a heterocyclic group having 1 to 8 carbon atoms optionally having a substituent or a hydrogen atom.]
Y²' is preferably a hydrogen atom.

It is preferable for the group represented by the above-described formula (2-1) to carry a group having a chelating ability on R³, from the standpoint of the luminance life of the light emitting device of the present invention.

The group having a chelating ability means a group having a polydentate ligand capable of coordinating with a metal ion to form a chelate compound. The group having a chelating ability is constituted of a hetero atom selected from the group consisting of a nitrogen atom, an oxygen atom, a phosphorus atom and a sulfur atom, and a carbon atom and a hydrogen atom, and may contain other elements such as a halogen atom and the like, and may be any of linear, branched and cyclic. The number of carbon atoms thereof is usually 3 to 60, and preferably 5 to 30. The hetero atom is preferably selected from a nitrogen atom, an oxygen atom and a sulfur atom, with an oxygen atom being more preferred. The number of hetero atoms is usually 2 to 20, and preferably 3 to 10.

The group having a chelating ability is preferably a hydrocarbyl group substituted with two or more hetero atoms, and preferably has a structural unit with which two hetero atoms and cations can form a 5-membered ring or 6-membered ring, and optionally has a substituent, from the standpoint of the luminance life of the light emitting device of the present invention.

The group having a chelating ability is more preferably a group represented by the formula (5-0) or a group represented by the formula (6-0). 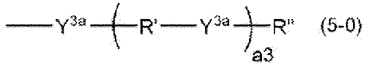 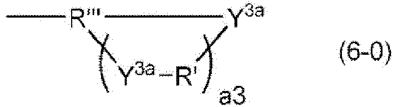 [wherein,
a3, R', R" and R‴ represent the same meaning as described above.
Y^{3a} represents an oxygen atom, a sulfur atom or - NR"-. When a plurality of Y^{3a} are present, they may be the same or different.]
Y^{3a} is preferably an oxygen atom.

The group represented by the above-described formula (2-1) is preferably a group represented by the formula (2-3). 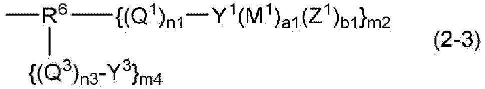
n3 is usually an integer of 0 to 30, preferably an integer of 0 to 20, and more preferably an integer of 0 to 8.
m4 is usually an integer of 1 to 5, and since synthesis of a compound represented by the formula (1) is easy, it is preferably 1 or 2, and more preferably 1.
Y³ represents a group represented by the formula (5) or the formula (6).

   ―O― (R'O)ₐ₃-R" (5) 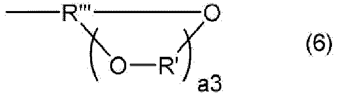
a3 is usually an integer of 1 to 20, preferably an integer of 3 to 10, and more preferably an integer of 5 to 10.

The number of carbon atoms of the alkylene group represented by R', not including the number of carbon atoms of the substituent, is usually 1 to 10, preferably 2 to 10, and more preferably 2 to 6.

The number of carbon atoms of the cycloalkylene group represented by R', not including the number of carbon atoms of the substituent, is usually 3 to 50, preferably 3 to 30, and more preferably 4 to 20.

The number of carbon atoms of the arylene group represented by R', not including the number of carbon atoms of the substituent, is usually 6 to 60, preferably 6 to 30, and more preferably 6 to 18.

R' is preferably an alkylene group or an arylene group, and more preferably an alkylene group, since synthesis of a compound represented by the formula (1) is easy.

The number of carbon atoms of the alkyl group represented by R", not including the number of carbon atoms of the substituent, is usually 1 to 50, preferably 1 to 30, and more preferably 1 to 10.

The number of carbon atoms of the cycloalkyl group represented by R", not including the number of carbon atoms of the substituent, is usually 3 to 50, preferably 3 to 30, and more preferably 4 to 20.

The number of carbon atoms of the aryl group represented by R", not including the number of carbon atoms of the substituent, is usually 6 to 60, preferably 6 to 20, and more preferably 6 to 10.

R" is preferably an alkyl group or a cycloalkyl group, more preferably an alkyl group, and further preferably methyl group or ethyl group.

The number of carbon atoms of the hydrocarbon group represented by R‴, not including the number of carbon atoms of the substituent, is usually 1 to 20, preferably 1 to 10, and more preferably 2 to 10.

The hydrocarbon group represented by R‴ may be an aliphatic hydrocarbon group or an aromatic hydrocarbon group, and since synthesis of a polymer compound containing a constitutional unit represented by the formula (1) is easy, it is preferably an aliphatic hydrocarbon group.

Y³ is preferably a group represented by the formula (5) .

The group represented by the formula (5) or the formula (6) represented by Y³ includes, for example, groups represented by the following formulae.

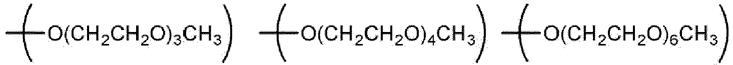

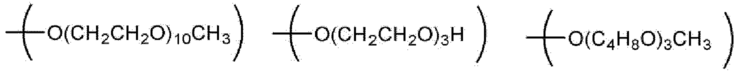

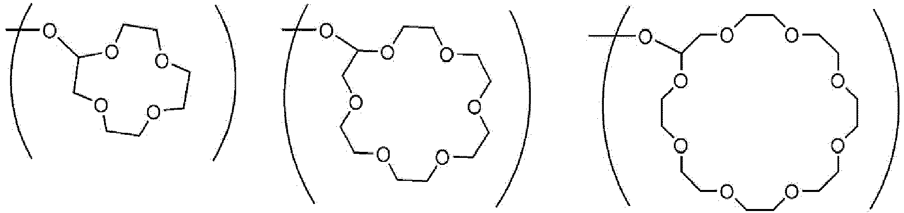

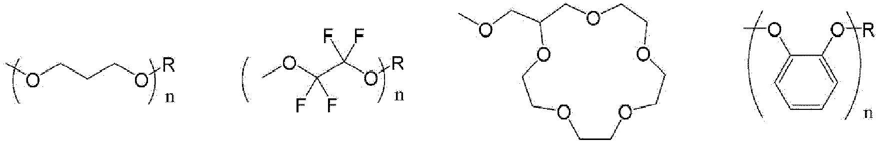

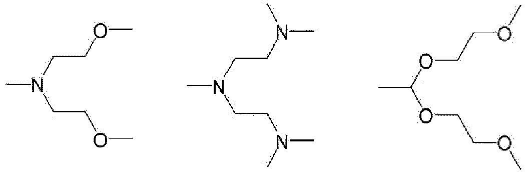

The group having a chelating ability includes, for example, groups represented by the following formulae, in addition to the group represented by the formula (5-0) and the group represented by the formula (6-0).

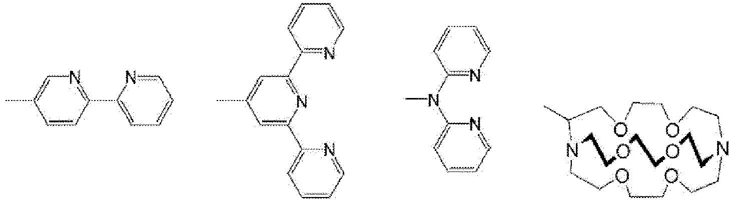

The group represented by the formula (2-1) includes, for example, groups represented by the following formulae.

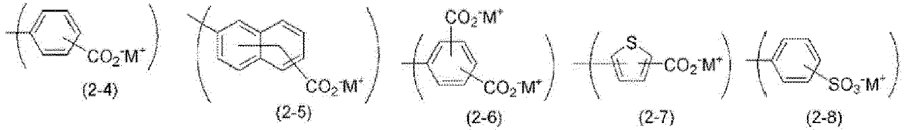

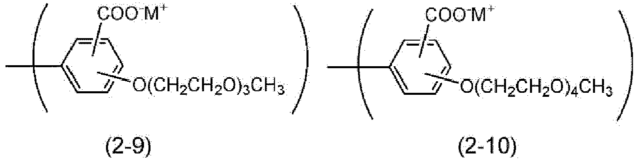

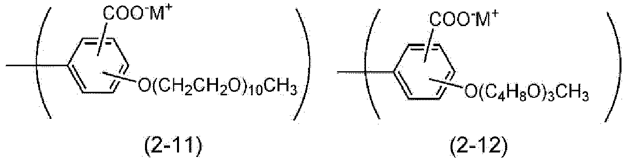

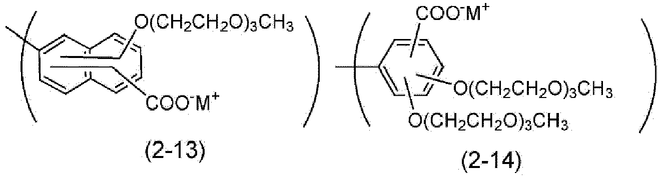

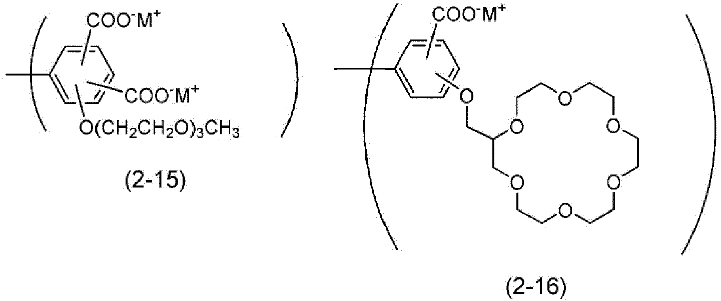

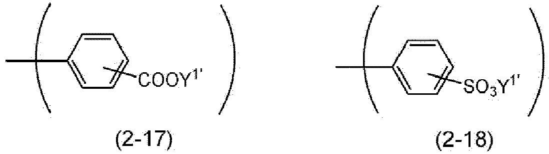

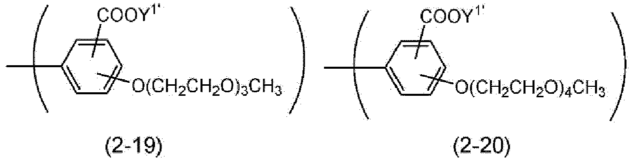

[wherein, Y¹' represents the same meaning as described above. M⁺ represents Li⁺, Na⁺, K⁺, Rb⁺, Cs⁺ or N⁺ (CH₃)₄. When a plurality of M⁺ are present, they may be the same or different.]

Regarding the group represented by the above-described formula (2-2):
n2 is usually an integer of 0 to 10, and since the light emitting device of the present invention is excellent in luminance life, it is preferably an integer of 0 to 8, and more preferably an integer of 0 to 2.
a2 is usually an integer of 1 to 10, and since the light emitting device of the present invention is excellent in luminance life, it is preferably an integer of 1 to 5, and more preferably 1 or 2.
b2 is usually an integer of 0 to 10, and since the light emitting device of the present invention is excellent in luminance life, it is preferably an integer of 0 to 4, and more preferably 0 or 1.
m3 is usually an integer of 1 to 5, and since the light emitting device of the present invention is excellent in luminance life, it is preferably 1 or 2, and more preferably 1.

The preferable ranges and examples of the aromatic hydrocarbon group and the heterocyclic group represented by R⁴ are the same as those described in the explanation of common terms. R³ is preferably an aromatic hydrocarbon group optionally having a substituent.

The preferable ranges and examples of the alkylene group, the cycloalkylene group and the arylene group represented by Q² are the same as those described in the explanation of common terms. Q² is preferably an alkylene group or an arylene group.

R^{c} in -C⁺R^{c}₂, -N⁺R^{c}₃, -P⁺R^{c}₃, -S⁺R^{c}₂ and -I⁺R^{c}₂ represented by Y² is preferably a hydrogen atom, an alkyl group or an aryl group, and more preferably a hydrogen atom or an alkyl group, since synthesis of a compound represented by the formula (1) is easy.

Y² is preferably -C⁺R^{c}₂, -N⁺R^{c}₃, -P⁺R^{c}₃ or -S⁺R^{c}₂, and more preferably -N⁺R^{c}₃, since synthesis of a compound represented by the formula (1) is easy and the compound is excellent in stability.

R^{b} in B (R^{b})₄⁻, R^{b}SO₃⁻ and R^{b}COO⁻ represented by M² is preferably an alkyl group or an aryl group, and more preferably an alkyl group, since synthesis of a compound represented by the formula (1) is easy.

M² is preferably F⁻, Cl⁻, Br⁻, I⁻, B (R^{b})₄⁻, R^{b}SO₃⁻, R^{b}COO, BF₄⁻ or SbF⁶⁻, and more preferably Br⁻, I⁻, B (R^{b})₄⁻, R^{b}COO⁻ or SbF⁶⁻, since the light emitting device of the present invention is excellent in luminance life.

The alkali metal cation represented by Z² includes, for example, Li⁺, Na⁺, K⁺, Rb⁺ and Cs⁺, and since synthesis of a compound represented by the formula (1) is easy, it is preferably Li⁺, Na⁺ or K⁺.

The alkaline earth metal cation represented by Z² includes, for example, Be²⁺, Mg²⁺, Ca²⁺, Sr²⁺ and Ba²⁺, and since synthesis of a compound represented by the formula (1) is easy, it is preferably Mg²⁺ or Ca²⁺.

Z² is preferably an alkali metal cation, since synthesis of a compound represented by the formula (1) is easy.

The group represented by the formula (2-2) includes, for example, groups represented by the following formulae. 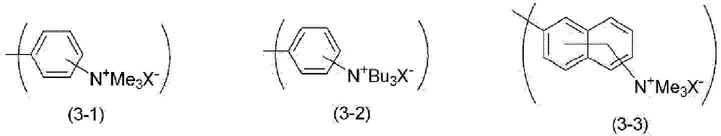 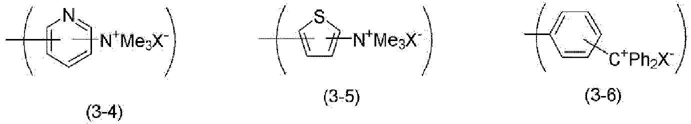 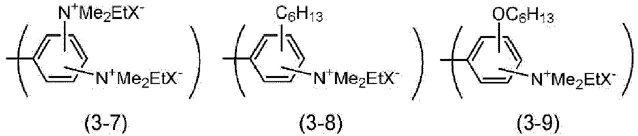 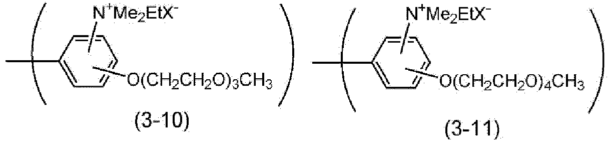 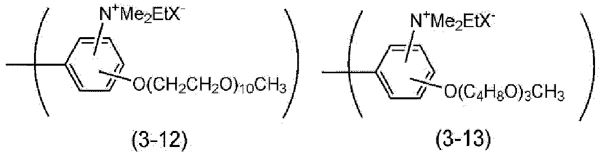 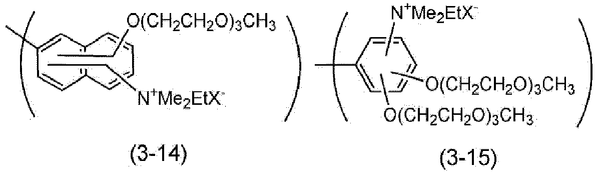 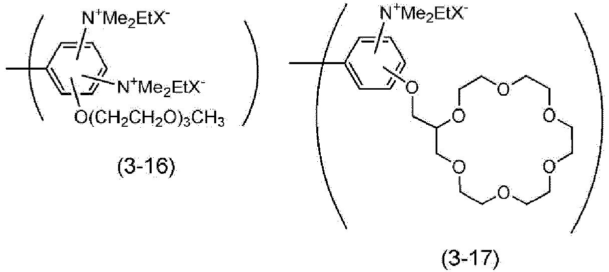 [wherein, X⁻ represents F⁻, Cl⁻, Br⁻, I⁻, B⁻ (C₆H₅)₄, CH₃COO⁻ or CF₃SO₃⁻.]

Of groups represented by the above-described formula (2-1) or the above-described formula (2-2), R^{1g} is preferably a group represented by the above-described formula (2-1).

In the above-described formula (1), it is more preferable that at least two groups represented by the formula (2) are groups represented by the formula (2) in which at least one of R^{1g} is a group represented by the formula (2-1), and in the above-described formula (1), it is further preferable that at least two groups represented by the formula (2) are groups represented by the formula (2) in which at least one of R^{1g} is a group represented by the formula (2-3).

In the above-described formula (1), at least one of Ar¹ is preferably a mono-cyclic or condensed-cyclic arylene group other than a group represented by the above-described formula (2), a mono-cyclic or condensed-cyclic divalent heterocyclic group other than a group represented by the above-described formula (2) or a group represented by -N(R^{X1})-.

The compound represented by the above-described formula (1) is preferably a compound represented by the formula (1-1), from the standpoint of the luminance life of the light emitting device of the present invention. 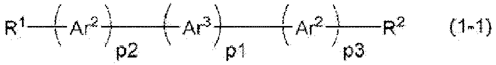 p1 is usually an integer of 2 to 18, preferably an integer of 2 to 13, and more preferably an integer of 2 to 10. p2 and p3 are each usually an integer of 1 to 9, preferably an integer of 1 to 6, and more preferably an integer of 1 to 3. The sum of p1, p2 and p3 is preferably 15 or less, and more preferably 12 or less.

The group represented by Ar³ optionally has a substituent represented by the above-described formula (2-1A) or the above-described formula (2-2A), in addition to substituents described in the explanation of common terms.

The compound represented by the formula (1) is preferably a compound represented by the formula (1A), from the standpoint of the luminance life of the light emitting device of the present invention. 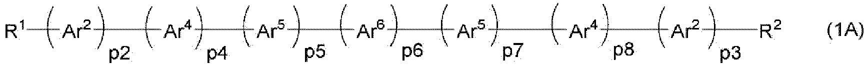
p4 and p8 each independently represent usually an integer of 1 to 9, and since synthesis is easy, it is preferably an integer of 1 to 3, and more preferably 1.
p5 and p7 each independently represent usually an integer of 0 to 16, and preferably an integer of 0 to 6.
p6 is usually an integer of 0 to 16, and since synthesis is easy, it is preferably an integer of 0 to 6, more preferably an integer of 0 to 3, and further preferably 1.

The sum of p2, p3, p4, p5, p6, p7 and p8 is preferably 15 or less, and more preferably 12 or less.

It is preferable for Ar⁴ that at least one of R^{1g} is a group represented by the above-described formula (2-1) or a group represented by the above-described formula (2-2) in at least one of p4 Ar⁴, and at least one of R^{1g} is a group represented by the above-described formula (2-1) or a group represented by the above-described formula (2-2) in at least one of p8 Ar⁴.

The group represented by Ar⁵ optionally has a substituent represented by the above-described formula (2-1A) or the above-described formula (2-2A), in addition to substituents described in the explanation of common terms.

In the group represented by the above-described formula (2) represented by Ar⁶, may be a group other than the group represented by the above-described formula (2-1) or the above-described formula (2-2).

It is preferable that p6 is 1 or more, and in at least one Ar⁶, one of the above-described X^{1a} and the above-described X^{1b} is a single bond, and it is more preferable that the above-described X^{1a} is a single bond.

It is preferable that in all Ar⁶, one of the above-described X^{1a} and the above-described X^{1b} is a single bond.

In the above-described formula (1), the above-described formula (1-1) and the above-described formula (1A), it is preferable that at least two groups represented by the above-described formula (2) are groups represented by the above-described formula (2A), it is more preferable that all groups represented by the above-described formula (2) are groups represented by the above-described formula (2A), and it is further preferable that at least two groups represented by the above-described formula (2A) are groups represented by the formula (2A').

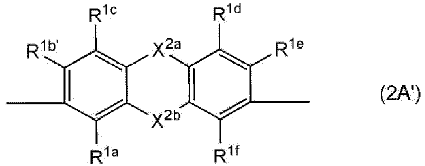

The group represented by R^{1b}' optionally has a substituent represented by the above-described formula (2-1A) or the above-described formula (2-2A), in addition to substituents described in the explanation of common terms.

X^{2a} is preferably a single bond.

In the above-described formula (2), the above-described formula (2A) and the above-described formula (2A'), it is preferable that at least one of the above-described is the above-described formula (2-1), more preferably the above-described formula (2-3), from the standpoint of the luminance life of the light emitting device of the present invention.

The compound represented by the above-described formula (1A) is more preferably a compound presented by the formula (1B). [wherein, 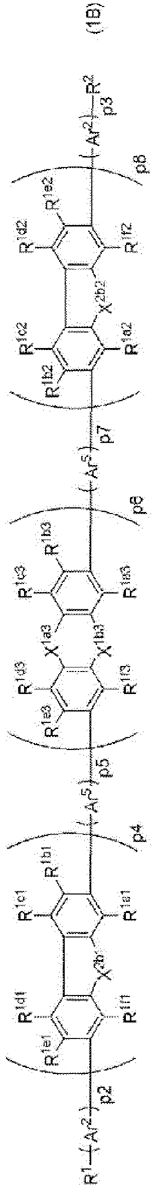
R¹, R², Ar², Ar⁵, p2 to p8 represent the same meaning as described above.
R^{1a1} to R^{1f1}, R^{1a2} to R^{1f2}, R^{1a3} to R^{1f3} each independently represent a hydrogen atom, an alkyl group, a cycloalkyl group, an alkoxy group, a cycloalkoxy group, an aryl group, an aryloxy group, a monovalent heterocyclic group, an amino group, a halogen atom, a group represented by the formula (2-1) or a group represented by the formula (2-2), and these groups optionally have a substituent.
X^{1a3} and X^{1b3} each independently represent a single bond, an oxygen atom, a sulfur atom, a group represented by -S(=O)-, a group represented by -S(=O)₂-, a group represented by -C(=O)-, a group represented by -C(R^{1g})₂-, a group represented by -Si(R^{1g})₂-, a group represented by -NR^{1g}- or a group represented by C(R^{1g})₂-C(R^{1g})₂-. At least one of X^{1a3} and X^{1b3} represents a group represented by -C(R^{1g})₂- or a group represented by -NR^{1g}-. R^{1g} represents the same meaning as described above.
X^{2b1} and X^{2b2} each independently represent a group represented by -C(R^{1g})₂- or a group represented by-NR^{1g}-, and represents the same meaning as described above. At least one R^{1g} in X^{2b1} and at least one R^{1g} in X^{2b2} are groups represented by the formula (2-1) or groups represented by the formula (2-2).]

It is preferable that X^{1a3} and X^{1b3} are each a single bond, a group represented by -C(R^{1g})₂- or a group represented by -NR^{1g}-, and one of X^{1a3} and X^{1b3} is a single bond, and it is more preferable that X^{1a3} is a single bond.

From the standpoint of the luminance life of the light emitting device of the present invention, it is preferable that at least one of p2 Ar² is a group obtained by removing two hydrogen atoms constituting the ring from a benzene ring or an aromatic hydrocarbon ring in which only two or more and four or less benzene rings are condensed (the group optionally has a substituent), and at least one of p3 Ar² is a group obtained by removing two hydrogen atoms constituting the ring from a benzene ring or an aromatic hydrocarbon ring in which only two or more and four or less benzene rings are condensed (the group optionally has a substituent), and it is more preferable that all Ar² are groups obtained by removing two hydrogen atoms constituting the ring from a benzene ring or an aromatic hydrocarbon ring in which only two or more and four or less benzene rings are condensed (the group optionally has a substituent).

In X^{2b1} and X^{2b2}, it is preferable that p4 and p8 are each 1, and it is more preferable that R^{1b1} and R^{1b2} each independently represent an alkyl group, a cycloalkyl group, an alkoxy group, a cycloalkoxy group, an aryl group, an aryloxy group, a monovalent heterocyclic group, an amino group, a halogen atom, a group represented by the formula (2-1) or a group represented by the formula (2-2), and these groups optionally have a substituent.

The compound represented by the above-described formula (1B) is more preferably a compound represented by the formula (1C). 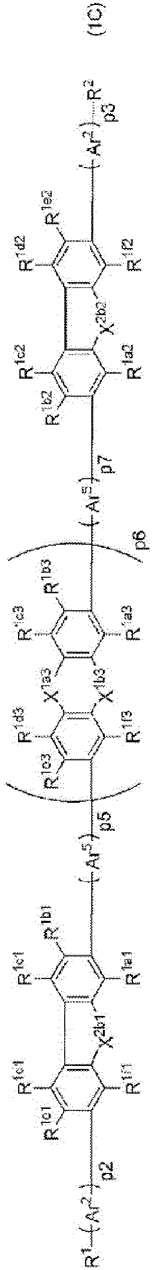 [wherein,
R¹, R², Ar², Ar⁵, p2, p3, p5 to p7, R^{1a1} to R^{1f1}, R^{1a2} to R^{1f2}, R^{1a3} to R^{1f3}, X^{1a3} and X^{1b3} represent the same meaning as described above.
X^{2b1} and X^{2b2} each independently represent a group represented by -C(R^{1g})₂- or a group represented by-NR^{1g}-, and at least one R^{1g} in X^{2b1} and at least one in X^{2b2} are groups represented by the formula (2-1) or groups represented by the formula (2-2).
R^{1g} represents the same meaning as described above.]

In the above-described formula (1C), it is preferable that p6 is an integer of 1 or more, X^{1a3} and X^{1b3} are each a single bond, a group represented by-C(R^{1g})₂- or a group represented by -NR^{1g}-, and one of X^{1a3} and X^{1b3} is a single bond, and more preferably, X^{1a3} is a single bond.

It is preferable that X^{1a3} is a single bond and X^{1b3} is a group represented by -NR^{1g}-.

When X^{1a3} is a single bond and X^{1b3} is a group represented by -C(R^{1g})₂-, it is preferable that at least one of R^{1a3}, R^{1b3}, R^{1e3} and R^{1f3} is an alkyl group, a cycloalkyl group, an alkoxy group, a cycloalkoxy group, an aryl group, an aryloxy group, a monovalent heterocyclic group, an amino group or a halogen atom, it is more preferable that at least one of R^{1b3} and R^{1e3} is an alkyl group, a cycloalkyl group, an alkoxy group, a cycloalkoxy group, an aryl group, an aryloxy group, a monovalent heterocyclic group, an amino group or a halogen atom, and it is further preferable that R^{1b3} and R^{1e3} are each an alkyl group, a cycloalkyl group, an alkoxy group, a cycloalkoxy group, an aryl group, an aryloxy group, a monovalent heterocyclic group, an amino group or a halogen atom.

It is more preferable that at least one of R^{1a3}, R^{1b3}, R^{1e3} and R^{1f3} is an alkyl group, at least one of R^{1b3} and R^{1e3} is an alkyl group, or R^{1b3} and R^{1e3} are each an alkyl group.

These groups optionally have a substituent represented by the above-described formula (2-1A) or the above-described formula (2-2A),in addition to substituents described in the explanation of common terms.

In the above-described formula (1C), it is preferable that p5 or p7 is 1 or more, and at least one group represented by -NR^{x1}- is contained. R^{x1} represents the same meaning as described above.

Regarding Ar¹ other than the group represented by the above-described formula (2):
As the mono-cyclic or condensed-cyclic arylene group and the mono-cyclic or condensed-cyclic divalent heterocyclic group other than a group represented by the above-described formula (2) represented by Ar¹ other than the group represented by the above-described formula (2), preferable is a group obtained by removing 2 hydrogen atoms constituting the ring from a benzene ring or an aromatic hydrocarbon ring in which only 2 or more and 10 or less benzene rings are condensed (the group optionally has a substituent) or a group represented by the formula (4).

The group obtained by removing 2 hydrogen atoms constituting the ring from a benzene ring or an aromatic hydrocarbon ring in which only 2 or more and 10 or less benzene rings are condensed is preferably a mono-cyclic benzene ring or a ring obtained by condensing 2 to 4 benzene rings.

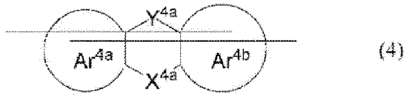

Ar^{4a} and Ar^{4b} optionally have a substituent represented by the above-described formula (2-1) or the above-described formula (2-2), in addition to substituents described in the explanation of common terms. When a plurality of the above-described substituents are present, they may be combined together to form a ring together with atoms to which they are attached.

It is preferable for X^{4a} and Y^{4a} that one of X^{4a} and Y^{4a} is a single bond.

The preferable ranges and examples of the alkyl group, the cycloalkyl group, the aryl group and the monovalent heterocyclic group represented by R are the same as those described in the explanation of common terms. As the substituent which R optionally has, a substituent represented by the above-described formula (2-1A) or the above-described formula (2-2A) may be carried, in addition to substituents described in the explanation of common terms. When a plurality of R are present, they may be the same or different and may be combined together to form a ring. When a plurality of R are present and they are combined together to form a ring, it is typical that the ring is formed together with atom to which these groups are attached.

The substituent which Ar^{4a} optionally has and R, and the substituent which Ar^{4b} optionally has and R each may be combined together to form a ring together with atoms to which they are attached.

It is preferable for Ar^{4a} and Ar^{4b} that Ar^{4a} and Ar^{4b} are each a benzene ring, more preferably are represented by the formula (4A).

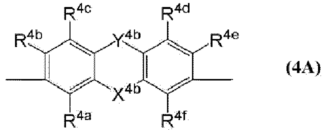

The preferable ranges and examples of the alkyl group, the cycloalkyl group, the alkoxy group, the cycloalkoxy group, the aryl group, the aryloxy group, the monovalent heterocyclic group and the amino group represented by R^{4a} to R^{4f} are the same as those described in the explanation of common terms.

The alkyl group, the cycloalkyl group, the alkoxy group, the cycloalkoxy group, the aryl group, the aryloxy group and the monovalent heterocyclic group represented by R^{4a} to R^{4f} optionally have a group represented by the above-described formula (2-1A) and the above-described formula (2-2A), in addition to substituents described in the explanation of common terms.

As the compound represented by the above-described formula (4), compounds represented by the following formulae are exemplified.

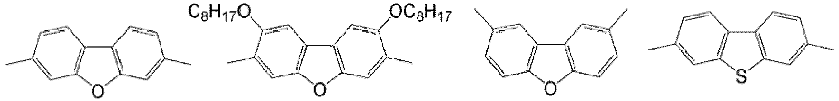

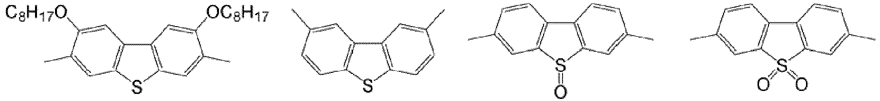

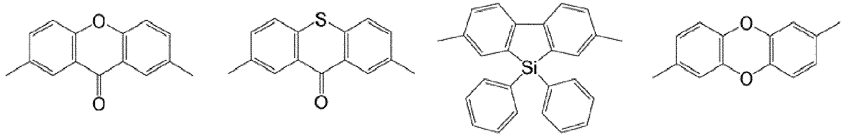

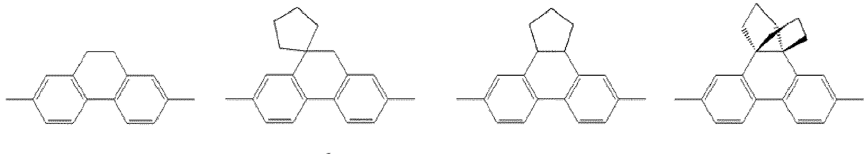

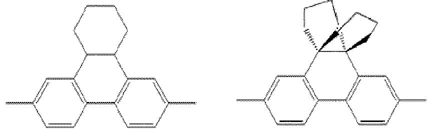

It is preferable for the compound represented by the above-described formula (1) that the group represented by Ar¹ is composed only of groups selected from the group consisting of the above-described formula (2), a group obtained by removing 2 hydrogen atoms constituting the ring from a benzene ring or an aromatic hydrocarbon ring in which only 2 or more and 10 or less benzene rings are condensed (the group optionally has a substituent), a group represented by the above-described formula (4) and a group represented by -N(R^{X1})- (R^{1g} represents the same meaning as described above).

As the compound represented by the above-described formula (1), compounds represented by the following formulae are exmplified.

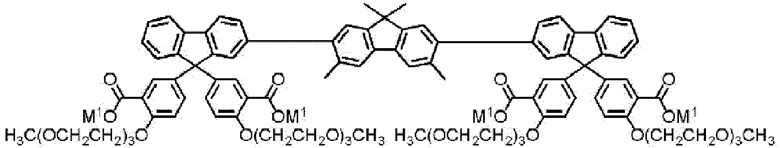

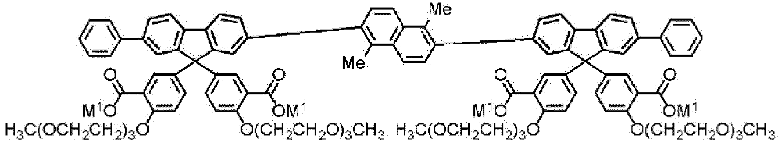

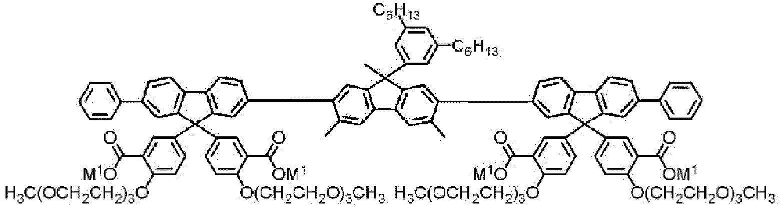

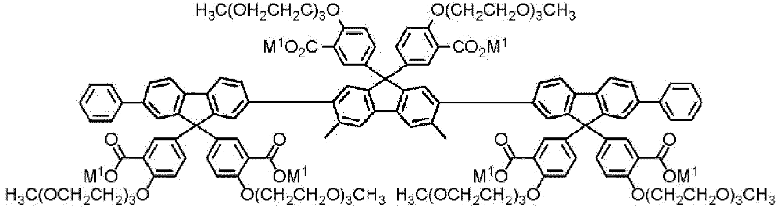

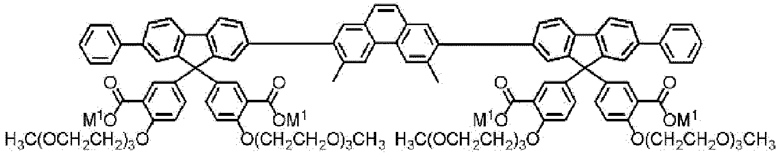

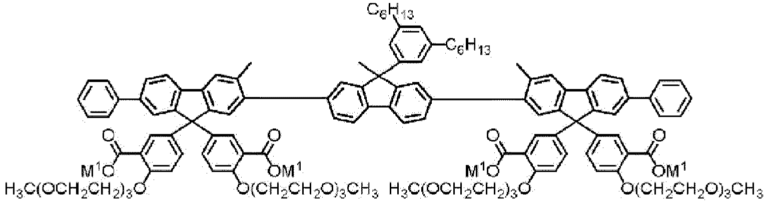

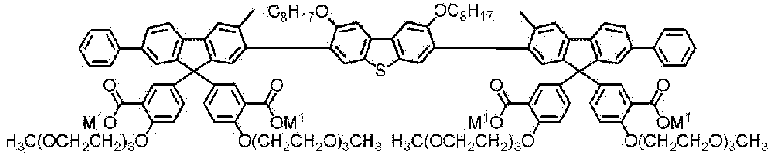

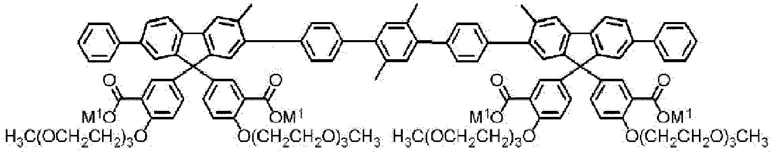

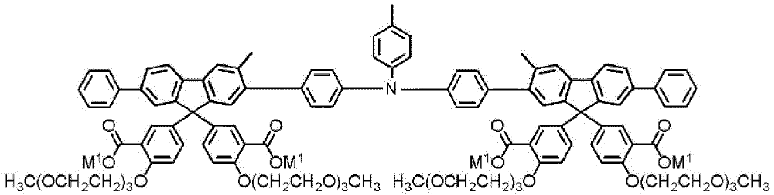

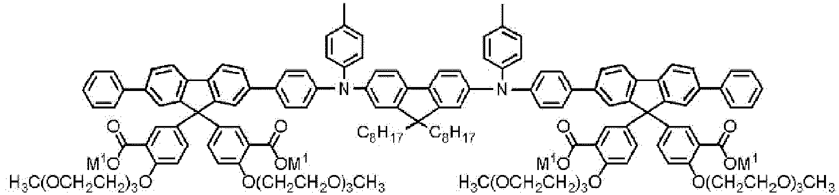

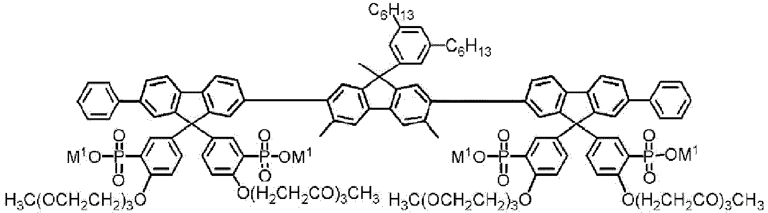

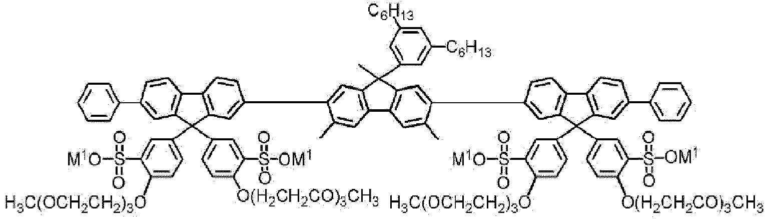

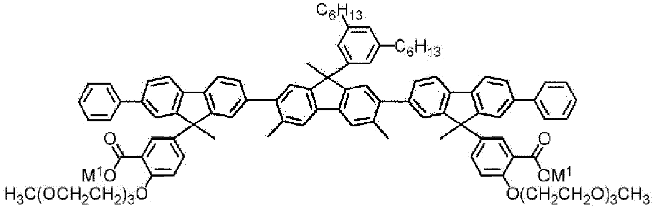

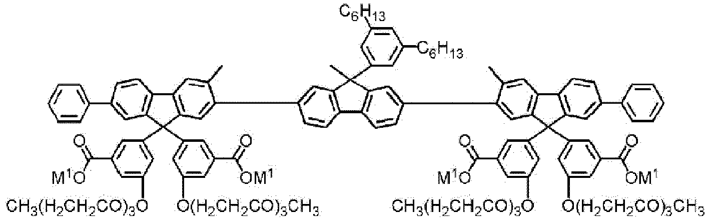

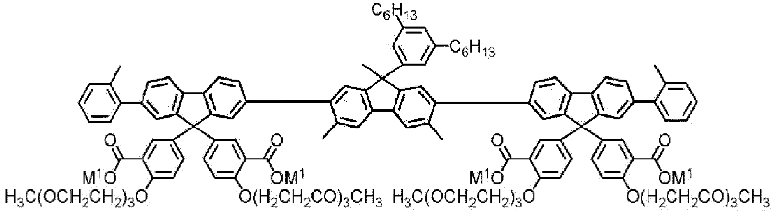

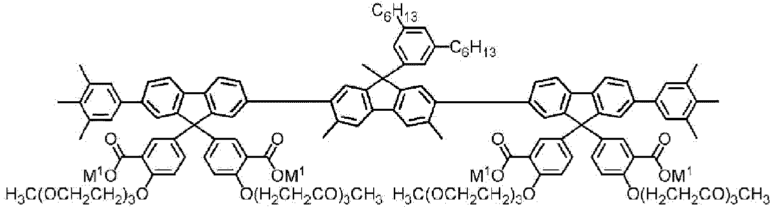

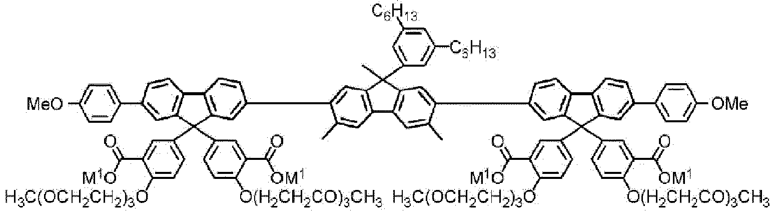

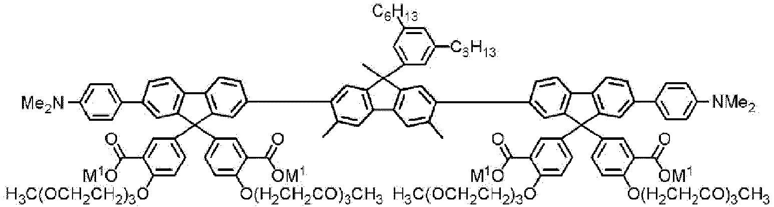

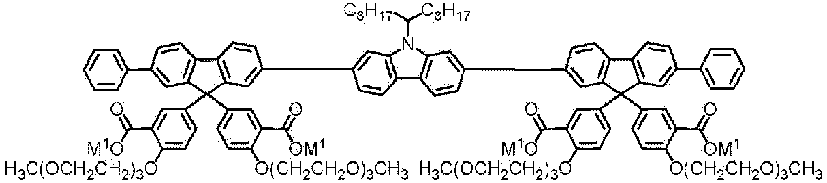

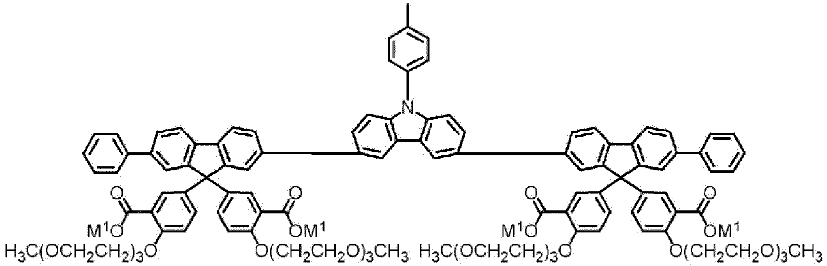

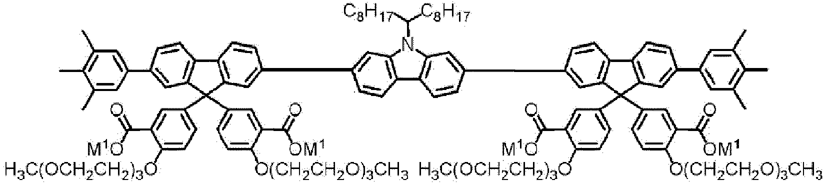

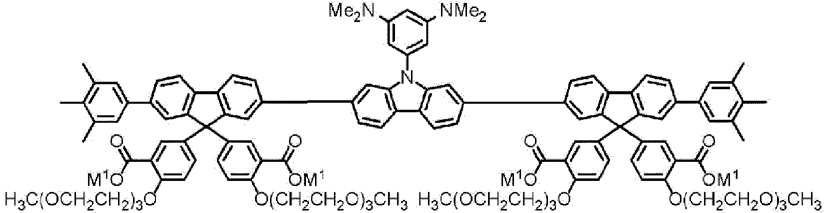

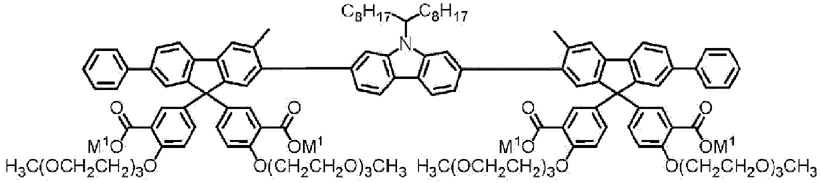

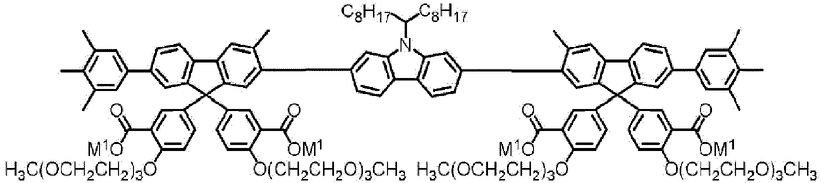

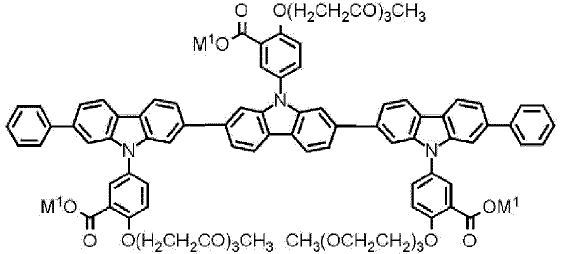

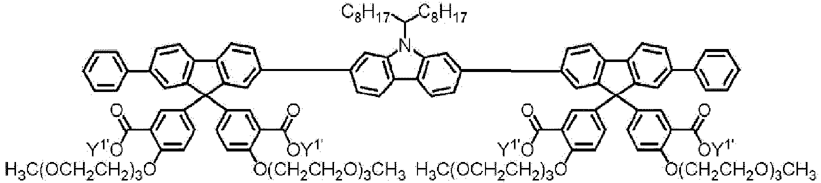

The compound represented by the formula (1) preferably has a symmetric structure, from the standpoint of synthesis.

The mother skeleton can be synthesized using reactions for forming a carbon-carbon bond such as, for example, Suzuki coupling, Negishi coupling, Stille coupling or Kumada coupling.

The concept of the synthesis method includes, for example, a method of constructing the skeleton from the center part (route 1), a method of constructing the skeleton from the outer part (route 2), a method combining route 1 and route 2 (route 3), and the like. The route 1 method includes, for example, the following methods (in the following explanations for routes 1 to 3, A, B, C, D and E each independently represent Ar¹ or a raw material compound that becomes Ar¹ by the above-described coupling reaction.):

route 1 (1) A + 2B → B-A-B + 2C → C-B-A-B-C + 2D → D-C-B-A-B-C-D + 2E → E-D-C-B-A-B-C-D-E

route 1 (2) A-A + 2B → B-A-A-B + 2C → C-B-A-A-B-C + 2D → D-C-B-A-A-B-C-D + 2E → E-D-C-B-A-A-B-C-D-E

The route 2 method includes, for example, the following methods.

route 2 (1) E + D → D-E + C → C-D-E + B → B-C-D-E ×2 + A → E-D-C-B-A-B-C-D-E route 2 (2) B-C-D-E ×2 + A-A → E-D-C-B-A-A-B-C-D-E

The route 3 method includes, for example, the following methods.

route 3 (1) B-A-B + 2C-D → D-C-B-A-B-C-D

route 3 (2) A-A + 2B-C → C-B-A-A-B-C

Based on the above methods, introduction of a reactive group necessary for the coupling reaction and necessary conversion of functional groups may be appropriately carried out.

The present invention also provides compounds represented by the formulae (11) to (13). These compounds are useful because they can be used as production intermediates for the compound represented by the formula (1).

Compound represented by the formula (11):

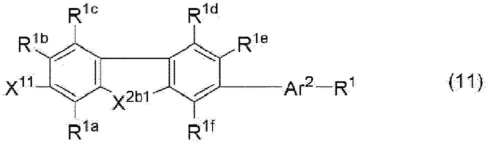

X^{2b1} is preferably a group represented by -C(R^{1g})₂-. may have a group represented by the above-described formula (2-1A), a group represented by the above-described formula (2-2A), a hydroxyl group or a mercapto group, in addition to substituents described in the explanation of common terms. It is preferable that at least one of R^{1g} is a group represented by the above-described formula (2-1), and it is more preferable that at least one of is a group represented by the above-described formula (2-3). In the above-described formulae (2-1) and (2-3), it is preferable that Y¹ is -CO₂Y¹', -SO₃Y¹', -SO₂Y¹',-P(=O)(OY¹') (O⁻) or -P(=O)(OY¹')₂, more preferably-CO₂Y¹'.

The halogen atom represented by X¹¹ includes a fluorine atom, a chlorine atom, a bromine atom and an iodine atom, and a chlorine atom, a bromine atom and an iodine atom are preferred from the standpoint of reactivity. The preferable ranges and examples of the alkyl group, the cycloalkyl group and the aryl group represented by R^{c2} in B(OR^{c2})₂ are the same as those described in the explanation of common terms. These groups optionally have a substituent. A plurality of R^{c2} may be the same or different and may be combined together to form a ring structure together with oxygen atoms to which they are attached.

R^{1a}, R^{1b}, R^{1c}, R^{1d}, R^{1e}, R^{1f}, R¹ and Ar² represent the same meaning as described above, and since synthesis is easy, R^{1a}, R^{1c}, R^{1d} and R^{1f} are each preferably a hydrogen atom.

The compound represented by the above-described formula (1-1) is preferably a compound represented by the formula (11-1), since synthesis is easy 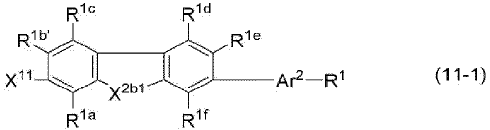 [wherein,
R^{1b}' represents an alkyl group, a cycloalkyl group, an aryl group or a monovalent heterocyclic group.
R^{1a}, R^{1c} to R^{1f}, R¹, Ar², X¹¹ and X^{2b1} represent the same meaning as described above.]

The compound represented by the above-described formula (11-1) can be synthesized by methods such as, for example, Suzuki coupling of a compound represented by the formula (12) with R¹-Ar²-B(OR^{c2})₂ (R^{c2} represents the same meaning as described above), Negishi coupling with R¹-Ar²-ZnX' (X' represents a halogen atom), Stille coupling with R¹-Ar²-SnR^{c3}₃ (R^{c3} represents an alkyl group) or Kumada coupling with R¹-Ar²-MgX' (X' represents a halogen atom). Since X¹² adjacent to R^{1b}' is lowered in reactivity due to steric hindrance by R^{1b}', X¹² adjacent to R^{1e} reacts selectively.

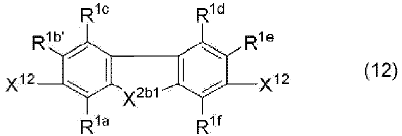

The preferable range of X^{2b1} is the same as the preferable range for the above-described formula (11).

The compound represented by the above-described formula (12) is preferably a compound represented by the formula (12-1), since synthesis is easy. 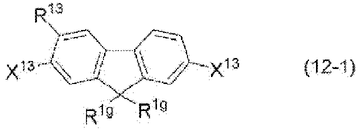 [wherein,
R^{1g} represents the same meaning as described above.
R¹³ represents an alkyl group, a cycloalkyl group, an aryl group or a monovalent heterocyclic group, and these groups optionally have a substituent.
X¹³ represents a chlorine atom, a bromine atom, an iodine atom or a group represented by B(OR^{c2})₂ (wherein, R^{c2} represents the same meaning as described above.).]

The preferable ranges and examples of the alkyl group, the cycloalkyl group, the aryl group and the monovalent heterocyclic group are the same as those described in the explanation of common terms. As the substituent which R¹³ optionally has, a substituent represented by the above-described formula (2-1A) or the above-described formula (2-2A) may be carried, in addition to substituents described in the explanation of common terms.

The compound represented by the above-described formula (12-1) can be synthesized, for example, by the Friedel-Crafts reaction of a compound represented by the formula (13) and a salicylic acid derivative [a compound represented by the formula (12-1A) is generated], and further, can also be synthesized by derivatization such as etherification and the like of the generated compound represented by the formula (12-1A) . 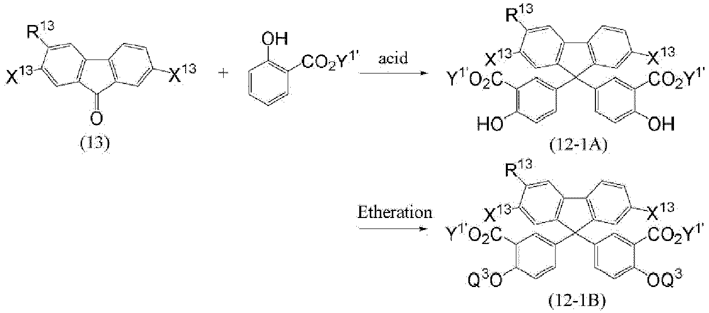 [wherein, R¹³, X¹³, Y¹' and Q³ represent the same meaning as described above.]

As the compound represented by the above-described formula (1-1), compounds represented by the following formulae are exemplified.

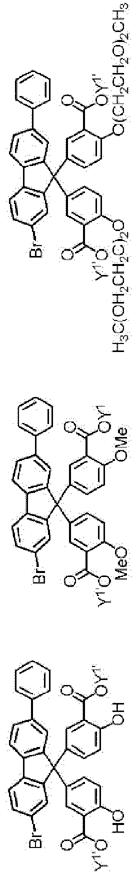

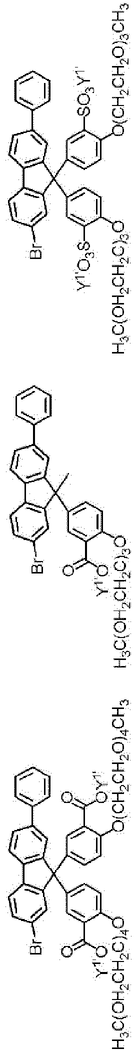

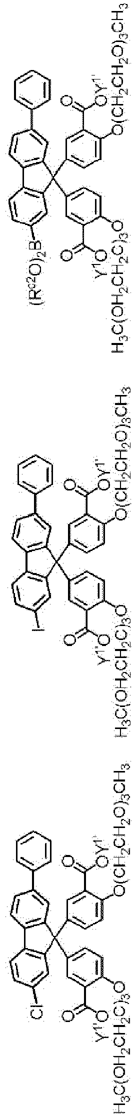

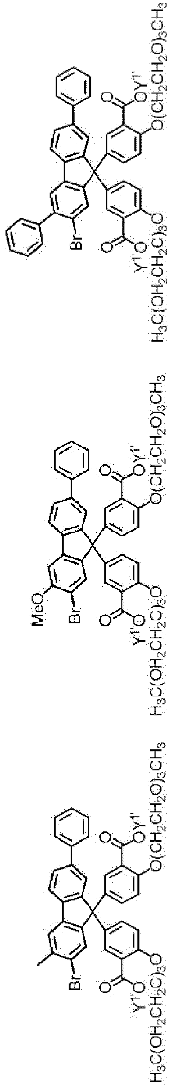

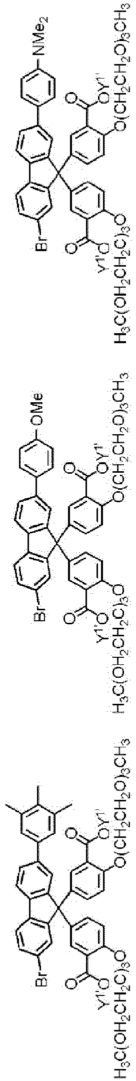

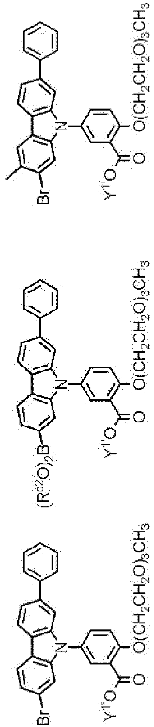

As the compound represented by the above-described formula (12), compound represented by the following formulae are exemplified.

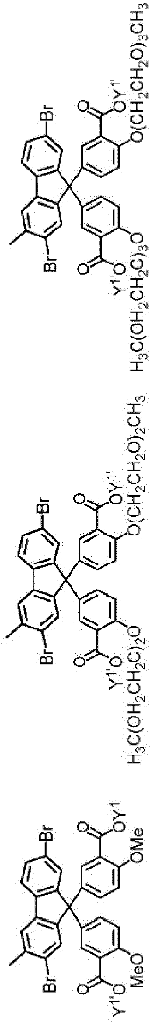

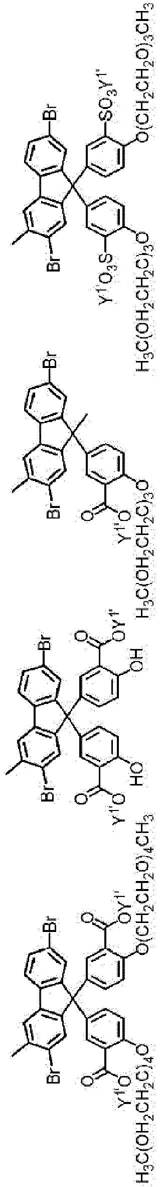

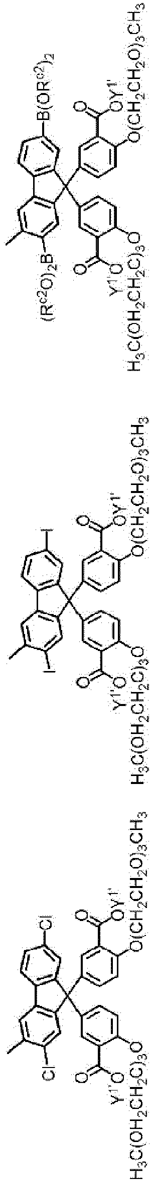

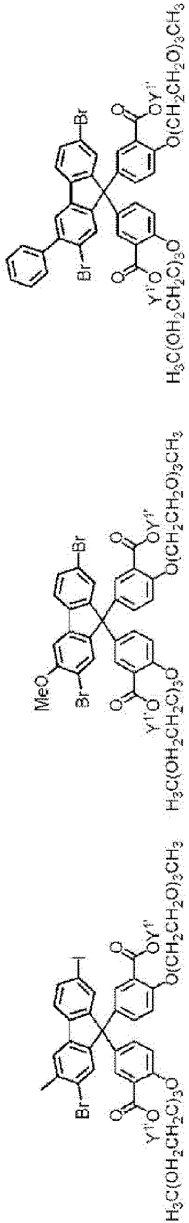

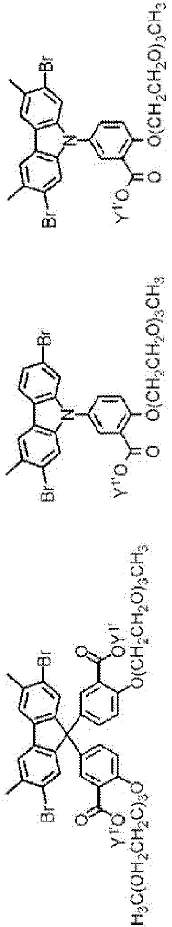

As the compound represented by the above-described formula (13), compound represented by the following formulae are exemplified.

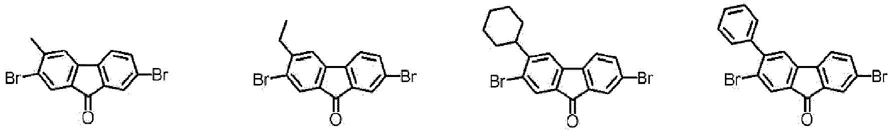

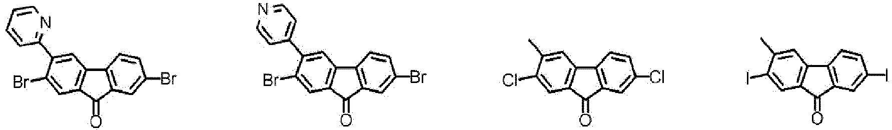

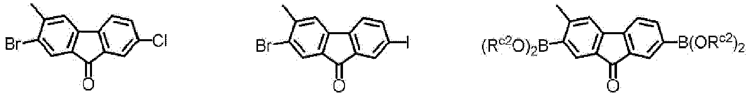

Composition comprising compound of the present invention:
The present invention provides a composition comprising a compound represented by the formula (1). The compound represented by the formula (1) is preferably used for producing each layer of a light emitting device. Hence, the composition of the present invention comprises the compound represented by the formula (1) of the present invention, and at least one compound selected from the group consisting of a hole transporting material, a hole injection material, an electron transporting material, an electron injection material, a light emitting material, an antioxidant and a solvent. The composition of the present invention can be formed into a film by an application method, and a composition containing at least one solvent is preferred.

As the solvent used in the composition of the present invention, highly-polar solvents are preferable, and it is more preferable to contain at least one protonic solvent. Since the compound represented by the formula (1) has high polarity, it shows high solubility in the highly-polar solvent. When the composition of the present invention is applied on an adjacent lower layer to form a film, if solubility in the adjacent lower layer is high, the adjacent lower layer is dissolved and a laminated structure cannot be formed, thus, it is preferable to use a solvent having low solubility in the adjacent lower layer.

The solvent used for film formation from a solution includes, for example, water, alcohols, fluorinated alcohols, ethers, esters, nitrile compounds, nitro compounds, halogenated alkyls, halogenated aryls, thiols, sulfides, sulfoxides, thioketones, amides and carboxylic acids, and it is preferable to contain at least one of water, alcohols, fluorinated alcohols, ethers, sulfoxides or amides. More specifically, exemplified as the solvent are water, methanol, ethanol, 2-propanol, 1-butanol, tert-butylalcohols, acetonitrile, 1,2-ethanediol, N,N-dimethylformamide, dimethyl sulfoxide, acetic acid, nitrobenzene, nitromethane, 1,2-dichloroethane, dichloromethane, chlorobenzene, bromobenzene, 1,4-dioxane, propylene carbonate, pyridine, and carbon disulfide, 2,2,3,3-tetrafluoropropanol, 1,1,1-trifluoro-2-propanol, 1,1,1,3,3,3-hexafluoro-2-propanol, 2,2,3,3,4,4-hexafluorobutanol, 2,2,3,3,4,4,4-heptafluoro-1-butanol, 2,2,3,3,3-pentafluoro-1-propanol, 3,3,4,4,5,5,5-heptafluoro-2-pentanol, 2,2,3,3,4,4,5,5-octafluoro-1-pentanol, 3,3,4,4,5,5,6,6,6-nonafluoro-1-hexanol and 2,2,3,3,4,4,5,5,6,6,7,7-dodecafluoroheptanol. These solvents may be used singly or in combination of two or more.

M¹ is preferably an alkali metal cation, an alkaline earth metal cation or an ammonium cation optionally having a substituent, more preferably an alkali metal cation or an alkaline earth metal cation, and further preferably an alkali metal cation, from the standpoint of luminance life when used as electron injection and transporting layers in a device. As described above, a compound in which M¹ is an alkali metal cation, an alkaline earth metal cation or an ammonium cation optionally having a substituent can be prepared from a compound in which Y¹ is a hydrocarbon group optionally having a substituent, a heterocyclic group optionally having a substituent or a hydrogen atom, and they can be reacted in the solvent used for application and film formation, and used as it is for fabrication of a device

A compound in which Y¹ is a hydrocarbon group optionally having a substituent, a heterocyclic group optionally having a substituent or a hydrogen atom can be reacted with a reagent such as a metal hydroxide, a metal carbonate or an alkylammonium hydroxide in the solvent used for application and film formation, to prepare a product.

A compound in which Y¹ is a hydrogen atom is preferable since the reaction progresses approximately quantitatively around room temperature (25°C) since it is a neutralization reaction. The generated water and carbonic acid can be distilled off in application and film formation.

In the case of a compound in which Y¹ is a hydrocarbon group optionally having a substituent or a heterocyclic group optionally having a substituent, it is preferable to react it at 0°C to the boiling point of the solvent. In this operation, a hydroxide of the corresponding hydrocarbon compound optionally having a substituent and a hydroxide of the corresponding heterocyclic compound optionally having a substituent are by-produced. The number of carbon atoms of the hydrocarbon group optionally having a substituent or the heterocyclic group optionally having a substituent represented by Y¹ is preferably 1 to 10, and more preferably 1 to 8 including the number of carbon atoms of the substituent, since it can be distilled off after film formation.

It is preferable for the above-described formula (2-1) that n1 is 0 and Y¹ is -CO₂⁻ or -CO₂Y¹', from the standpoint of luminance life when used as a composition for electron injection and transporting layers in a device. It is preferable that Y¹' is a hydrocarbon group optionally having a substituent, a heterocyclic group optionally having a substituent or a hydrogen atom, or M¹ is an alkali metal cation or an alkaline earth metal cation, it is more preferable that Y¹' is a hydrocarbon group optionally having a substituent, a heterocyclic group optionally having a substituent or a hydrogen atom, or M¹ is an alkali metal cation, it is further preferable that Y¹' is a hydrogen atom, or M¹ is an alkali metal cation, and it is particularly preferable that M¹ is an alkali metal cation.

The examples and preferable ranges of the metal hydroxide, the metal carbonate and the alkylammonium salt are the same as described above.

### Light Emitting Device

The present invention provides a light emitting device comprising a compound represented by the above-described formula (1). In the light emitting device of the present invention, the organic layer (that is, a layer containing a compound represented by the formula (1)) is preferably at least one layer selected from the group consisting of an electron injection layer and and electron transporting layer, and more preferably an electron transporting layer. The light emitting device of the present invention may further have a substrate.

Preferable embodiments of the light emitting device of the present invention are a light emitting device in which an anode is provided on a substrate, a light emitting layer is laminated on the upper layer, a layer containing a compound represented by the formula (1) is laminated on the upper layer, and further, a cathode is laminated on the upper layer, and a light emitting device in which a cathode is provided on a substrate, a layer containing a compound represented by the formula (1) is laminated on the upper layer, a light emitting layer is laminated on the upper layer, and further, an anode is laminated on the upper layer.

In these embodiments, layers having other functions such as protective layers, buffer layers, reflective layers, sealing layers (sealing film, sealing substrate, etc.) and the like may be further provided. Further, the layer containing a compound represented by the formula (1) may be a single-layered layer or a multi-layered layer.

The light emitting device of the present invention may be any of a bottom emission type, a top emission type, and a double-sided lighting type.

From the viewpoint of hole injectability and hole transportability, the light emitting device of the present invention preferably has at least one of a hole injection layer and a hole transporting layer between the anode and the light emitting layer.

From the viewpoint of electron injectability and electron transportability, the light emitting device of the present invention preferably has at least one of an electron injection layer and an electron transporting layer between the cathode and the light emitting layer.

Examples of the material of the electron transporting layer and the electron injection layer include an electron transporting material and an electron injection material, respectively, in addition to the compound represented by the formula (1).

Examples of the materials of the hole transporting layer, the hole injection layer and the light emitting layer include a hole transporting material, a hole injection material and a light emitting material, respectively.

### Hole Transporting Material

When a hole injection material, an electron transporting material, an electron injection material and a light emitting material are soluble in a solvent used in forming layers adjacent to a hole transporting layer, an electron transporting layer and a light emitting layer, respectively, in fabricating a light emitting device, it is preferable that the material has a crosslinkable group for avoiding the material from being dissolved in the solvent. After forming each layer using the material having a crosslinkable group, the crosslinkable group can be cross-linked to insolubilize the layer.

The method for forming each layer such as a light emitting layer, a hole transporting layer, an electron transporting layer, a hole injection layer, an electron injection layer and the like in a light emitting device of the present invention includes, for example, a vacuum vapor deposition method from a powder and a method by film formation from a solution or melted state when a low molecular weight compound is used, and includes, for example, a method by film formation from a solution or melted state when a polymer compound is used. Of them, the method by film formation from a solution is preferable as the method for forming each layer.

The order, number and thickness of layers to be laminated may be adjusted in consideration of light emission efficiency and luminance life.

The solvent used for film formation from a solution includes water, alcohols, fluorinated alcohols, ethers, esters, nitrile compounds, nitro compounds, halogenated alkyls, halogenated aryls, thiols, sulfides, sulfoxide, thioketones, amides and carboxylic acids. These solvents may be used singly or in combination of two or more.

The method for film formation from a solution includes application methods such as, for example, a spin coat method, a casting method, a micro gravure printing method, a gravure printing method, a bar coat method, a roll coat method, a wire bar coat method, a dip coat method, a slit coat method, a cap coat method, a spray coat method, a screen printing method, a flexo printing method, an offset printing method, an inkjet printing method, a nozzle coat method and the like.

The preferable layer constitution of the light emitting device of the present invention includes, for example, the following constitutions. In the constitutions, the layer containing a compound represented by the formula (1) can be used as an electron injection layer and/or an electron transporting layer.
(a) anode-hole injection layer-light emitting layer-cathode
(b) anode-light emitting layer-electron injection layer-cathode
(c) anode-hole injection layer-light emitting layer-electron injection layer-cathode
(d) anode-hole injection layer-hole transporting layer-light emitting layer-cathode
(e) anode-hole injection layer-hole transporting layer-light emitting layer-electron injection layer-cathode
(f) anode-light emitting layer-electron transporting layer-electron injection layer-cathode
(g) anode-hole injection layer-light emitting layer-electron transporting layer-electron injection layer-cathode
(h) anode-hole injection layer-hole transporting layer-light emitting layer-electron transporting layer-electron injection layer-cathode

The light emitting device of the present invention may be further provided with an insulating layer adjacent to the electrode in order to improve the adhesion to the electrode and improve the charge injection from the electrode, and further, a thin buffer layer may be inserted at the interface between the hole transporting layer, the electron transporting layer or the light emitting layer in order to improve the adhesion of the interface and prevent mixing. The order and number of layers to be laminated and the thickness of each layer may be adjusted in consideration of light emission efficiency and luminance life.

The constitution of the light emitting device of the present invention will be illustrated in detail below.

### [Substrate]

The substrate which the light emitting device of the present invention can have may be one which does not chemically change in forming an electrode and forming an organic layer, and for example, substrates made of glass, plastic, polymer film, metal film, silicon and the like, and substrates obtained by laminating them, are used.

### [Hole injection layer]

In the light emitting device of the present invention, suitably used as the hole injection material are carbazole and derivatives thereof, triazole and derivatives thereof, oxazole and derivatives thereof, oxadiazole and derivatives thereof, imidazole and derivatives thereof, fluorene and derivatives thereof, polyarylalkane and derivatives thereof, pyrazoline and derivatives thereof, pyrazolone and derivatives thereof, phenylenediamine and derivatives thereof, arylamines and derivatives thereof, starburst type amines, phthalocyanine and derivatives thereof, amino-substituted chalcones and derivatives thereof, styrylanthracene and derivatives thereof, fluorenone and derivatives thereof, hydrazone and derivatives thereof, stilbene and derivatives thereof, silazane and derivatives thereof, aromatic tertiary amine compounds, styrylamine compounds, aromatic dimethylidyne compounds, porphyrin compounds, polysilane compounds, poly(N-vinylcarbazole) and derivatives thereof, organic silanes and derivatives thereof, and polymers containing them; electrically conductive metal oxides such as vanadium oxide, tantalum oxide, tungsten oxide, molybdenum oxide, ruthenium oxide, aluminum oxide and the like; electrically conductive polymers and oligomers such as polyalinines, aniline copolymers, thiophene oligomers, polythiophenes and the like; organic conductive materials such as poly(3,4-ethylenedioxythiophene)-polystyrenesulfonic acid, polypyrrole and the like, and polymers containing them; amorphous carbon; accepting organic compounds such as tetracyanoquinodimethane and derivatives thereof (for example, 2,3,5,6-tetrafluoro-7,7,8,8-tetracyanoquinodimethane), 1,4-naphthoquinone and derivatives thereof, diphenoquinone and derivatives thereof, polynitro compounds and the like; silane coupling agents such as octadecyltrimethoxysilane and the like.

The hole injection materials may be used singly or in combination of two or more. The hole injection layer may have a single-layered structure compose only of the above-described material or may have a multi-layered structure composed of several layers having the same formulation or different formulations.

The method for forming the hole injection layer is as described above, and it is also possible to form a hole injection layer using a mixed solution prepared by dispersing a polymer compound binder and a low molecular weight organic material.

As the polymer compound binder to be mixed, one not extremely inhibiting charge transportation is preferable, and a compound that does not strongly absorb visible light is preferably used. As this polymer compound binder, exemplified are poly(N-vinylcarbazole), polyaniline and derivatives thereof, polythiophene and derivatives thereof, poly(p-phenylenevinylene) and derivatives thereof, poly(2,5-thienylenevinylene) and derivatives thereof, polycarbonate, polyacrylate, polymethyl acrylate, polymethyl methacrylate, polystyrene, polyvinyl chloride and polysiloxane.

When an organic compound layer such as a hole transporting layer or a light emitting layer is formed following the hole injection layer, particularly when both layers are formed by an application method, the layer applied first may dissolve in the solvent contained in the solution of the layer applied later, making it difficult to fabricate a laminated structure. In this case, a method of insolubilizing the lower layer in a solvent can be used. The method for insolubilizing in a solvent includes a method of attaching a crosslinkable group to a polymer compound and cross-linking it to insolubilize it, a method in which a low molecular weight compound having a crosslinkable group having an aromatic ring typified by an aromatic bisazide is mixed as a cross-linking agent, and it is cross-linked to insolubilize it, a method in which a low molecular weight compound having a crosslinkable group having no aromatic ring typified by an acrylate group is mixed as a cross-linking agent, and it is cross-linked to insolubilize it, a method in which the lower layer is exposed to ultraviolet light to be crosslinked to insolubilize against an organic solvent used in producing the upper layer, a method of heating the lower layer to crosslink it, to insolubilize against an organic solvent used in producing the upper layer, and the like. The temperature when heating the lower layer is usually 100°C to 300°C, and the time is usually 1 minute to 1 hour.

As the method of laminating without dissolving the lower layer not by cross-linking, there is a method of using solutions having different polarities for producing adjacent layers, and there is, for example, a method in which a water-soluble polymer compound is used for the lower layer and an oil-soluble polymer compound is used for the upper layer such that the lower layer does not dissolve even when applied.

The thickness of the hole injection layer is usually 1 nm to 1 µm.

### [Hole transporting layer]

In the light emitting device of the present invention, the hole transporting material is classified into low molecular weight compounds and polymer compounds, and polymer compounds are preferable, polymer compounds having a crosslinkable group being more preferred.

The polymer compound includes, for example, polyvinylcarbazole and derivatives thereof; polyarylenes having an aromatic amin structure in the side chain or main chain, and derivatives thereof. The polymer compound may be a compound to which an electron accepting site is bonded. The electron accepting site includes, for example, fullerenes, tetrafluorotetracyanoquinodimethane, tetracyanoethylene, trinitrofluorenone and the like.

The hole transporting materials may be used singly or in combination of two or more.

If the lower layer is dissolved in the solvent contained in the solution of the layer to be applied later in forming an organic layer such as a light emitting layer and the like by an application method following the hole transporting layer, the lower layer can be insolubilized in a solvent by the same methods as exemplified for the method of forming the hole injection layer.

The thickness of the hole transporting layer is usually 1 nm to 1 µm.

### [Light emitting layer]

In the light emitting device of the present invention, the light emitting material may be used alone, but is usually used in combination with the host material. The host material is a material for the purpose of suppressing a decrease in luminous efficiency due to aggregation of the light emitting material, for the purpose of transporting electric charges and achieving a decrease in driving voltage, and the like.

In the light emitting device of the present invention, the light emitting material is classified into low molecular weight compounds and polymer compounds. The light emitting material may have a crosslinkable group.

The low molecular weight compound includes, for example, naphthalene and derivatives thereof, anthracene and derivatives thereof, perylene and derivatives thereof, and triplet light emitting complexes having iridium, platinum or europium as the central metal.

The polymer compound includes polymer compounds containing, for example, a phenylene group, a naphthalenediyl group, a fluorenediyl group, a phenanthrenediyl group, a dihydrophenanthrenediyl group, a group represented by the formula (3), a carbazolediyl group, a phenoxazinediyl group, a phenothiazinediyl group, an anthracenediyl group, a pyrenediyl group and the like.

The light emitting material may contain a low molecular weight compound and a polymer compound.

As the triplet light emitting complex, iridium complexes such as metal complexes represented by the formulae Ir-1 to Ir-3, and Ir-A are preferable. 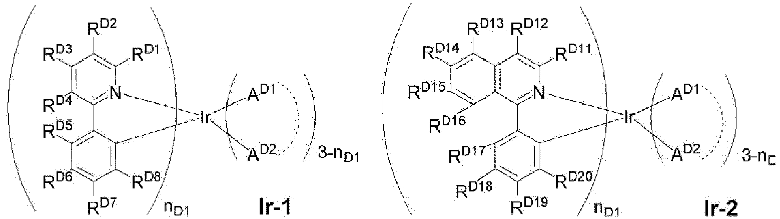 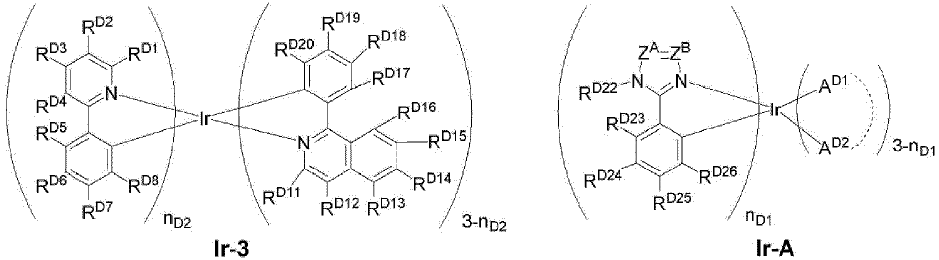 [wherein,
R^{D1} to R^{D8}, R^{D11} to R^{D26} each independently represent a hydrogen atom, an alkyl group, a cycloalkyl group, an alkoxy group, a cycloalkoxy group, an aryl group, an aryloxy group, a monovalent heterocyclic group or a halogen atom, and these groups optionally have a substituent. When a plurality of R^{D1} to R^{D8}, R^{D11} to R^{D26} are present, they may be the same or different at each occurrence.
Z^{A} and Z^{B} each independently represent a group represented by -CR^{D21}= or a group represented by -N=. When a plurality of Z^{A} and Z^{B} are present, they may be the same or different. R^{D21} each independently represent a hydrogen atom, an alkyl group, a cycloalkyl group, an alkoxy group, a cycloalkoxy group, an aryl group, an aryloxy group, a monovalent heterocyclic group or a halogen atom, and these groups optionally have a substituent. When a plurality of R^{D21} are present, they may be the same or different.
-A^{D1}---A^{D2}- represents an anionic bidentate ligand, A^{D1} and A^{D2} each independently represent a carbon atom, an oxygen atom or a nitrogen atom bonding to an iridium atom, and these atoms may be ring-constituent atoms. When a plurality of -A^{D1}---A^{D2}- are present, they may be the same or different.
n_{D1} represents 1, 2 or 3, and n_{D2} represents 1 or 2.]

As the iridium complex represented by Ir-A, iridium complexes represented by the formulae Ir-4 to Ir-6 are preferable. 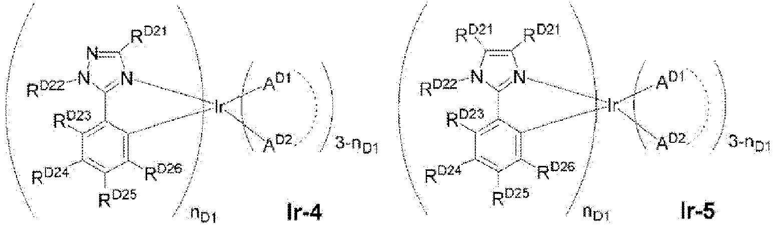 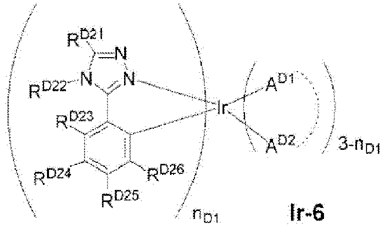 [wherein,
R^{D1} to R^{D8}, R^{D11} to R^{D26}, Z^{A}, Z^{B} , -A^{D1}---A^{D2}- and n_{D1} represent the same meaning as described above.]

In the metal complex represented by the formula Ir-1, at least one of R^{D1} to R^{D8} is preferably a group represented by the formula (D-A).

In the metal complex represented by the formula Ir-2, at least one of R^{D11} to R^{D20} is a group represented by the formula (D-A).

In the metal complex represented by the formula Ir-3, at least one of R^{D1} to R^{D8} and R^{D11} to R^{D20} is a group represented by the formula (D-A).

In the metal complex represented by the formula Ir-A, the formula Ir-4, the formula Ir-5 or the formula Ir-6, at least one of R^{D21} to R^{D26} is a group represented by the formula (D-A). 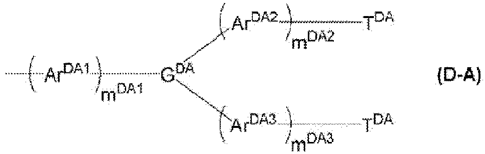 [wherein,
m^{DA1} to m^{DA3} each independently represent an integer of 0 or more.
G^{DA} represents a nitrogen atom, an aromatic hydrocarbon group or a heterocyclic group, and these groups optionally have a substituent.
Ar^{DA1} to Ar^{DA3} each independently represent an arylene group or a divalent heterocyclic group, and these groups optionally have a substituent. Whe a plurality of Ar^{DA1} to Ar^{DA3} are present, they may be the same or different at each occurrence.
T^{DA} represents an aryl group or a monovalent heterocyclic group, and these groups optionally have a substituent. A plurality of T^{DA} may be the same or different.]
m^{DA1} to m^{DA3} are each preferably an integer of 5 or less, and more preferably 0 or 1.

The anionic bidentate ligand represented by -A^{D1}-A^{D2}- includes, for example, ligands represented by the following formulae. 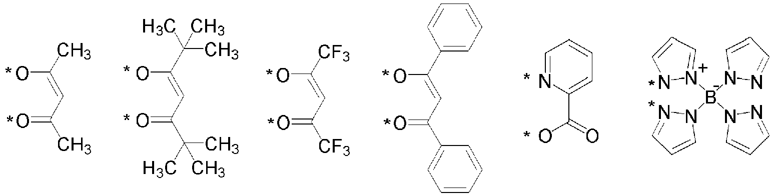 [wherein, * represents a site binding to Ir.]

The metal complex represented by the formula Ir-1 is preferably a metal complex represented by the formulae Ir-11 to Ir-13. The metal complex represented by the formula Ir-2 is preferably a metal complex represented by the formula Ir-21. The metal complex represented by the formula Ir-3 is preferably a metal complex represented by the formulae Ir-31 to Ir-33. The metal complex represented by the formula Ir-4 is preferably an iridum complex represented by the formulae Ir-41 to Ir-43. The metal complex represented by the formula Ir-5 is preferably an iridum complex represented by the formulae Ir-51 to Ir-53. The metal complex represented by the formula Ir-6 is preferably an iridum complex represented by the formulae Ir-61 to Ir-63. 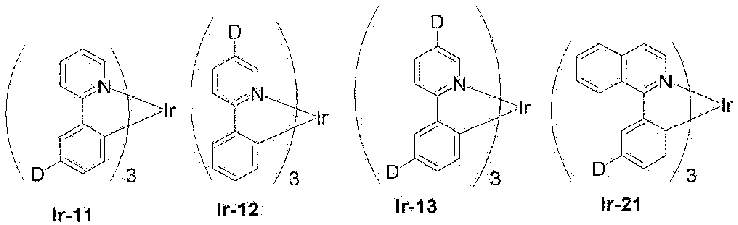 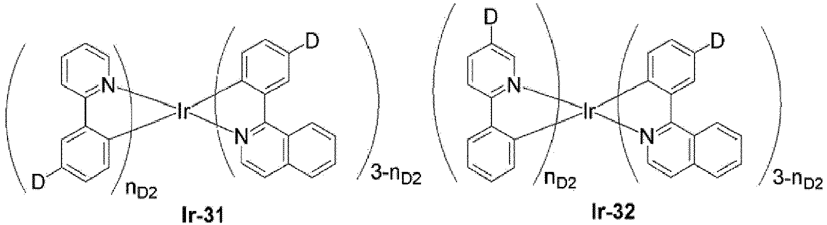 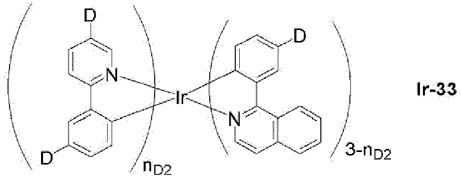 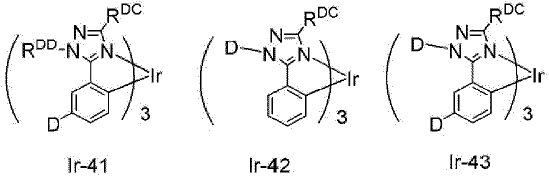 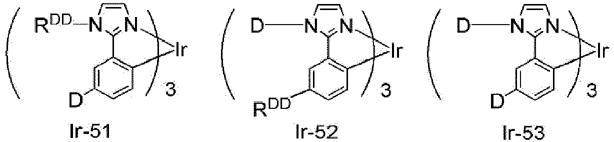 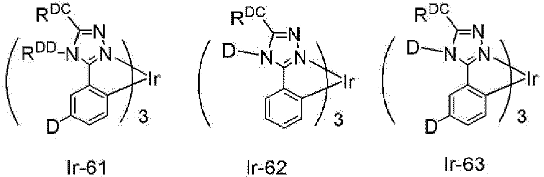 [wherein, n_{D2} represents 1 or 2. D represents a group represented by the formula (D-A). A plurality of D may be the same or different. R^{DC} represents a hydrogen atom, an alkyl group, a cycloalkyl group, an aryl group or a monovalent heterocyclic group, and these groups optionally have a substituent. A plurality of R^{DC} may be the same or different. R^{DD} represents an alkyl group, a cycloalkyl group, an aryl group or a monovalent heterocyclic group, and these groups optionally have a substituent. A plurality of R^{DD} may be the same or different.]

The triplet light emitting complex includes, for example, metal complexes shown below.

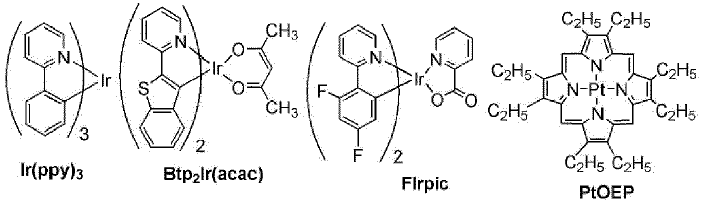

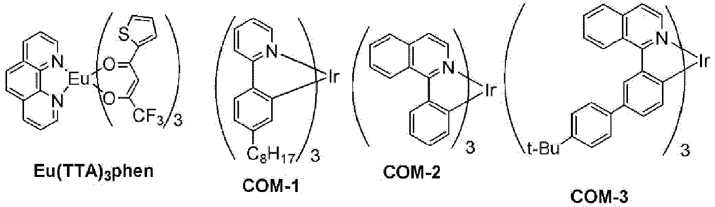

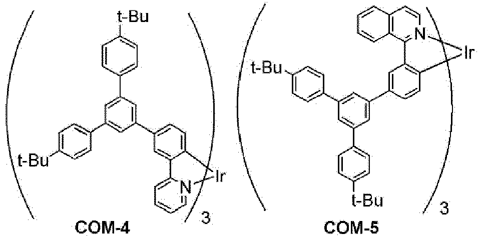

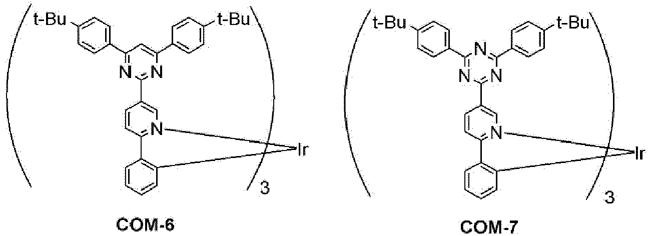

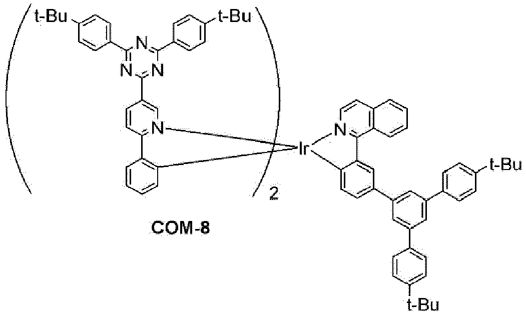

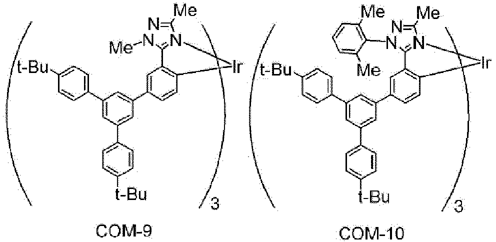

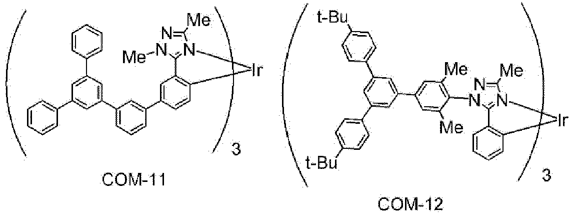

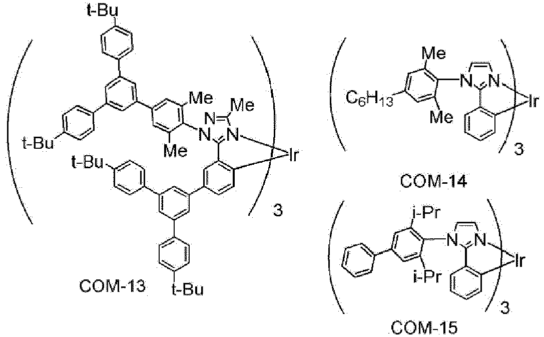

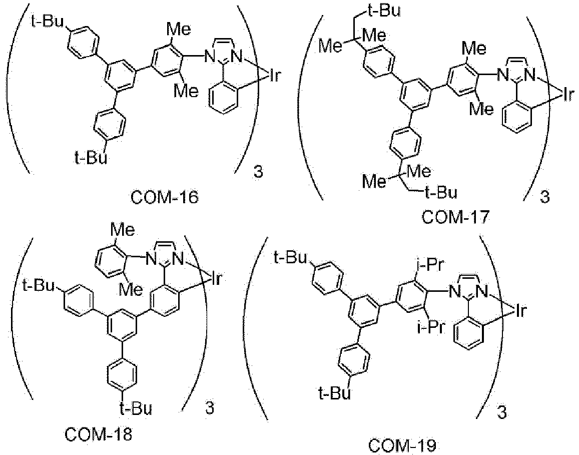

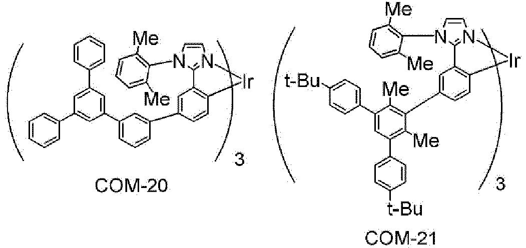

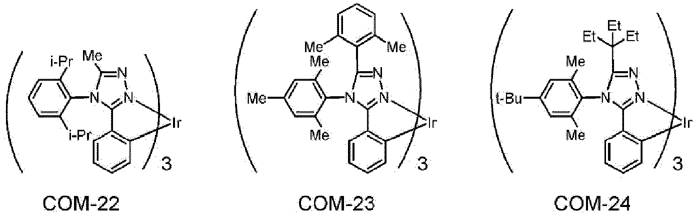

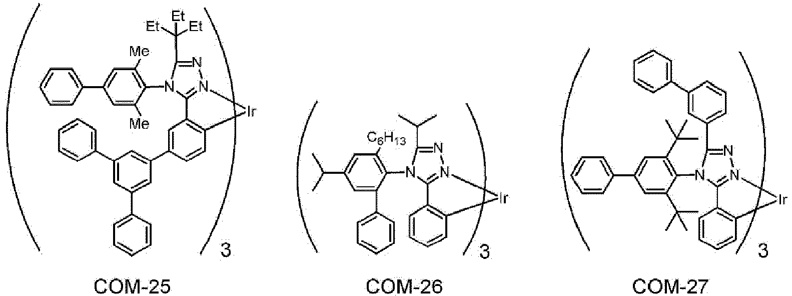

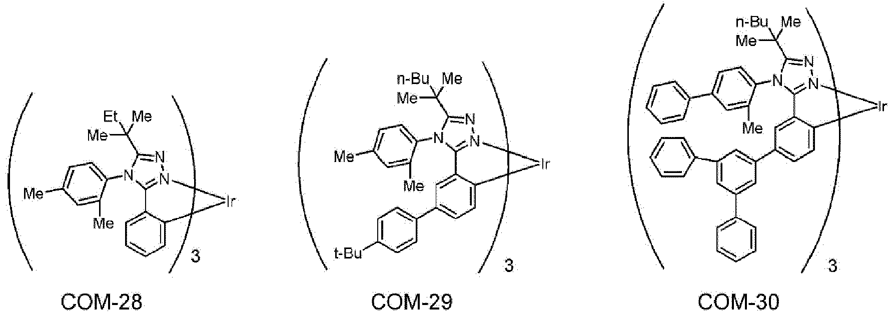

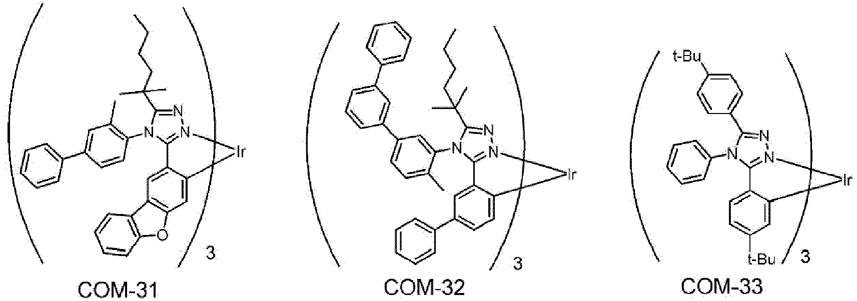

The light emitting materials may be used singly or in combination of two or more.

The host material is classified into low molecular weight compounds and polymer compounds.

When a metal complex is used in the light emitting material, the host material is preferably a compound of which lowest excited triplet state (T₁) is at energy level not lower than the lowest excited triplet state (T₁) of the metal complex, since the light emitting device is excellent in external quantum efficiency.

When a metal complex is used in the light emitting material, the host material is preferably a compound showing solubility in a solvent capable of dissolving the metal complex, since the light emitting device can be fabricated by an application method.

The low molecular weight compound used as the host material includes low molecular weight compounds exemplified as the above-described hole transporting material, low molecular weight compounds exemplified as the above-described electron transporting material, and the like, and is preferably a compound having higher excitation state energy than the excitation state energy of the light emitting material to be used in combination. When the light emitting material utilizes light emission from the lowest excited triplet state, it is preferable that the energy of the lowest excited triplet state of the host material is not lower than the energy of the lowest excited triplet state of the light emitting material.

Of them, preferable as the low molecular weight compound used as the host material are compounds having a carbazole structure, compounds having a triarylamine structure, compounds having a phenanthroline structure, compounds having a triaryltriazine structure, compounds having an azole structure, compounds having a benzothiophene structure, compounds having a benzofuran structure, compounds having a fluorene structure and compounds having a spirofluorene structure.

The low molecular weight compound used as the host material includes, for example, the following compounds.

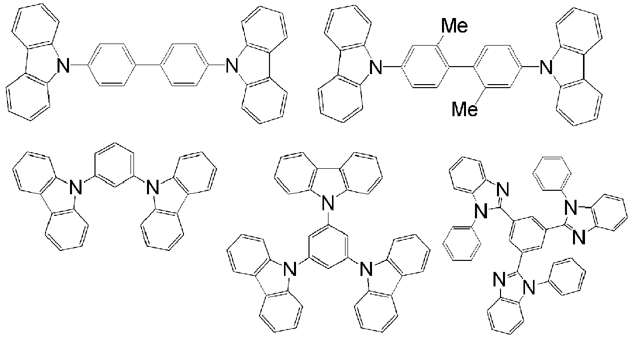

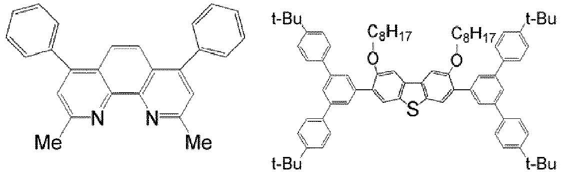

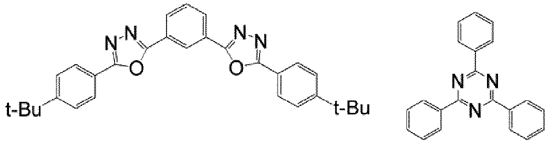

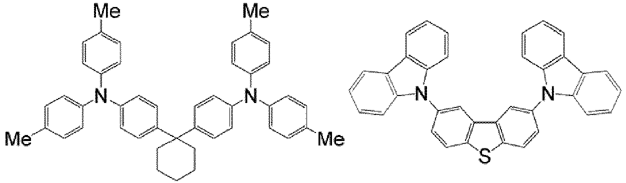

The polymer compound used as the host material (hereinafter, referred to as "polymer host") includes, for example, polymer compounds described as the hole transporting material or the electron transporting material.

The polymer host is preferably a polymer compound containing a constitutional unit represented by the above-described formula (X) or the above-described formula (Y). The descriptions and examples of the polymer compound containing a constitutional unit represented by the formula (X) or the formula (Y) are the same as described above.

The constitutional unit represented by the formula (X) or the formula (Y) may each be contained singly or in combination of two or more in the polymer host.

The polymer host can be produced by known polymerization methods described in Chemical Reviews (Chem. Rev.), vol. 109, pp. 897-1091 (2009) and the like, for example, methods of polymerizing by the coupling reaction using a transition metal catalyst such as Suzuki reaction, Yamamoto reaction, Buchwald reaction, Stille reaction, Negishi reaction, Kumada reaction and the like.

In the above-described polymerization method, the method of charging monomers includes a method of charging the entire amount of monomers into the reaction system at once, a method in which a part of the monomers is charged and reacted, and then the remaining monomers are charged in a batch, continuously or dividedly, a method of charging monomers continuously or dividedly, and the like.

The transition metal catalyst includes, for example, palladium catalysts and nickel catalysts.

The post treatment of the polymerization reaction is performed using known methods, for example, a method of removing water-soluble impurities by liquid separation, a method in which the reaction liquid after the polymerization reaction is added to a lower alcohol such as methanol and the like, and the deposited precipitate is filtrated before drying, and the like, singly or in combination. Whe the purity of the polymer host is low, it can be purified by usual methods such as, for example, crystallization, reprecipitation, continuous extraction with a Soxhlet extractor, column chromatography and the like.

When the light emitting material and the host material are used in combination, the content of the light emitting material is usually 0.01 to 80 parts by weight, preferably 0.05 to 40 parts by weight, more preferably 0.1 to 20 parts by weight, and further preferably 1 to 20 parts by weight, when the sum of the light emitting material and the host material is taken as 100 parts by weight.

The light emitting layer may have a single-layered structure composed of one or more kinds of the light emitting materials, or may have a multi-layered structure composed of several layers having the same formulation or different formulations.

If the lower layer is dissolved in the solvent contained in the solution of the layer to be applied later in forming an organic compound layer such as an electron transporting layer and the like by an application method following the light emitting layer, the lower layer can be insolubilized in a solvent by the same methods as exemplified for the method of forming the hole injection layer.

The thickness of the light emitting layer is usually 5 nm to 1 µm.

### [Electron transporting layer]

Known materials can be used as the electron transporting material other than a compound represented by the formula (1) in the light emitting device of the present invention, and the material includes triazole and derivatives thereof, oxazole and derivatives thereof, oxadiazole and derivatives thereof, imidazole and derivatives thereof, fluorene and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone and derivatives thereof, anthraquinodimethane and derivatives thereof, anthrone and derivatives thereof, thiopyrandioxide and derivatives thereof, carbodiimide and derivatives thereof, fluorenylidenemethane and derivatives thereof, distyrylpyrazine and derivatives thereof; aromatic ring tetracarboxylic acid anhydrides such as naphthalene, perylene and the like; phthalocyanine and derivatives thereof; metal complexes of 8-quinolinol and derivatives thereof, and metal phthalocyanines; various metal complexes typified by metal complexes having benzoxazole or benzothiazole as a ligand; organic silanes and derivatives thereof, metal complexes of 8-hydroxyquinoline and derivatives thereof, polyquinoline and derivatives thereof, polyquinoxaline and derivatives thereof, polyfluorene and derivatives thereof, and the like. Of them, triazole and derivatives thereof, oxadiazole and derivatives thereof, benzoquinone and derivatives thereof, anthraquinone and derivatives thereof, and metal complexes of 8-hydroxyquinoline and derivatives thereof, polyquinoline and derivatives thereof, polyquinoxaline and derivatives thereof, and polyfluorene and derivatives thereof are preferable.

The electron transporting materials may be used singly or in combination of two or more. The electron transporting layer may have a single-layered structure composed of one or more types of the electron transporting materials or may have a multi-layered structure composed of several layers having the same formulation or different formulations.

If the lower layer is dissolved in the solvent contained in the solution of the layer to be applied later in forming an organic compound layer such as an electron injection layer and the like by an application method following the electron transporting layer, the lower layer can be insolubilized in a solvent by the same methods as exemplified for the method of forming the hole injection layer.

The thickness of the electron transporting layer is usually 1 nm to 1 µm.

### [Electron injection layer]

Known compounds can be used as the electron injection material other than a compound represented by the formula (1) in the light emitting device of the present invention, and the material includes triazole and derivatives thereof, oxazole and derivatives thereof, oxadiazole and derivatives thereof, imidazole and derivatives thereof, fluorene and derivatives thereof, benzoquinone and derivatives thereof, naphthoquinone and derivatives thereof, anthraquinone and derivatives thereof, tetracyanoanthraquinodimethane and derivatives thereof, fluorenone and derivatives thereof, diphenyldicyanoethylene and derivatives thereof, diphenoquinone and derivatives thereof, anthraquinodimethane and derivatives thereof, anthrone and derivatives thereof, thiopyrandioxide and derivatives thereof, carbodiimide and derivatives thereof, fluorenylidenemethane and derivatives thereof, distyrylpyrazine and derivatives thereof; aromatic ring tetracarboxylic acid anhydrides such as naphthalene, perylene and the like; phthalocyanine and derivatives thereof; metal complexes of 8-quinolinol and derivatives thereof, and metal phthalocyanines; various metal complexes typified by metal complexes having benzoxazole or benzothiazole as a ligand; organic silanes derivatives and the like.

The electron injection materials may be used singly or in combination of two or more. The electron injection layer may have a single-layered structure composed only of the electron injection material or may have a multi-layered structure composed of several layers having the same formulation or different formulations.

The thickness of the electron injection layer is usually 1 nm to 1 µm.

The electron injection and transporting compounds may each be used singly or in combination, and it is preferable to contain two or more types of compounds containing at least one type of the compound of the present invention.

That is, it is considered that when two or more types of electron injection and transporting compounds are contained in the electron injection and transporting layers in injecting electrons from a cathode into a light emitting layer, the electron injection and transporting compound having a low LUMO value receives electrons from the layer adjacent to the cathode, electrons are transferred between two compounds in the electron injection and transporting layer, and electrons are transported from the electron injection and transporting compound having a large LUMO value into the light emitting layer, thus, the electron injection barrier decreases, and the device characteristics are improved. It is preferable to contain two or more types of the compounds of the present invention.

When two or more types of the electron injection and transporting compounds are mixed, the mixing ratio is usually 1% by mol or more for each component, and preferably 10% by mol or more for each component.

### [Anode]

The material of the anode includes, for example, electrically conductive metal oxides and semitransparent metals, preferably includes indium oxide, zinc oxide, tin oxide; electrically conductive compounds such as indium-tin-oxide (ITO), indium-zinc-oxide and the like; argentine-palladium-copper (APC) complex; NESA, gold, platinum, silver and copper. The anode may have a single-layered structure composed of one or more types of these materials or may have a multi-layered structure composed of several layers having the same formulation or different formulations.

As the method for fabricating the anode, known methods can be used, and the method includes a vacuum vapor deposition method, a sputtering method, an ion plating method, a plating method, a method by film formation from a solution (a mixed solution with a polymer binder may be used), and the like.

The thickness of the anode is usually 10 nm to 10 µm.

After fabricated by the above-described method, the anode may be surface-treated with a solution containing an electron accepting compound such as UV ozone, silane coupling agents 2,3,5,6-tetrafluoro-7,7,8,8-tetracyanoquinodimethane and the like, to improve electrical connection to a layer in contact with the anode.

The anode may take a single-layered structure or a multi-layered structure.

### [Cathode]

The material of the cathode includes, for example, metals such as lithium, sodium, potassium, rubidium, cesium, beryllium, magnesium, calcium, strontium, barium, aluminum, zinc, indium and the like; alloys composed of two or more of them; alloys composed of one or more of them and one or more of silver, copper, manganese, titanium, cobalt, nickel, tungsten and tin; and graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy, a calcium-aluminum alloy, metal nano particles, metal nano wires, and nano particles of electrically conductive metal oxides.

As the method for fabricating the cathode, known methods can be used, and exemplified are a vacuum vapor deposition method, a sputtering method, an ion plating method and a method by film formation from a solution (a mixed solution with a polymer binder may be used). When the cathode is made of metal nano particles, metal nano wires or electrically conductive metal oxide nano particles, the method by film formation from a solution is used.

The thickness of the cathode is usually 1 to 1000 nm. The cathode may take a single-layered structure or a multi-layered structure.

### [Production method]

The light emitting device of the present invention can be produced, for example, by sequentially laminating layers on a substrate. Specifically, a light emitting device can be produced by providing an anode on a substrate, providing layers such as a hole injection layer, a hole transporting layer and the like thereon, providing a light emitting layer thereon, providing layers such as an electron transporting layer, and electron injection layer and the like thereon, and further, laminating a cathode thereon. In another production method, a light emitting device can be produced by providing a cathode on a substrate, providing layers such as an electron injection layer, an electron transporting layer, a light emitting layer, a hole transporting layer, a hole injection layer and the like thereon, and further, laminating an anode thereon. In still another production method, a light emitting device can be produced by joining an anode or an anode side base material carrying various layers laminated on the anode and a cathode or a cathode side base material carrying various layers laminated on the cathode so as to face each other.

### [Application]

In order to obtain planar light emission using a light emitting device, the planar anode and the planar cathode may be arranged so as to overlap each other.

In order to obtain patterned light emission, there are a method of installing a mask having a patterned window on the surface of a planar light emitting device, a method in which a layer to be formed as a non-light emitting part is formed extremely thick so as to cause substantially non light emission and a method of forming an anode or a cathode, or both electrodes in a pattern. A segment type display capable of displaying numerals, letters and the like can be obtained by forming a pattern by any one of these methods and disposing several electrodes so that several electrodes can be turned on and off independently. In order to obtain a dot matrix display, both the anode and the cathode may be formed in a stripe shape and arranged so as to be orthogonal to each other. Partial color display and multicolor display become possible by a method of separately coating plural kinds of polymer compounds having different emission colors or a method using a color filter or a fluorescence conversion filter. The dot matrix display can be driven passively or can be driven actively in combination with a TFT and the like. These displays can be used for displays of computers, televisions, portable terminals, and the like. The planar light emitting device can be suitably used as a planar light source for backlight of a liquid crystal display, or as a planar light source for illumination. If a flexible substrate is used, it can be used as a curved light source and a curved display.

### [Heating treatment of light emitting device]

The light emitting device of the present invention may be subjected to a heating treatment, to reduce the driving voltage and to improve the luminance life of the light emitting device. The heating temperature is preferably 40°C to 200°C. The heating time is preferably 1 minute to 3 hours.

### EXAMPLES

The present invention will be illustrated further in detail by examples below, but the present invention is not limited to these examples.

In examples, the polystyrene-equivalent number-average molecular weight (Mn) and the polystyrene-equivalent weight-average molecular weight (Mw) of a polymer compound were determined by any of the following size exclusion chromatography (SEC) using tetrahydrofuran as the mobile phase. Each measurement conditions of SEC are as described below.

The polymer compound to be measured was dissolved at a concentration of about 0.05% by weight in tetrahydrofuran, and 10 µL of the solution was injected into SEC. The mobile phase was flowed at a flow rate of 1.0 ml/min. As the column, PLgel MIXED-B (manufactured by Polymer Laboratories, Ltd.) was used. As the detector, a UV-VIS detector (manufactured by Tosoh Corporation, trade name: UV-8320GPC) was used.

LC-MS was measured by the following method.

The measurement sample was dissolved in chloroform or tetrahydrofuran to a concentration of about 2 mg/mL, and about 1 µL of the solution was injected into LC-MS (manufactured by Agilent Technologies Inc., trade name: 1290 Infinity LC and 6230 TOF LC/MS). As the mobile phase of LC-MS, acetonitrile and tetrahydrofuran were flowed at a flow rate of 1.0 ml/min while changing the ratio thereof. As the column, SUMIPAX ODS Z-CLUE (manufactured by Sumitomo Chemical Analysis Center, internal diameter: 4.6 mm, length: 250 mm, particle size: 3 µm) was used.

TLC-MS was measured by the following method.

The measurement sample was dissolved in any solvent of toluene, tetrahydrofuran or chloroform at an arbitrary concentration, and the solution was applied on a TLC plate for DART (manufactured by Techno Applications, trade name: YSK5-100), and it was measured using TLC-MS (manufactured by JEOL Ltd., trade name: JMS-T100TD (The AccuTOF TLC)). The temperature of a helium gas in measurement was adjusted in the range of 200 to 400°C.

NMR was measured by the following method.

Five to ten milligrams of measurement sample was dissolved in about 0.5 mL of deuterated chloroform (CDCl₃), deuterated tetrahydrofuran, deuterated dimethyl sulfoxide, deuterated acetone, deuterated N,N-dimethylformamide, deuterated toluene, deuterated methanol, deuterated ethanol, deuterated 2-propanol or deuterated methylene chloride, and NMR was measured using an NMR apparatus (manufactured by Agilent Technologies Inc., trade name: INOVA300, or manufactured by JEOL RESONANCE, trade name: JNM-ECZ400S/L1).

As the index of the purity of a compound, the value of high performance liquid chromatography (HPLC) area percentage was used. This value is a value at UV = 254 nm by HPLC (manufactured by Shimadzu Corp., trade name: LC-20A), unless otherwise specified. In this procedure, the compound to be measured was dissolved in tetrahydrofuran or chloroform so as to be a concentration of 0.01 to 0.2% by weight, and 1 to 10 µL of the solution was injected into HPLC depending on the concentration. As the mobile phase of HPLC, acetonitrile and tetrahydrofuran were flowed at a flow rate of 1.0 ml/min while changing the ratio of acetonitrile/tetrahydrofuran from 100/0 to 0/100 (volume ratio). As the column, SUMIPAX ODS Z-CLUE (manufactured by Sumika Chemical Analysis Service, Ltd., internal diameter: 4.6 mm, length: 250 mm, particle diameter: 3 µm) or an ODS column having the equivalent performance was used. As the detector, a photo diode array detector (manufactured by Shimadzu Corp., trade name: SPD-M20A) was used.

As the index of the purity of a compound, the value of gas chromatography (GC) area percentage was used. This value is a value at GC (manufactured by Agilent Technologies Inc., trade name: Agilent7820), unless otherwise specified. In this procedure, the compound to be measured was dissolved in tetrahydrofuran or chloroform so as to be a concentration of 0.01 to 0.2% by mass, and 1 to 10 µL of the solution was injected into GC depending on the concentration. As the carrier, helium was used and flowed at a flow rate of 1.0 ml/min. The column oven was used while changing the temperature from 50°C to 300°C. The heater temperature was 280°C at the injection port, and 320°C at the detector. As the column, BPX-5 (30mx0.25mmx0.25pm) manufactured by SGE was used.

### <Synthesis of compound A-Cs>

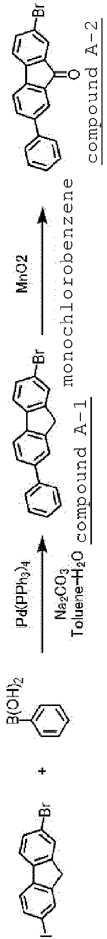

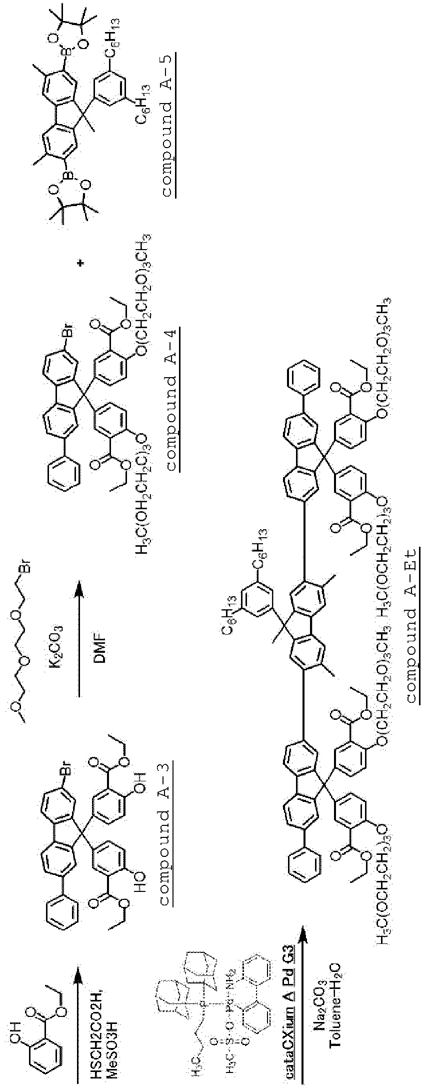

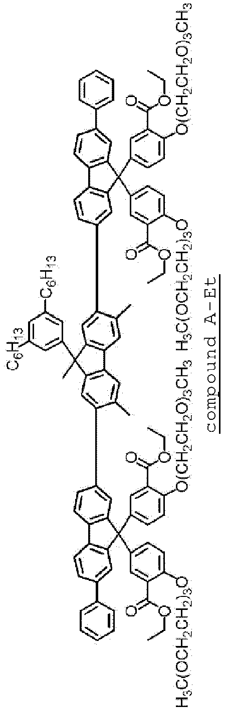

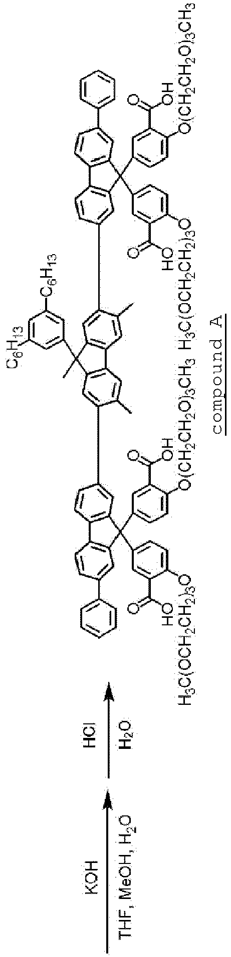

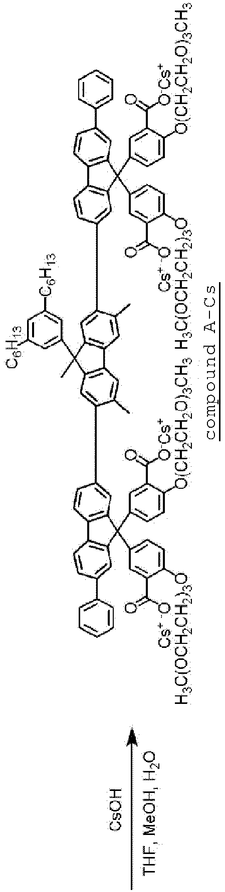

### <Synthesis Example 1> (Synthesis of compound A-1)

Bromoiodo fluorene was purchased from Tokyo Chemical Industry Co., Ltd.

A nitrogen gas atmosphere was prepared in a light-shielded reaction vessel, then, bromoiodo fluorene (35.0 g), phenylboronic acid (11.5 g), tetrakis(triphenylphosphine)palladium(0) (2.2 g), Aliquat336 (2.1 g), sodium carbonate (25.0 g), ion exchanged water (282.7 g) and toluene (525.1 g) were added, and the mixture was stirred at 80°C for 9 hours and 30 minutes. The resultant reaction liquid was cooled down to 50°C, then, toluene was added and the mixture was stirred, and washed with ion exchanged water. To the resultant organic layer was added activated carbon and they were stirred, and the resultant mixture was filtrated, and the resultant filtrate was concentrated under reduced pressure and cooled down to room temperature, to obtain a suspension. To the resultant suspension was added acetonitrile and they were stirred, and the resultant mixture was filtrated, and the resultant solid was washed with acetonitrile. The resultant solid was dried under reduced pressure, to obtain a compound A-1 (20.3 g) as a pale brown solid. The HPLC area percentage value of the compound A-1 was 99.5%. The detection wavelength was 285 nm.

TLC-MS (DART positive): m/z = 320[M]⁺

¹H-NMR (CD₂Cl₂, 400 MHz): δ (ppm) = 7.81 (d, 1H), 7.78-7.76 (m, 1H), 7.70-7.59 (m, 5H), 7.52-7.48 (m, 1H), 7.47-7.41 (m, 2H), 7.36-7.31 (m, 1H), 3.95 (s, 2H) .

### <Synthesis Example 2> (Synthesis of compound A-2)

A nitrogen gas atmosphere was prepared in a light-shielded reaction vessel, then, the compound A-1 (12.0 g), manganese dioxide (48.8 g) and monochlorobenzene (363.9 g) were added, and the mixture was stirred at 60°C for 3 hours. The resultant reaction liquid was cooled down to 40°C, then, filtrated, and the resultant filtrate was concentrated under reduced pressure and cooled down to room temperature, to obtain a suspension. To the resultant suspension was added methanol and they were stirred, and the resultant mixture was filtrated, and the resultant solid was washed with methanol. The resultant solid was recrystallized using a mixed solvent of toluene and methanol, then, dried under reduced pressure, to obtain a compound A-2 (9.3 g) as an orange-colored solid. The HPLC area percentage value of the compound A-2 was 99.4%. The detection wavelength was 285 nm.

TLC-MS (DART positive): m/z = 335[M+H]⁺

### <Example 1> (Synthesis of compound A-3)

A nitrogen gas atmosphere was prepared in a light-shielded reaction vessel, then, the compound A-2 (7.0 g), ethyl salicylate (27.8 g), mercaptoacetic acid (0.2 g) and methanesulfonic acid (17.9 g) were added, and the mixture was stirred at 65°C for 6 hours and 30 minutes, to obtain a reaction liquid A-3-1.

A nitrogen gas atmosphere was prepared in a light-shielded reaction vessel, then, the compound A-2 (1.0 g), ethyl salicylate (3.0 g), mercaptoacetic acid (0.03 g) and methanesulfonic acid (5.0 g) were added, and the mixture was stirred at 65°C for 3 hours, to obtain a reaction liquid A-3-2.

A nitrogen gas atmosphere was prepared in a light-shielded reaction vessel, then, the compound A-2 (1.0 g), ethyl salicylate (4.0 g), mercaptoacetic acid (0.03 g) and methanesulfonic acid (2.6 g) were added, and the mixture was stirred at 65°C for 6 hours and 30 minutes, to obtain a reaction liquid A-3-3.

The reaction liquid A-3-1, the reaction liquid A-3-2 and the reaction liquid A-3-3 obtained above were mixed and adjusted to 40°C, then, toluene was added and the mixture was stirred, and washed with a sodium carbonate aqueous solution and ion exchanged water.

The resultant organic layer was concentrated under reduced pressure, and cooled down to room temperature, to obtain a suspension. To the resultant suspension was added methanol and they were stirred, and the resultant mixture was filtrated, and the resultant solid was washed with methanol. The resultant solid was recrystallized using a mixed solvent of toluene and methanol, then, dried under reduced pressure, to obtain a compound A-3 (12.0 g) as a white solid. The HPLC area percentage value of the compound A-3 was 99.2%. The detection wavelength was 285 nm.

TLC-MS (DART positive): m/z = 648[M]⁺

¹H-NMR (CD₂Cl₂, 400 MHz): δ (ppm) = 10.76 (S, 2H), 7.86 (d, 1H), 7.75-7.64 (m, 4H), 7.59-7.51 (m, 5H), 7.46-7.39 (m, 2H), 7.38-7.30 (m, 3H), 6.89 (d, 2H), 4.31 (q, 4H), 1.27 (t, 6H).

### <Example 2> (Synthesis of compound A-4)

Diethylene glycol 2-bromoethyl methyl ether was purchased from Tokyo Chemical Industry Co., Ltd.

A nitrogen gas atmosphere was prepared in a light-shielded reaction vessel, then, the compound A-3 (10.0 g), diethylene glycol 2-bromoethyl methyl ether (8.4 g), potassium carbonate (6.4 g) and N,N-dimethylformamide (50.2 g) were added, and the mixture was stirred at 70°C for 7 hours. The resultant reaction liquid was cooled down to room temperature, then, toluene was added and the mixture was stirred, and washed with ion exchanged water. To the resultant organic layer was added magnesium sulfate and they were stirred, and the resultant mixture was filtrated, and the resultant filtrate was concentrated under reduced pressure, to obtain a crude product (15.5 g). The resultant crude product (11.7 g) was purified by silica gel column chromatography (a mixed solvent of hexane, toluene and ethanol), and concentrated under reduced pressure, and dried, to obtain a compound A-4 (10.3 g) as a colorless oil. The HPLC area percentage value of the compound A-4 was 99.7%. The detection wavelength was 285 nm.

LC-MS (ESI positive): m/z = 979[M+K]⁺

¹H-NMR (CD₂Cl₂, 400 MHz): δ (ppm) = 7.85 (d, 1H), 7.71 (d, 1H), 7.66 (dd, 1H), 7.60-7.51 (m, 7H), 7.45-7.40 (m, 2H), 7.37-7.29 (m, 3H), 6.92 (d, 2H), 4.23 (q, 4H), 4.16-4.12 (m, 4H), 3.84-3.80 (m, 4H), 3.70-3.66 (m, 4H), 3, 61-3.55 (m, 8H), 3.50-3.46 (m, 4H), 3.31 (s, 6H), 1.27 (t, 6H).

### <Example 3> (Synthesis of compound A-Et)

A compound A-5 was synthesized according to a method described in International Publication WO2013/191086.

cataCXium A Pd G3 was purchased from Sigma-Aldrich Inc.

A nitrogen gas atmosphere was prepared in a light-shielded reaction vessel, then, the compound A-4 (8.0 g), the compound A-5 (2.9 g), cataCXium A Pd G3 (0.01 g), Aliquat336 (0.2 g), sodium carbonate (1.3 g), ion exchanged water (22.9 g) and toluene (40.0 g) were added, and the mixture was stirred at 80°C for 6 hours and 30 minutes. The resultant reaction liquid was cooled down to room temperature, then, toluene was added and the mixture was stirred, and washed with ion exchanged water. To the resultant organic layer was added magnesium sulfate and they were stirred, and the resultant mixture was filtrated, and the resultant filtrate was concentrated under reduced pressure, to obtain a crude product. The resultant crude product was purified by silica gel column chromatography (a mixed solvent of hexane, toluene and ethanol), and concentrated under reduced pressure, and dried, to obtain a compound A-Et (6.2 g) as a colorless oil. The HPLC area percentage value of the compound A-Et was 99.9%. The detection wavelength was 300 nm.

LC-MS (ESI negative): m/z = 2232[M+AcO]⁻

¹H-NMR (CD₂Cl₂, 400 MHz): δ (ppm) = 7.90-7.81 (m, 4H), 7.69-7.63 (m, 4H), 7.62-7.54 (m, 10H), 7.45-7.29 (m, 14H), 7.16 (s, 2H), 6.92-6.86 (m, 4H), 6.85-6.79 (m, 3H), 4.24-4.15 (m, 8H), 4.14-4.08 (m, 8H), 3, 83-3.77 (m, 8H), 3.69-3.64 (m, 8H), 3.60-3.53 (m, 16H), 3.49-3.44 (m, 8H), 3.31-3.28 (m, 12H), 2.48-2.42 (m, 4H), 2.32 (s, 6H), 1.83 (s, 3H), 1.53-1.45 (m, 4H), 1.28-1.19 (m, 24H), 0.87-0.80 (m, 6H).

### <Example 4> (Synthesis of compound A)

A nitrogen gas atmosphere was prepared in a light-shielded reaction vessel, then, the compound A-Et (4.6 g), methanol (7.0 g), ion exchanged water (6.9 g), potassium hydroxide (0.8 g) and tetrahydrofuran (23.2 g) were added, and the mixture was stirred at 60°C for 3 hours. The resultant reaction liquid was cooled down to room temperature, then, hydrochloric acid and methyl isobutyl ketone were added and they were stirred, and the mixture was washed with ion exchanged water. To the resultant organic layer was added magnesium sulfate and they were stirred, and the resultant mixture was filtrated, and the resultant filtrate was concentrated under reduced pressure, to obtain a crude product. The resultant crude product was recrystallized using a mixed solvent of butyl acetate and n-heptane, then, dried under reduced pressure, to obtain a compound A (3.7 g) as a white solid. The HPLC area percentage value was 99.5%. The detection wavelength was 300 nm. LC-MS (ESI negative): m/z = 2060[M-H]⁻

¹H-NMR (CD₂Cl₂, 400 MHz): δ (ppm) = 10.90 (br, 4H), 8.03 (d, 4H), 7.93-7.84 (m, 4H), 7.71-7.66 (m, 4H), 7.62-7.56 (m, 6H), 7.47-7.31 (m, 14H), 7.15 (s, 2H), 7.00-6.94 (m, 4H), 6.85-6.79 (m, 3H), 4.36-4.29 (m, 8H), 3.91-3.84 (m, 8H), 3.70-3.65 (m, 8H), 3.64-3.59 (m, 8H), 3.58-3.53 (m, 8H), 3.48-3.43 (m, 8H), 3.31-3.28 (m, 12H), 2.50-2.42 (m, 4H), 2.33 (s, 6H), 1.84 (s, 3H), 1.56-1.44 (m, 4H), 1.29-1.19 (m, 12H), 0.87-0.81 (m, 6H)

### <Example 5> (Synthesis of compound A-Cs)

A nitrogen gas atmosphere was prepared in a light-shielded reaction vessel, then, the compound A (1.2 g), methanol (6.1 g), ion exchanged water (1.2 g), cesium hydroxide monohydrate (0.6 g) and tetrahydrofuran (18.1 g) were added, and the mixture was stirred at 60°C for 6 hours. The resultant reaction liquid was concentrated under reduced pressure, to obtain a crude product. The resultant crude product was purified by reverse phase silica gel column chromatography (a mixed solvent of ion exchanged water and methanol), concentrated under reduced pressure, dried, and further, washed with acetonitrile, then, dried under reduced pressure, to obtain a compound A-Cs (1.4 g) as a white solid.

¹H-NMR (CD₃OD, 400 MHz): δ (ppm) = 7.84-7.75 (m, 4H), 7.65 (s, 2H), 7.59-7.49 (m, 8H), 7.37-7.31 (m, 6H), 7.30-7.21 (m, 6H), 7.18-7.13 (m, 4H), 7.10 (d, 2H), 6.97 (s, 2H), 6.88 (d, 4H), 6.79-6.72 (m, 3H), 4.14-4.05 (m, 8H), 3.79-3.69 (m, 8H), 3.63-3.58 (m, 8H), 3.57-3.50 (m, 16H), 3.47-3.42 (m, 8H), 3.26-3.24 (m, 12H), 2.44 (t, 4H), 2.27 (s, 6H), 1.77 (s, 3H), 1.53-1.42 (m, 4H), 1.22-1.12 (m, 12H), 0.81-0.75 (m, 6H).

### <Synthesis of compound B>

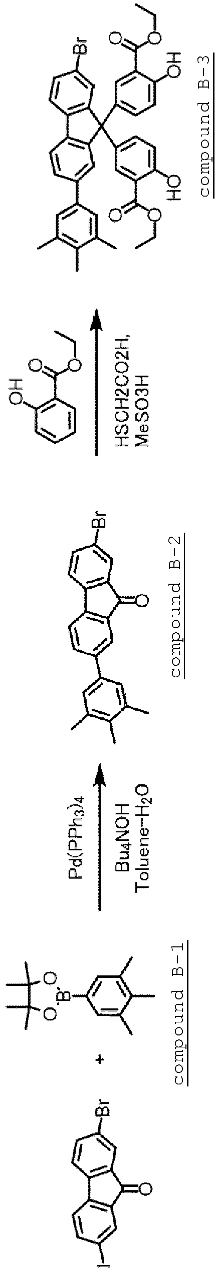

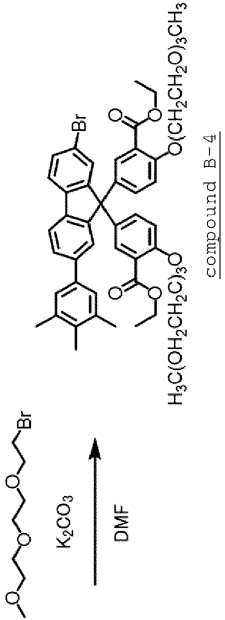

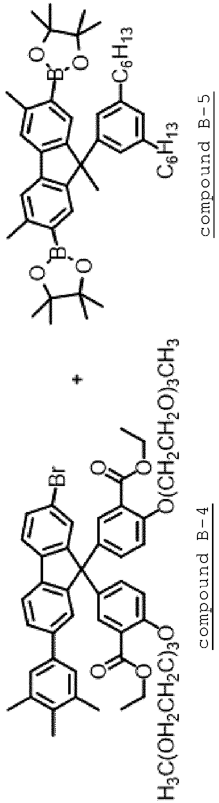

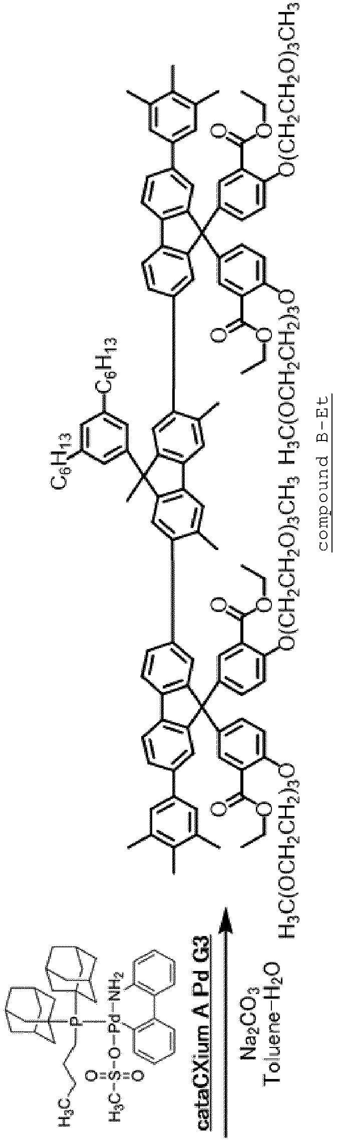

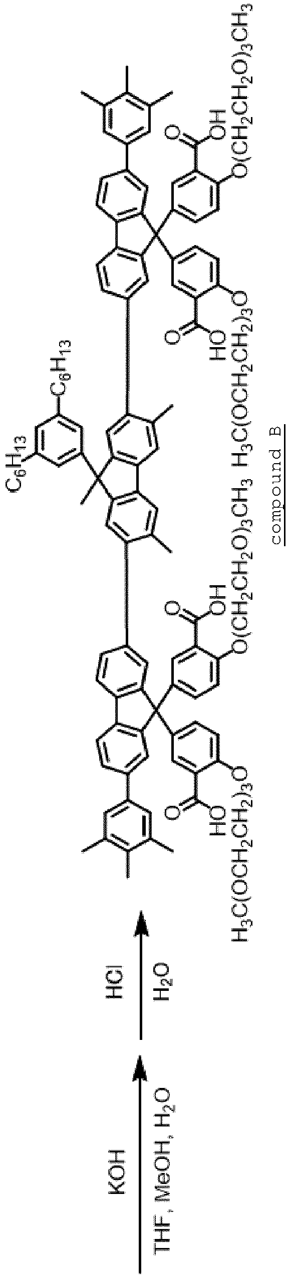

### <Synthesis Example 3> (Synthesis of compound B-2)

A compound B-1 was synthesized according to a method described in International Publication WO2017/170916.

A nitrogen gas atmosphere was prepared in a reaction vessel, then, 2-bromo-7-iodo-9H-fluoren-9-one (50.09 g), the compound B-1 (32.01 g), a 40% by weight tetrabutylammonium hydroxide aqueous solution (211.0 g), tetrakis(triphenylphosphine)palladium (3.00 g), toluene (870 ml) and water (250.48 g) were added, and the mixture was stirred at 85°C for 3.5 hours. The aqueous phase of the resultant reaction product was removed, chloroform was added, and the mixture was washed with ion exchanged water. To the organic phase was added activated carbon, and the mixture was stirred for 1 hour, then, filtrated through a filter paved with silica gel, and washed with chloroform. The solvent was distilled off, to obtain a crude product. It was recrystallized from a mixed solvent of toluene and acetonitrile, to obtain 42.31 g of a compound B-2. The HPLC area percentage value was 99.7%. The detection wavelength was 280 nm.

TLC-MS (DART): 377.18 ([M+H]⁺, Exact Mass: 376.05)

### <Example 6> (Synthesis of compound B-3)

A nitrogen gas atmosphere was prepared in a reaction vessel, then, the compound B-2 (20.00 g), ethyl salicylate (52.91 g), methanesulfonic acid (89.40 g) and mercaptoacetic acid (0.49 g) were added, and the mixture was stirred at 70°C for 9 hours. To the resultant reaction product was added chloroform (60 ml). An atmosphere in another reaction vessel was purged with nitrogen, and methanol (600 ml) was charged and cooled with an ice bath. A chloroform solution of the reaction product was dropped, to deposit a crystal, and the mixture was stirred for 30 minutes, then, filtrated and washed with methanol, to obtain a crude product. It was recrystallized from a mixed solvent of toluene and acetonitrile, to obtain 22.55 g of a compound B-3. The HPLC area percentage was 95.7%. The detection wavelength was 310 nm.

TLC-MS (DART): 691.50 ([M+H]⁺, Exact Mass: 690.16)

### <Example 7> (Synthesis of compound B-4)

A nitrogen gas atmosphere was prepared in a reaction vessel, then, the compound B-3 (15.29 g), diethylene glycol 2-bromoethyl methyl ether (12.07 g), potassium carbonate (9.17 g) and dehydrated DMF (72 ml) were added, and the mixture was stirred at 70°C for 8.5 hours. Toluene (150 ml) was added and the mixture was cooled with an ice bath, and water (150 ml) was dropped. The liquid was separated, and the organic phase was liquid-separated and washed with ion exchanged water, dehydrated with magnesium sulfate, then, filtrated and the solvent was distilled off, to obtain a crude product. It was purified by silica gel column chromatography (a mixed solvent of hexane, toluene and ethanol), and concentrated under reduced pressure, and dried, to obtain a compound B-4 (19.59 g). The HPLC area percentage was 99.9%. The detection wavelength was 310 nm.

¹H-NMR (CDCl₃, 400 MHz): δ (ppm) = 1.28 (6H, t), 2.19 (3H, s), 2.32 (6H, s), 3.34 (6H, s), 3.50-3.53 (4H, m), 3.60-3.65 (8H, m), 3.71-3.75 (4H, m), 3.83-3.87 (4H, m), 4.12-4.16 (4H, m), 4.25 (4H, q), 6.85 (2H, d), 7.14-7.18 (3H, m), 7.24 (1H, dd), 7.44-7.50 (3H, m), 7.54-7.57 (3H, m), 7.61 (1H, d), 7.73 (1H, d)

### <Example 8> (Synthesis of compound B-Et)

A nitrogen gas atmosphere was prepared in a reaction vessel, then, the compound B-4 (4.41 g), the compound A-5 (1.46 g), sodium carbonate (0.68 g), cataCXium A Pd G3 (0.008 g), Aliquat336 (91 mg), toluene (21.9 g) and ion exchanged water (12.0 g) were added, and the mixture was stirred at 80°C for 5 hours. It was diluted with toluene and the liquid was separated, and the organic phase was liquid-separated and washed with dilute hydrochloric acid and ion exchanged water, and magnesium sulfate and activated carbon were added and the mixture was stirred for 1 hour. It was filtrated through a filter paved with Celite, washed with toluene, and the solvent was distilled off, to obtain a crude product. It was purified by silica gel column chromatography (a mixed solvent of hexane, toluene and ethanol), and concentrated under reduced pressure, and dried, to obtain a compound B-Et (4.31 g). The HPLC area percentage was 99.3%.

¹H-NMR (CDCl₃, 400 MHz): δ (ppm) = 0.83 (6H, t), 1.18-1.31 (24H, m), 1.47-1.55 (4H, m), 1.84 (3H, s), 2.20 (6H, s), 2.28 (6H, s), 2.33 (12H, s), 2.43-2.49 (4H, m), 3.34 (6H, s), 3.35 (6H, s), 3.50-3.55 (8H, m), 3.60-3.66 (16H, m), 3.71-3.75 (8H, m), 3.83-3.87 (8H, m), 4.11-4.15 (8H, m), 4.18-4.25 (8H, m), 6.79 (1H, s), 6.81-6.85 (6H, m), 7.14 (2H, s), 7.15-7.19 (6H, m), 7.28-7.34 (4H, m), 7.38 (2H, s), 7.51 (2H, s), 7.57 (2H, d), 7.60-7.63 (6H, m), 7.75 (2H, d), 7.78 (2H, d)

### <Example 9> (Synthesis of compound B)

A nitrogen gas atmosphere was prepared in a reaction vessel, then, the compound B-Et (4.53 g), methanol (6.79 g), ion exchanged water (6.81 g), potassium hydroxide (0.82 g) and tetrahydrofuran (22.6 g) were added, and the mixture was stirred at 60°C for 3 hours. The resultant reaction liquid was cooled down to room temperature, then, hydrochloric acid and methyl isobutyl ketone were added and the mixture was stirred, and the liquid was separated and washed with ion exchanged water. To the resultant organic layer was added magnesium sulfate and they were stirred, and the resultant mixture was filtrated, and the resultant filtrate was concentrated under reduced pressure, to obtain a crude product. The resultant crude product was recrystallized using a mixed solvent of ethyl acetate and n-hexane, then, dried under reduced pressure, to obtain a compound B (3.95 g). The HPLC area percentage value was 99.9%.

¹H-NMR (CDCl₃, 400 MHz): δ (ppm) = 0.83 (6H, t), 1.18-1.28 (12H, m), 1.46-1.55 (4H, m), 1.84 (3H, s), 2.19 (6H, s), 2.28 (6H, s), 2.33 (12H, s), 2.43-2.49 (4H, m), 3.33 (6H, s), 3.34 (6H, s), 3.49-3.53 (8H, m), 3.60-3.63 (8H, m), 3.64-3.68 (8H, m), 3.69-3.72 (8H, m), 3.86-3.90 (8H, m), 4.28-4.32 (8H, m), 6.79 (1H, s), 6.82-6.88 (6H, m), 7.11 (2H, s), 7.18 (4H, s), 7.25-7.33 (6H, m), 7.36 (2H, s), 7.50 (2H, s), 7.57-7.61 (4H, m), 7.75 (2H, d), 7.78 (2H, d), 8.14 (4H, s), 10.92 (4H, s)

### <Synthesis of compound C>

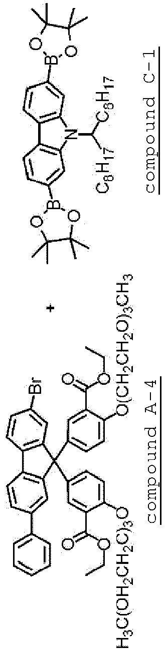

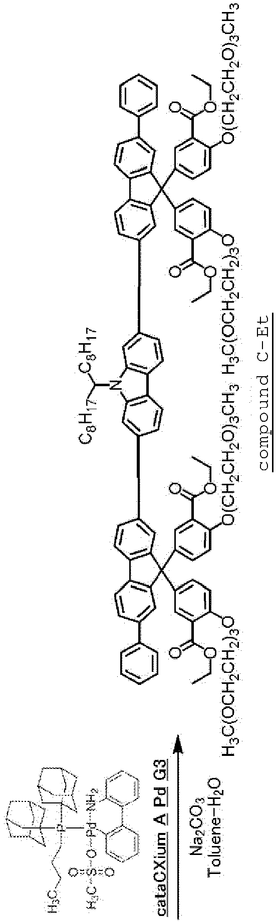

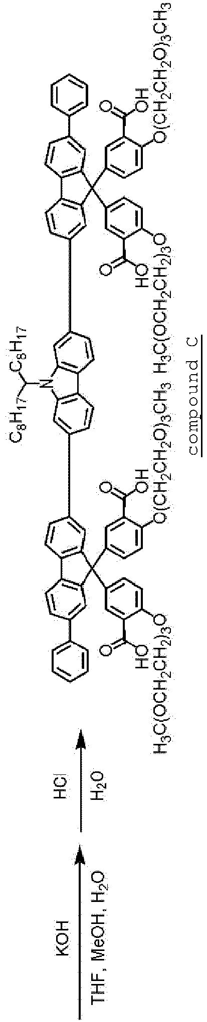

### <Example 10> (Synthesis of compound C-Et)

A compound C-1 was purchased from Sigma-Aldrich Inc.

A nitrogen gas atmosphere was prepared in a reaction vessel, then, the compound A-4 (4.37 g), the compound C-1 (1.37 g), cataCXium A Pd G3 (7.56 mg), sodium carbonate (1.1 g), ion exchanged water (20.6 g), Aliquat336 (126 mg) and toluene (27 mL) were added, and the mixture was stirred at 85°C for 4 hours. Thereafter, the resultant reaction liquid was cooled down to room temperature, toluene and ion exchanged water were added and the liquid was separated and washed, then, the resultant organic layer was dried over anhydrous sodium sulfate, and filtrated. The resultant filtrate was concentrated under reduced pressure, to obtain an oily compound. The resultant oil was purified by reverse phase silica gel column chromatography (acetonitrile and methanol), then, concentrated under reduced pressure at 50°C and dried, to obtain a compound C-Et (3.3 g). The HPLC area percentage value was 99.0%.

¹H-NMR (CD₂Cl₂, 400 MHz): δ (ppm) = 0.74 (6H, t), 1.08-1.24 (36H, m), 1.93 (2H, m), 2.32 (2H, m), 3.26 (12H, s), 3.41-3.45 (8H, m), 3.51-3.55 (16H, m), 3.62-3.66 (8H, m), 3.76-3.80 (8H, m), 4.08-4.12 (8H, m), 4.19 (8H, q), 4.67 (1H, m), 6.90 (4H, d), 7.40 (18H, m), 7.56-7.76 (12H, m), 7.90 (4H, t), 8.09 (2H, t)

### <Example 11> (Synthesis of compound C)

A nitrogen gas atmosphere was prepared in a reaction vessel, then, the compound C-Et (3.0 g), potassium hydroxide (0.55 g), tetrahydrofuran (30 ml), methanol (7.5 mL) and ion exchanged water (7.5 g) were added, and the mixture was stirred at 65°C for 2 hours. Thereafter, the resultant reaction liquid was cooled down to room temperature, then, hydrochloric acid water was added and the mixture was stirred, then, chloroform and ion exchanged water were added and the liquid was separated and washed, then, the resultant organic layer was dried over anhydrous sodium sulfate, and filtrated. The resultant filtrate was concentrated under reduced pressure, to obtain a solid. The resultant solid was recrystallized using a mixed solvent of tetrahydrofuran and heptane, then, dried at 50°C under reduced pressure, to obtain a compound C (1.9 g). The HPLC area percentage value was 99.5%.

¹H-NMR (CD₂Cl₂, 400 MHz): δ (ppm) = 0.74 (6H, t), 1.07-1.21 (24H, m), 1.97 (2H, m), 2.32 (2H, m), 3.24 (12H, s), 3.41-3.63 (32H, m), 3.85 (8H, m), 4.30 (8H, m), 4.66 (1H, m), 6.97 (4H, d), 7.30-7.42 (12H, m), 7.55-7.75 (14H, m), 7.92 (4H, m), 8.07 (6H, t), 10.89 (4H, s)

### <Synthesis of compound D>

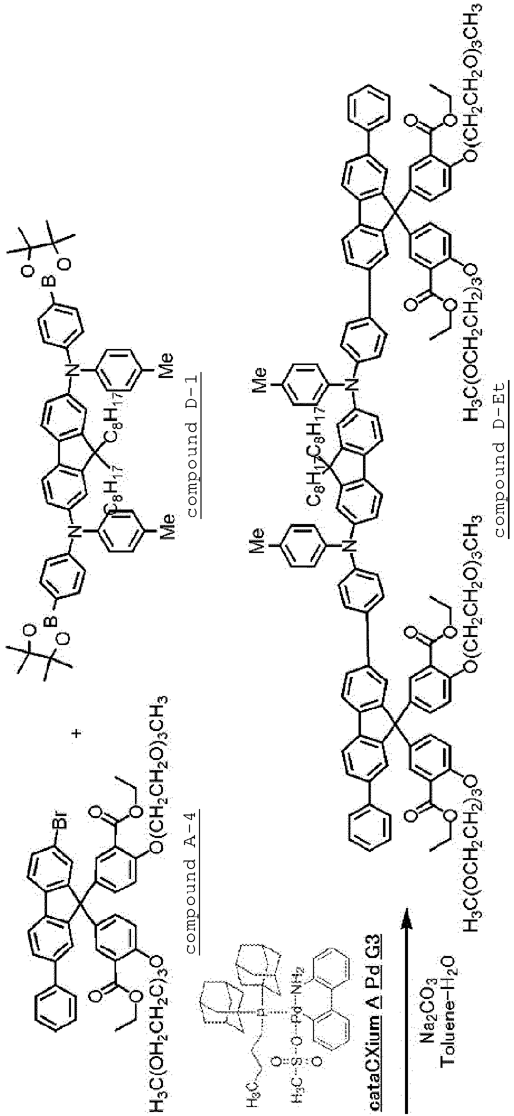

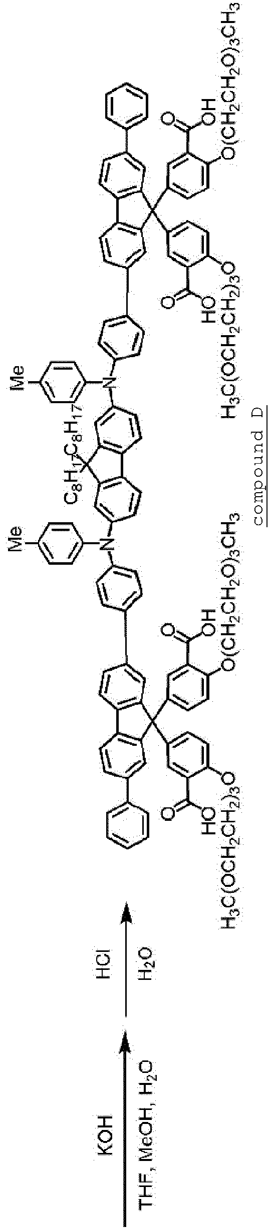

### <Example 12> (Synthesis of compound D-Et)

A compound D-1 was synthesized according to a method described in International Publication WO2013/114976.

A nitrogen gas atmosphere was prepared in a reaction vessel, then, the compound D-1 (1.25 g), the compound A-4 (2.41 g), cataCXium A Pd G3 (4.4 mg), sodium carbonate (0.40 g), ion exchanged water (7.60 g), Aliquat336 (50 mg) and toluene (20 ml) were added, and the mixture was stirred at 85°C for 5 hours. Thereafter, the resultant reaction liquid was cooled down to room temperature, then, diluted with toluene and washed with ion exchanged water, to the resultant organic layer were added anhydrous sodium sulfate and activated carbon and the mixture was stirred for 30 minutes, then, filtrated. The resultant filtrate was concentrated under reduced pressure, to obtain a crude product. The resultant crude product was purified by silica gel column chromatography (hexane, toluene and ethanol), then, concentrated under reduced pressure at 50°C and dried, to obtain a compound D-Et (2.63 g). The HPLC area percentage value was 99.8%. The detection wavelength was 300 nm.

¹H-NMR (CDCl₃, 400 MHz): δ (ppm) 0.65 to 0.74 (4H, m), 0.83 (6H, t), 0.99 to 1.23 (20H, m), 1.26 (12H, t), 1.72 to 1.79 (4H, m), 2.31 (6H, brs), 3.33 (12H, s), 3.48 to 3.51 (8H, m), 3.59 to 3.64 (16H, m), 3.70 to 3.74 (8H, m), 3.82 to 3.86 (8H, m), 4.11 to 4.15 (8H, m), 4.24 (8H, q), 6.85 (4H, d), 6.92 to 7.18 (14H, m), 7.28 to 7.34 (6H, m), 7.38 to 7.43 (8H, m), 7.52 to 7.62 (16H, m), 7.64 to 7.66 (4H, m), 7.78 to 7.82 (4H, m)

### <Example 13> (Synthesis of compound D)

A nitrogen gas atmosphere was prepared in a reaction vessel, then, the compound D-Et (1.95 g), potassium hydroxide (0.31 g), tetrahydrofuran (12 ml), methanol (3.5 ml) and ion exchanged water (3.19 g) were added, and the mixture was stirred at 65°C for 10 hours. Thereafter, the resultant reaction liquid was cooled down to room temperature, then, hydrochloric acid water was added and the mixture was stirred, then, methyl isobutyl ketone was added and the liquid was separated and washed with ion exchanged water, then, the resultant organic phase was dried over anhydrous magnesium sulfate, and filtrated. The resultant filtrate was concentrated under reduced pressure, to obtain a crude product. The resultant crude product was recrystallized using a mixed solvent of ethyl acetate and hexane, then, dried at 50°C under reduced pressure, to obtain a compound D (2.12 g). The HPLC area percentage value was 99.8%. The detection wavelength was 300 nm.

¹H-NMR (CDCl₃, 400 MHz): δ (ppm) 0.65 to 0.74 (4H, m), 0.83 (6H, t), 1.00 to 1.29 (20H, m), 1.73 to 1.80 (4H, m), 2.33 (6H, s), 3.33 (12H, s), 3.49 to 3.52 (8H, m), 3.60 to 3.63 (8H, m), 3.64 to 3.67 (8H, m), 3.69 to 3.73 (8H, m), 3.87 to 3.90 (8H, m), 4.29 to 4.33 (8H, m), 6.88 (4H, d), 6.99 to 7.12 (16H, m), 7.29 to 7.34 (6H, m), 7.38 to 7.43 (8H, m), 7.46 (2H, d), 7.53 to 7.56 (8H, m), 7.59 to 7.64 (4H, m), 7.80 to 7.84 (4H, m), 8.19 (4H, d), 10.98 (4H, brs)

### <Synthesis of compound E>

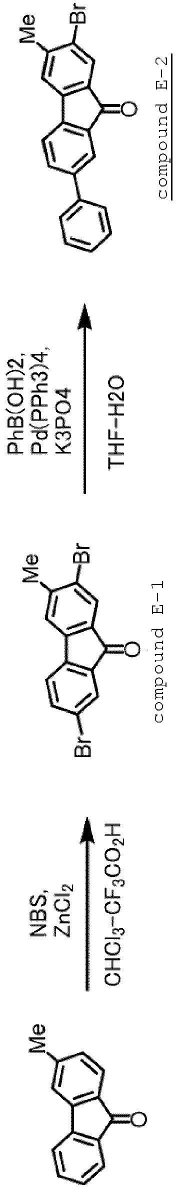

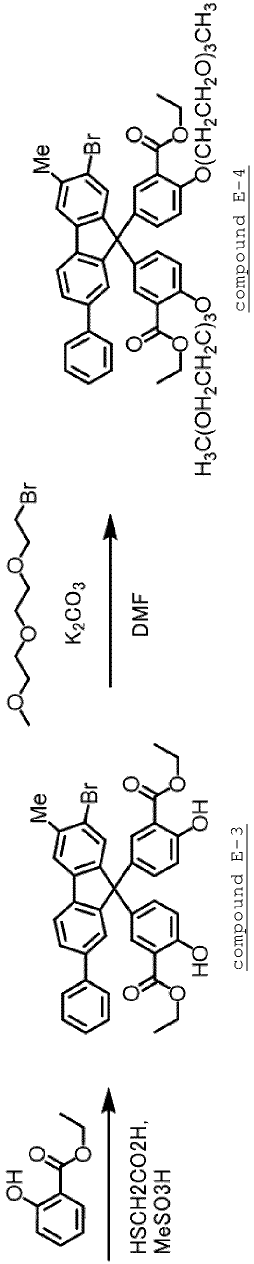

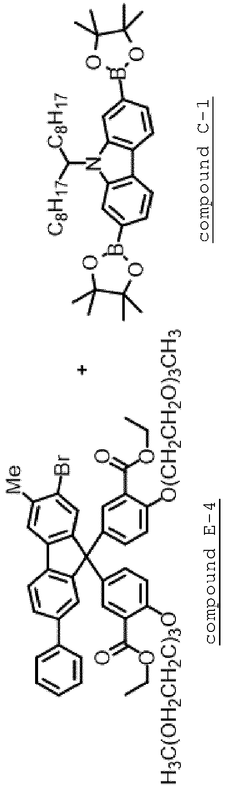

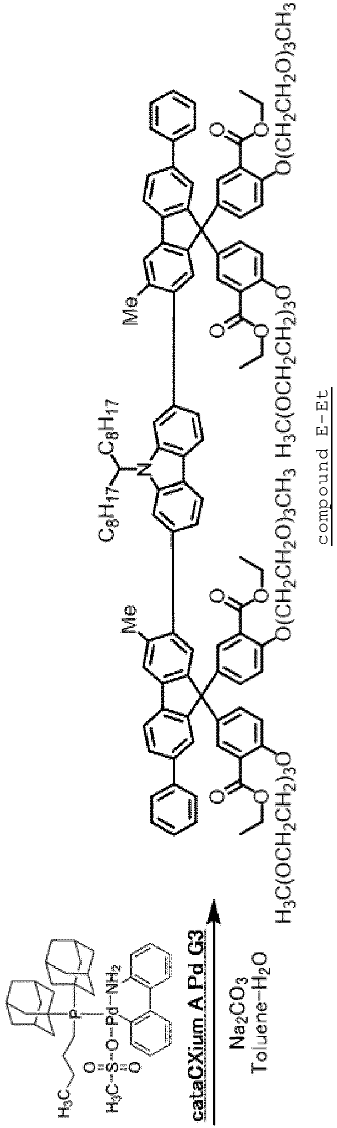

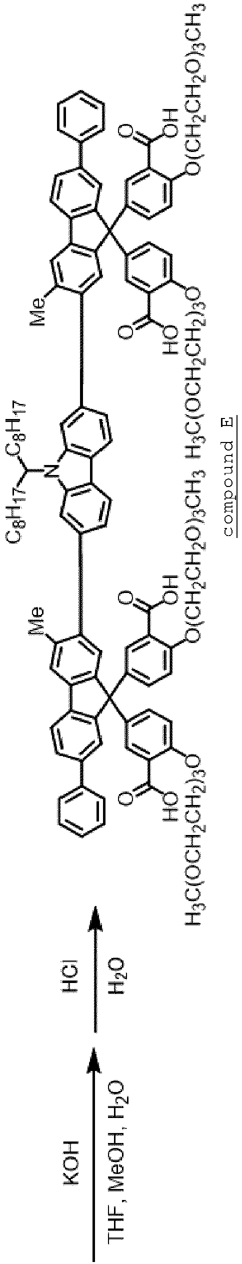

### <Example 14> (Synthesis of compound E-1)

An inert gas atmosphere was prepared in a light-shielded reaction vessel, then, 3-methyl-9H-fluoren-9-one (20.00 g), zinc chloride (29.54 g), chloroform (200 ml) and trifluoroacetic acid (100 ml) were added, and cooled with an ice bath. N-bromosuccinimide (36.64 g) was divided and charged so that the temperature in the reaction vessel was 10°C or lower. The ice bath was removed, and the mixture was stirred for 5 hours. It was cooled with an ice bath, a 5% sodium sulfite aqueous solution (240 g) was dropped, chloroform (480 ml) was added, the liquid was separated and washed with ion exchanged water, and concentrated under reduced pressure, to obtain a crude product. It was recrystallized using a mixed solvent of heptane and toluene, to obtain 20.83 g of a compound E-1. The HPLC area percentage value was 99.9%.

TLC-MS (DART) 349.88 ([M]⁺, Exact Mass: 349.89)

¹H-NMR (CDCl₃, 400 MHz): δ (ppm) 2.47 (3H, s), 7.33 to 7.37 (2H, m), 7.59 (1H, dd), 7.73 (1H, d), 7.78 (1H, s)

### <Synthesis Example 4> (Synthesis of compound E-2)

An inert gas atmosphere was prepared in a reaction vessel, then, the compound E-1 (20.67 g), phenylboric acid (7.88 g), potassium phosphate (18.77 g), tetrakis(triphenylphosphine)palladium(0) (2.59 g), THF (310 ml) and ion exchanged water (62 ml) were added, and the mixture was stirred at 60°C for 61 hours. Chloroform (460 ml) were added, the liquid was separated and washed with ion exchanged water, dehydrated with magnesium sulfate, then, filtrated and concentrated under reduced pressure, to obtain a crude product. It was recrystallized using a mixed solvent of heptane and toluene, to obtain 9.53 g of a compound E-2. The HPLC area percentage value was 99.9%.

¹H-NMR (CDCl₃, 400 MHz): δ (ppm) 2.47 (3H, s), 7.35 to 7.40 (2H, m), 7.43 to 7.48 (2H, m), 7.53 (1H, d), 7.60 (2H, dd), 7.70 (1H, dd), 7.79 (1H, s), 7.86 (1H, d)

### <Example 15> (Synthesis of compound E-3)

A nitrogen gas atmosphere was prepared in a reaction vessel, then, the compound E-2 (6.90 g), ethyl salicylate (17.4 g), mercaptoacetic acid (0.18 g) and methanesulfonic acid (22.6 ml) were added, and the mixture was stirred at 70°C for 2.5 minutes. Chloroform was added and the mixture was stirred, the liquid was separated and washed with ion exchanged water, and the resultant organic layer was concentrated under reduced pressure, to obtain a crude product. The resultant crude product was recrystallized using a mixed solvent of toluene and methanol, then, dried under reduced pressure, to obtain a compound E-3 (13.27 g). The HPLC area percentage value of the compound E-3 was 99.8%.

¹H-NMR (CDCl₃, 400 MHz): δ (ppm) 1.28 (6H, t), 2.50 (3H, s), 4.31 (4H, q), 6.89 (2H, d), 7.29 to 7.36 (3H, m), 7.39 to 7.44 (2H, m), 7.50 to 7.55 (4H, m), 7.59 to 7.62 (1H, m), 7.65 to 7.67 (3H, m), 7.79 (1H, d), 10.77

### (2H, s)

### <Example 16> (Synthesis of compound E-4)

A nitrogen gas atmosphere was prepared in a reaction vessel, then, the compound E-3 (11.53 g), diethylene glycol 2-bromoethyl methyl ether (9.23 g), potassium carbonate (7.04 g) and N,N-dimethylformamide (58 ml) were added, and the mixture was stirred at 75°C for 20 hours. The resultant reaction liquid was cooled down to room temperature, then, toluene was added and the mixture was stirred, and the liquid was separated and washed with ion exchanged water. To the resultant organic layer was added magnesium sulfate and they were stirred, and the resultant mixture was filtrated, and the resultant filtrate was concentrated under reduced pressure to obtain a crude product. The resultant crude product was purified by silica gel column chromatography (a mixed solvent of hexane, toluene and ethanol), and dried under reduced pressure, to obtain a compound E-4 (17.95 g). The HPLC area percentage value of the compound E-4 was 99.9%.

TLC-MS (DART) 954.20 ([M]⁺, Exact Mass: 954.32)

¹H-NMR (CDCl₃, 400 MHz): δ (ppm) 1.28 (6H, t), 2.48 (3H, s), 3.35 (6H, s), 3.50 to 3.53 (4H, m), 3.61 to 3.66 (8H, m), 3.71 to 3.75 (4H, m), 3.83 to 3.87 (4H, m), 4.13 to 4.16 (4H, m), 4.25 (4H, q), 6.85 (2H, d), 715 to 7.19 (1H, m), 7.23 to 7.25 (1H, m), 7.29 to 7.34 (1H, m), 7.38 to 7.43 (2H, m), 7.49 (1H, s), 7.51 to 7.55 (3H, m), 7.56 to 7.60 (3H, m), 7.62 (1H, s), 7.76

### (1H, d)

### <Example 17> (Synthesis of compound E-Et)

An inert gas atmosphere was prepared in a reaction vessel, then, the compound E-4 (2.46 g), the compound C-1 (0.82 g), toluene (11.48 g), Aliquat336 (0.05 g), a 6% by weight Na₂CO₃ aqueous solution (6.96 g) and cataCXium A Pd G3 (4.54 mg) were added, and the mixture was heated at 80°C and reacted for 6 hours.

The reaction liquid was cooled, then, toluene was added and the liquid was separated, the liquid was separated and washed with ion exchanged water 3 times, then, to the organic layer were added magnesium sulfate and activated carbon and the mixture was stirred for 1 hour, and the filtrate was dried, to obtain an oil.

The oil was purified by silica gel column chromatography (a mixed solvent of hexane, toluene and ethanol), then, concentrated and dried, to obtain 1.83 g (72%) of a compound E-Et.

¹H-NMR (CDCl₃, 400 MHz): δ (ppm) = 0.78 (6H, t), 1.01-1.27 (32H, m), 1.88 (2H, m), 2.21 (2H, m), 2.35 (6H, s), 2.40 (4H, s), 3.33 (12H, s), 3.50 (8H, m), 3.55-3.63 (16H, m), 3.72 (8H, m), 3.83 (8H, m)4.12 (8H, t), 4.22 (8H, q), 4.53 (1H, quin), 6.84 (4H, d), 7.09-7.20 (10H, m), 7.30-7.43 (10H, m), 7.53-7.65 (10H, m), 7.83 (2H, d), 8.08 (2H, dd)

### <Example 18> (Synthesis of compound E)

An inert gas atmosphere was prepared in a reaction vessel, then, the compound E-Et (1.68 g), potassium hydroxide (0.31 g), tetrahydrofuran (8.4 g), methanol (2.52 g) and water (2.52 g) were added, and the mixture was heated at 60°C and reacted for 3 hours.

The resultant reaction liquid was cooled down to room temperature, then, 1 N hydrochloric acid (6.36 g) and methyl isobutyl ketone (16.8 g) were added, then, the liquid was separated, and washed with ion exchanged water 3 times, and the organic layer was dried over magnesium sulfate (1.7 g) and filtrated. The filtrate was concentrated and dried, to obtain an oil.

The resultant oil was recrystallized using a mixed solvent of tetrahydrofuran and hexane, then, dried at 50°C, to obtain a compound E (1.28 g).

¹H-NMR (CDCl₃, 400 MHz): δ (ppm) = 0.79 (6H, t), 1.01-1.27 (24H, m), 1.89 (2H, m), 2.23 (2H, m), 2.40 (3H, s), 2.43 (3H, s), 3.33 (12H, s), 3.50 (8H, m), 3.56-3.63 (8H, m), 3.64-3.68 (8H, m), 3.87-3.71 (8H, m), 3.87 (8H, m), 4.29 (8H, m), 4.53 (1H, quin), 6.84 (4H, d), 7.09 (2H, m), 7.30-7.43 (13H, m), 7.53 (1H, s), 7.55 (6H, m), 7.63 (2H, dd), 7.71 (2H, s), 7.84 (2H, d), 8.03-8.12 (2H, m), 8.12 (4H, m), 10.95 (4H, brs)

### <Synthesis of compound F>

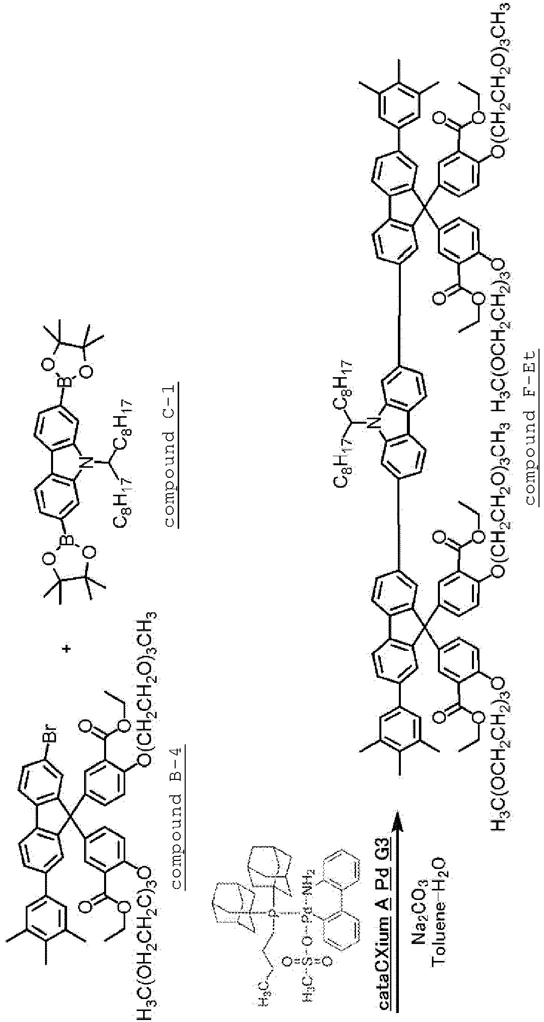

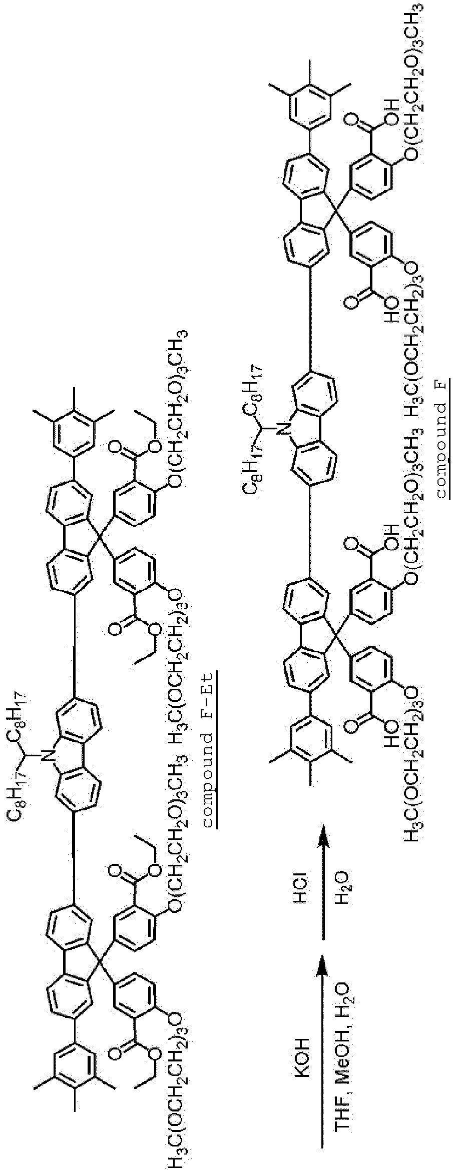

### <Example 19> (Synthesis of compound F-Et)

An inert gas atmosphere was prepared in a reaction vessel, then, the compound B-4 (2.50 g), the compound C-1 (0.78 g), toluene (10.9 g), Aliquat336 (0.05 g), a 6% by weight Na₂CO₃ aqueous solution (6.60 g) and cataCXium A Pd G3 (4.3 mg) were added, and the mixture was heated at 80°C and reacted for 4 hours.

The reaction liquid was cooled, then, toluene was added and the liquid was separated, and the liquid was separated and washed with ion exchanged water, then, to the organic layer were added magnesium sulfate and activated carbon and the mixture was stirred for 1 hour, then, filtrated, and the filtrate was dried, to obtain a crude product.

The crude product was purified by a silica gel column (a mixed solvent of hexane, toluene and ethanol), then, concentrated and dried, to obtain 1.75 g of a compound F-Et. The HPLC area percentage value was 99.6%. The detection wavelength was 300 nm.

¹H-NMR (CD₂Cl₂, 400 MHz): δ (ppm) 0.75 (6H, t), 0.99 to 1.28 (38H, m), 1.90 to 2.06 (2H, m), 2.18 (6H, s), 2.31 (12H, s), 3.26 (12H, s), 3.43 (8H, m), 3.52 (16H, m), 3.64 (8H, q), 3.77 (8H, t), 4.10 (8H, t), 4.18 (8H, q), 4.61 to 4.71 (1H, m), 6.88 (4H, d), 7.38 (6H, d), 7.56 to 7.65 (9H, m), 7.68 to 7.78 (5H, m), 7.88 (4H, q), 8.09 (6H, m)

### <Example 20> (Synthesis of compound F)

An inert gas atmosphere was prepared in a reaction vessel, then, the compound F-Et (1.65 g), potassium hydroxide (0.29 g), tetrahydrofuran (14.7 g), methanol (3.26 g) and ion exchanged water (4.13 g) were added, and the mixture was heated at 60°C and reacted for 3.5 hours.

The resultant reaction liquid was cooled down to room temperature, then, methyl isobutyl ketone was added, and the liquid was separated and washed with 1 N hydrochloric acid and ion exchanged water, and the organic layer was dried over magnesium sulfate added, and filtrated. The filtrate was dried, to obtain a crude product.

The resultant crude product was recrystallized using a mixed solvent of tetrahydrofuran and hexane, then, dried at 50°C, to obtain a compound F (1.27 g).

¹H-NMR (CD₂Cl₂, 400 MHz): δ (ppm) 0.75 (6H, t), 1.00 to 1.28 (24H, m), 1.90 to 2.05 (2H, m), 2.17 (6H, s), 2.31 (14H, s), 3.25 (12H, s), 3.45 (8H, m), 3.51 to 3.65 (24H, m), 3.84 (8H, t), 4.30 (8H, t), 4.61 to 4.71 (1H, m), 6.95 (4H, d), 7.19 (4H, s), 7.35 to 7.45 (6H, m), 7.52 to 7.79 (10H, m), 7.89 (4H, q), 8.08 (6H, m), 10.88 (4H, brs)

### <Synthesis of compound G>

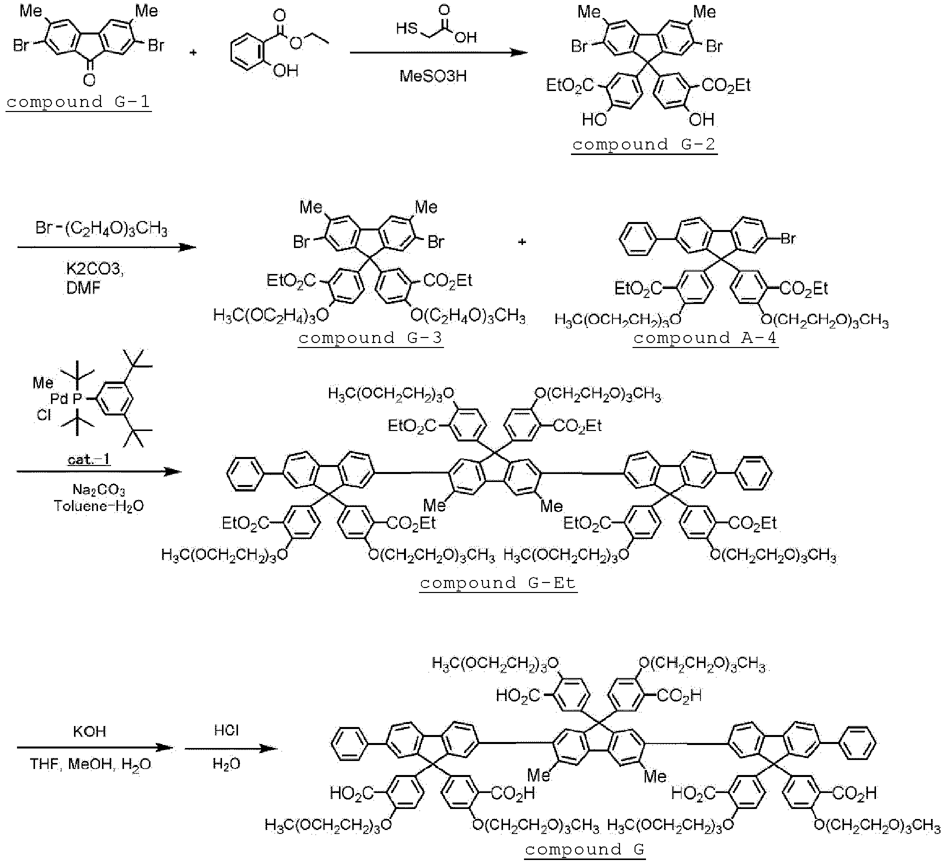

### <Example 21> (Synthesis of compound G-2)

A compound G-1 was synthesized according to a method described in International Publication WO2013/191086.

A nitrogen gas atmosphere was prepared in a reaction vessel, then, the compound G-1 (4.6 g), ethyl salicylate (12.5 g), methanesulfonic acid (21.2 g) and mercaptoacetic acid (116 mg) were added, and the mixture was stirred at 65°C for 6 hours. Thereafter, the resultant reaction liquid was cooled down to room temperature, then, filtrated through a filter. The resultant solid was washed with ion exchanged water and methanol, then, recrystallized twice using a mixed solvent of toluene and acetonitrile, then, concentrated under reduced pressure at 50°C and dried, to obtain a compound G-2 (4.8 g). The HPLC area percentage value was 99.5% or more.

¹H-NMR (CD₂Cl₂, 400 MHz): δ (ppm) = 1.26 (6H, t), 2.43 (6H, s), 4.30 (4H, m), 6.84 (2H, d), 7.19 (2H, d), 7.44 (2H, s), 7.61 (4H, m), 10.72 (2H, s)

### <Example 22> (Synthesis of compound G-3)

A nitrogen gas atmosphere was prepared in a reaction vessel, then, the compound G-2 (4.8 g), diethylene glycol 2-bromoethyl methyl ether (5.3 g), potassium carbonate (5.0 g) and N,N-dimethylformamide (53 mL) were added, and the mixture was stirred at 100°C for 3 hours. Thereafter, the resultant reaction liquid was cooled down to room temperature, then, ethyl acetate and ion exchanged water were added, and the liquid was separated and washed, then, the resultant organic layer was dried over anhydrous sodium sulfate, and filtrated. The resultant filtrate was concentrated under reduced pressure, to obtain an oily compound.

The resultant oil was purified by silica gel column chromatography (a mixed solvent of hexane and ethyl acetate), then, concentrated under reduced pressure at 50°C and dried, to obtain a compound G-3 (6.4 g). The HPLC area percentage value was 99.9% or more.

¹H-NMR (CD₂Cl₂, 400 MHz): δ (ppm) = 1.26 (6H, t), 2.43 (6H, s), 3.28 (6H, s), 3.44 (4H, m), 3.54 (8H, m), 3.64 (4H, m), 3.78 (4H, t), 4.10 (4H, t), 4.21 (4H, m), 6.89 (2H, d), 7.21 (2H, d), 7.41 (2H, d), 7.44 (2H, s), 7.61 (2H, s)

### <Example 23> (Synthesis of compound G-Et)

A cat.-1 was synthesized according to a method described in International Publication WO2017/094655.

A nitrogen gas atmosphere was prepared in a reaction vessel, then, the compound A-4 (1.53 g), the compound G-3 (0.67 g), cat.-1 (0.17 mg), sodium carbonate (0.44 g), ion exchanged water (10.1 g), Aliquat336 (42 mg) and toluene (14 mL) were added, and the mixture was stirred at 85°C for 7 hours. Thereafter, the resultant reaction liquid was cooled down to room temperature, then, toluene and ion exchanged water were added, and the liquid was separated and washed, then, the resultant organic layer was dried over anhydrous sodium sulfate, and filtrated. The resultant filtrate was concentrated under reduced pressure, to obtain an oily compound. The resultant oil was purified by silica gel column chromatography (a mixed solvent of toluene and ethanol), then, concentrated under reduced pressure at 50°C and dried, to obtain a compound G-Et (1.7 g). The HPLC area percentage value was 99.5% or more.

¹H-NMR (CD₂Cl₂, 400 MHz): δ (ppm) = 1.19 (18H, m), 2.30 (6H, s), 3.27 (18H, s), 3.44-3.64 (48H, m), 3.77 (12H, t), 4.07-4.16 (24H, m), 6.85 (6H, t), 7.26-7.41 (18H, m), 7.49-7.57 (12H, m), 7.63 (4H, m), 7.84 (4H, m)

### <Example 24> (Synthesis of compound G)

A nitrogen gas atmosphere was prepared in a reaction vessel, then, the compound G-Et (1.06 g), potassium hydroxide (0.24 g), tetrahydrofuran (10 ml), methanol (2.7 mL) and ion exchanged water (2.6 g) were added, and the mixture was stirred at 65°C for 2 hours. Thereafter, the resultant reaction liquid was cooled down to room temperature, then, hydrochloric acid water was added and the mixture was stirred, then, chloroform and ion exchanged water were added, and the liquid was separated and washed, then, the resultant organic layer was dried over anhydrous sodium sulfate, and filtrated. The resultant filtrate was concentrated under reduced pressure, to obtain a solid. The resultant solid was recrystallized twice using methyl isobutyl ketone, then, dried at 50°C under reduced pressure, to obtain a compound G (0.58 g). The HPLC area percentage value was 99.9% or more.

¹H-NMR (CD₂Cl₂, 400 MHz): δ (ppm) = 2.32 (6H, s), 3.24 (18H, s), 3.43 (12H, m), 3.50-3.64 (36H, m), 3.84 (12H, t), 4.31 (12H, m), 6.97 (6H, d), 7.20 (2H, s), 7.37 (16H, m), 7.55 (6H, t), 7.66 (4H, t), 7.86 (6H, m), 7.95 (4H, d), 10.84 (6H, s)

### <Synthesis of compound H>

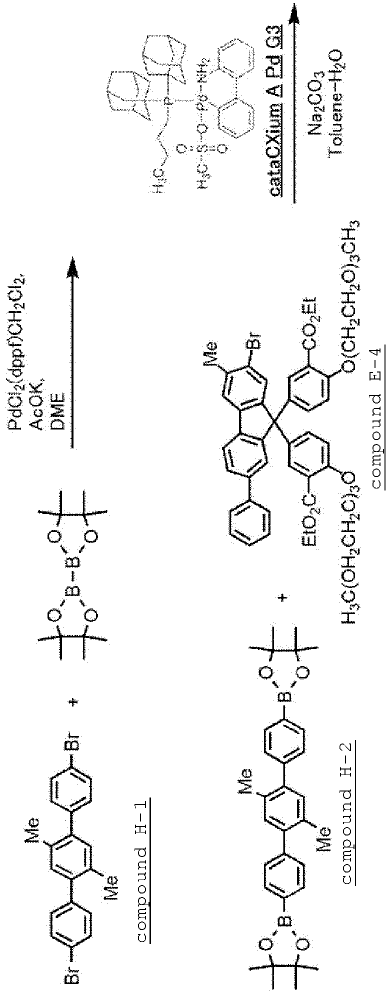

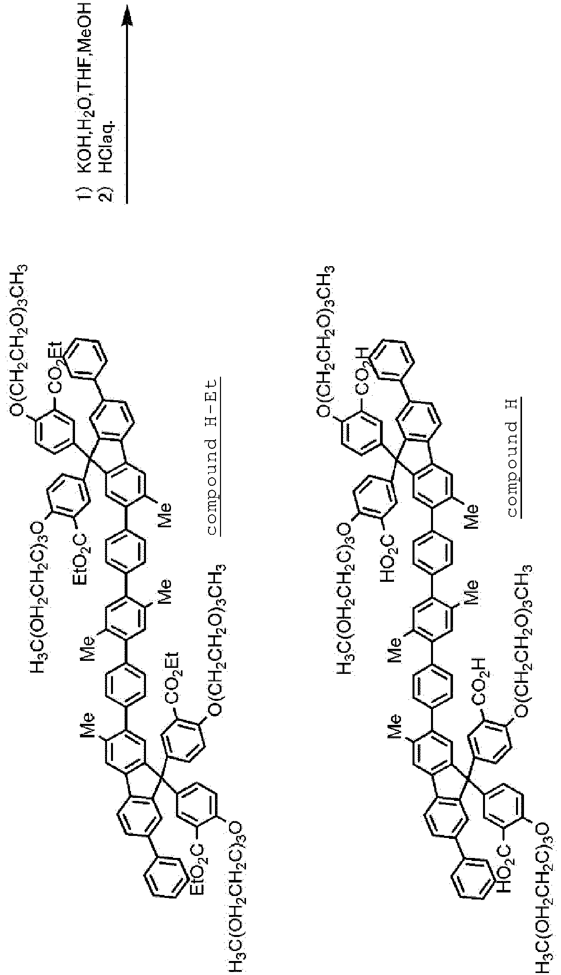

### <Synthesis Example 5> (Synthesis of compound H-2)

An inert gas atmosphere was prepared in a reaction vessel, then, the compound H-1 (7.49 g), potassium acetate (10.60 g), bis(pinacolato)diboron (13.71 g), [1,1'-bis(diphenylphosphino)ferrocene]palladium(II) dichloride-dichloromethane (0.23 g) and 1,2-dimethoxyethane (72 ml) were added, and the mixture was stirred at 90°C for 11 hours. The reaction product was cooled, then, toluene (100 ml) was added, and the mixture was filtrated through Celite, and the filtrate was concentrated. The resultant solid was dissolved in toluene (100 ml) and hexane (200 ml), activated carbon (10 g) was added and the mixture was stirred for 1 hour, then, filtrated through Celite, and the filtrate was concentrated, to obtain a crude product. Crystallization was performed using a mixed solvent of toluene and acetonitrile, to obtain 7.59 g of a compound H-2. The HPLC area percentage was 99.6%.

### <Example 25> (Synthesis of compound H-Et)

An inert gas atmosphere was prepared in a reaction vessel, then, the compound H-2 (0.935 g), the compound E-4 (3.675 g), sodium carbonate (0.58 g), Aliquat336 (0.07 g), cataCXium A Pd G3 (manufactured by Sigma-Aldrich Inc.) (6.6 mg), toluene (13.1 g) and ion exchanged water (7.5 g) were added, and the mixture was stirred at 90°C for 5 hours. The reaction product was cooled, then, toluene (10 ml) was added and the liquid was separated, and the organic phase was liquid-separated and washed three times with ion exchanged water (10 ml). After dehydration with magnesium sulfate, it was filtrated and concentrated, to obtain a crude product. The resultant crude product was purified by silica gel column chromatography (a mixed solvent of hexane, toluene and ethanol), and concentrated under reduced pressure, and dried, to obtain a compound H-Et (2.83 g). The HPLC area percentage was 99.9%.

¹H-NMR (acetone-d6, 400 MHz): δ (ppm) = 8.03 (d, 2H), 7.91 (s, 2H), 7.80 (d, 2H), 7.74 (dd, 2H), 7.67-7.64 (m, 8H), 7.54-7.33 (m, 20H), 7.26-7.09 (m, 6H), 4.23-4.15 (m, 16H), 3.80 (t, 8H), 3.66-3.64 (m, 8H), 3.56-3.52 (m, 16H), 3.42 (d, 4H), 3.41 (d, 4H), 3.23 (s, 12H), 2.84 (s, 6H), 2.80 (s, 6H), 1.25 (t, 12H)

### <Example 26> (Synthesis of compound H)

An inert gas atmosphere was prepared in a reaction vessel, then, the compound H-Et (2.57 g), potassium hydroxide (0.51 g), tetrahydrofuran (15 ml), methanol (5 ml) and ion exchanged water (4 ml) were added, and the mixture was stirred at 60°C for 5 hours. The resultant reaction liquid was cooled down to room temperature, then, 1 N hydrochloric acid (11.5 g) and methyl isobutyl ketone (30 ml) were added, then, the liquid was separated, and the organic phase was liquid-separated and washed three times with 13 ml of ion exchanged water. The organic phase was concentrated, to obtain a crude product. The resultant crude product was recrystallized using a mixed solvent of tetrahydrofuran and hexane, to obtain a compound H (2.07 g). The HPLC area percentage was 99.8%.

¹H-NMR (CDCl₃, 400 MHz): δ (ppm) = 2.34 (6H, s), 2.45 (6H, s), 3.33 (12H, s), 3.49 to 3.52 (8H, m), 3.60 to 3.63 (8H, m), 3.64 to 3.68 (8H, m), 3.69 to 3.72 (8H, m), 3.87 to 3.90 (8H, m), 4.30 to 4.33 (8H, m), 6.90 (4H, d), 7.22 (2H, s), 7.28 (2H, s), 7.29 to 7.37 (10H, m), 7.39 to 7.44 (8H, m), 7.54 to 7.57 (6H, m), 7.64 (2H, dd), 7.71 (2H, s), 7.84 (2H, s), 8.15 (4H, d), 10.99 (4H, brs)

### <Synthesis of compound I>

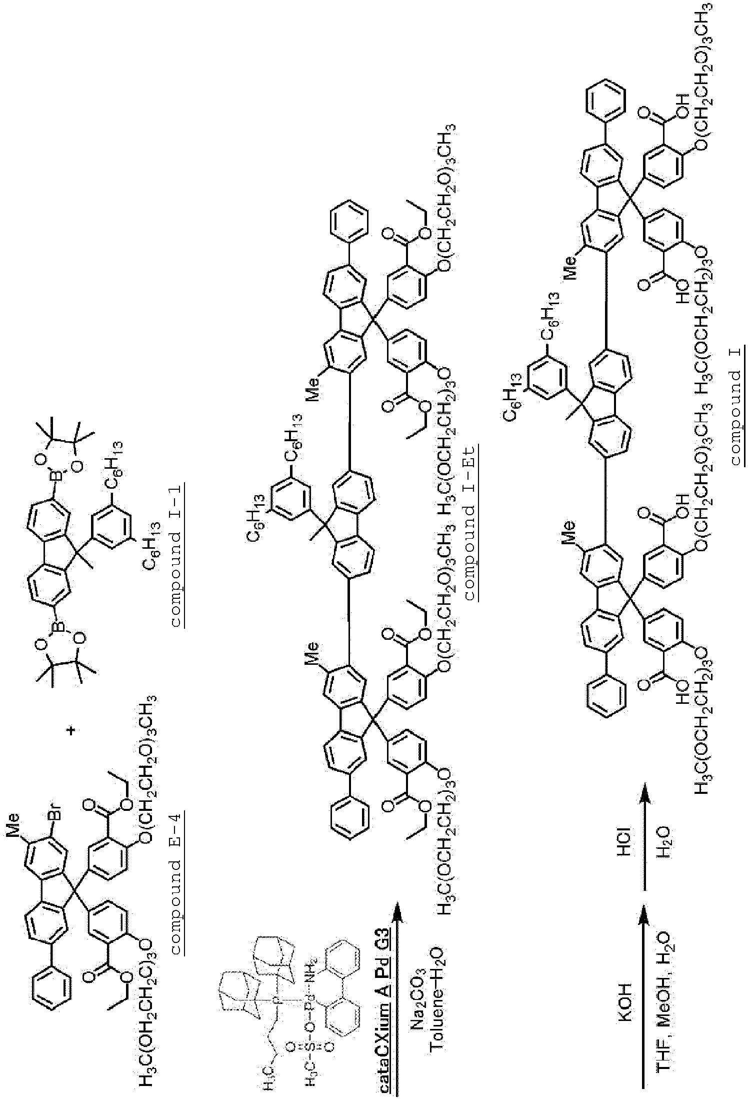

### <Example 27> (Synthesis of compound I-Et)

A compound I-1 was synthesized according to a method described in International Publication WO2012/086670.

An inert gas atmosphere was prepared in a reaction vessel, then, the compound I-1 (0.53 g), the compound E-4 (1.54 g), sodium carbonate (0.25 g), Aliquat336 (0.032 g), cataCXium A Pd G3 (manufactured by Sigma-Aldrich Inc.) (2.9 mg), toluene (6.7 g) and ion exchanged water (4.2 g) were added, and the mixture was stirred at 85°C for 7 hours. The reaction product was cooled, then, toluene was added and the liquid was separated, and the organic phase was liquid-separated and washed with ion exchanged water. After dehydration with magnesium sulfate, it was filtrated and concentrated, to obtain a crude product. The resultant crude product was purified by silica gel column chromatography (a mixed solvent of hexane, toluene and ethanol), and concentrated under reduced pressure, and dried, to obtain a compound I-Et (1.70 g). The HPLC area percentage was 99.7%. The detection wavelength was 300 nm.

¹H-NMR (CDCl₃, 400 MHz): δ (ppm) = 7.77 (4H, t), 7.53 to 7.62 (14H, m), 7.37 to 7.42 (4H, m), 7.21 to 7.32 (12H, m), 6.78 to 6.83 (7H, m), 4.12 to 4.24 (8H, m), 4.12 (8H, t), 3.84 (8H, t), 3.70 to 3.73 (8H, m), 3.59 to 3.63 (16H, m), 3.49 to 3.52 (8H, m), 3.33 (12H, d), 2.41 to 2.46 (4H, m), 2.24 (6H, s), 1.88 (3H, s), 1.20 to 1.28 (28H, m), 0.82 (6H, t)

### <Example 28> (Synthesis of compound I)

An inert gas atmosphere was prepared in a reaction vessel, then, the compound I-Et (1.60 g), potassium hydroxide (0.29 g), tetrahydrofuran (8.0 ml), methanol (2.4 ml) and ion exchanged water (2.0 ml) were added, and the mixture was stirred at 60°C for 2.5 hours. The resultant reaction liquid was cooled down to room temperature, then, 1 N hydrochloric acid (12.8 g) and methyl isobutyl ketone (16 ml) were added, then, the liquid was separated, and the liquid was separated and washed with ion exchanged water. The organic phase was concentrated, to obtain a crude product. The resultant crude product was recrystallized using a mixed solvent of ethyl acetate and hexane, to obtain a compound I (0.98 g). The HPLC area percentage was 99.8%. The detection wavelength was 300 nm.

¹H-NMR (CDCl₃, 400 MHz): δ (ppm) = 10.88 (4H, s), 8.11 (4H, dd), 7.75 to 7.81 (4H, dd), 7.58 to 7.63 (4H, m), 7.51 to 7.55 (8H, m), 7.39 (4H, t), 7.28 to 7.33 (6H, m), 7.20 to 7.23 (4H, m), 6.83 to 6.88 (6H, m), 6.78 (1H, s), 4.27 to 4.31 (8H, m), 3.85 to 3.89 (8H, m), 3.58 to 3.71 (24H, m), 3.47 to 3.51 (8H, m), 3.32 (6H, s), 3.31 (6H, s), 2.41 to 2.46 (4H, m), 2.25 (6H, s), 1.88 (3H, s), 1.42 to 1.51 (4H, t), 1.17 to 1.27 (12H, m), 0.71 to 0.83 (6H, m)

### <Synthesis of compound L>

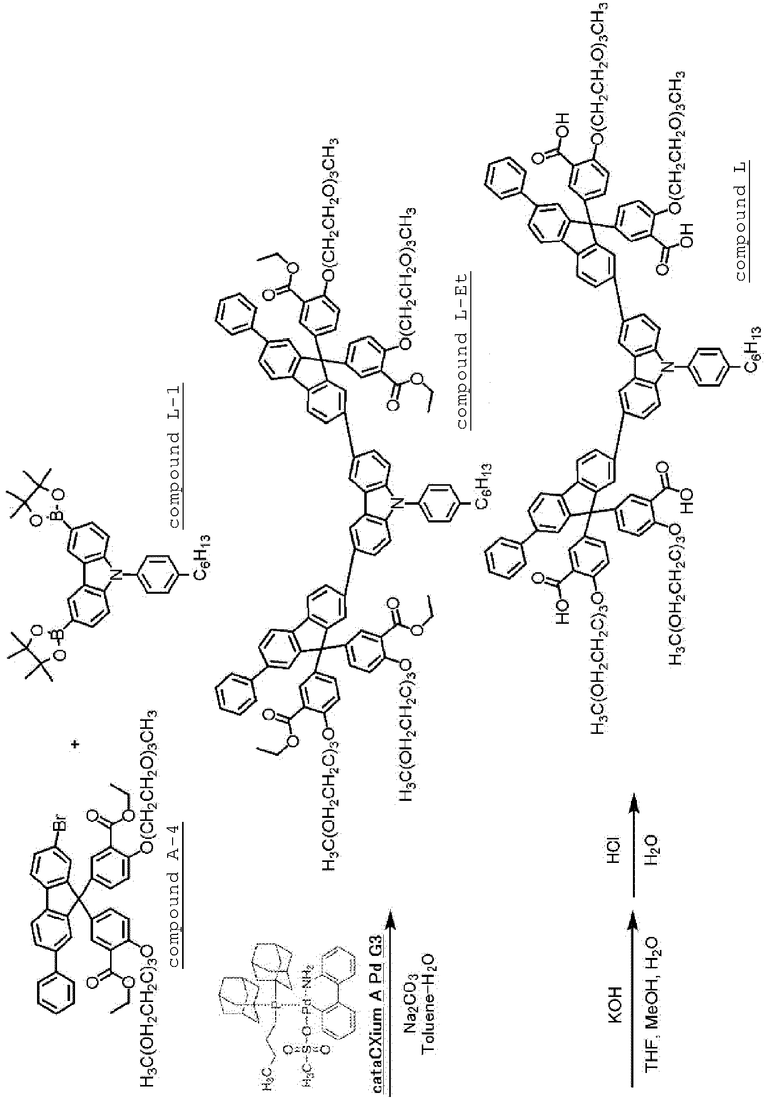

### <Example 29> (Synthesis of compound L-Et)

A compound L-1 was synthesized with reference to a method described in Thin Solid Films 209 (2006) 127.

An inert gas atmosphere was prepared in a reaction vessel, then, the compound L-1 (0.99 g), the compound A-4 (3.61 g), sodium carbonate (0.91 g), Aliquat336 (0.10 g), cataCXium A Pd G3 (manufactured by Sigma-Aldrich Inc.) (6.3 mg), toluene (20 ml) and ion exchanged water (14.9 g) were added, and the mixture was stirred at 90°C for 3 hours. The reaction product was cooled, then, toluene was added and the liquid was separated, and the organic phase was liquid-separated and washed with ion exchanged water. After dehydration with magnesium sulfate, it was filtrated and concentrated, to obtain a crude product. The resultant crude product was purified by silica gel column chromatography (a mixed solvent of toluene and ethanol), and concentrated under reduced pressure, and dried, to obtain a compound L-Et (2.90 g). The HPLC area percentage was 99.3%.

### <Example 30> (Synthesis of compound L)

An inert gas atmosphere was prepared in a reaction vessel, then, the compound L-Et (2.49 g), potassium hydroxide (0.48 g), tetrahydrofuran (24.9 ml), methanol (4.9 ml) and ion exchanged water (6.2 ml) were added, and the mixture was stirred at 60°C for 2 hours. The resultant reaction liquid was cooled down to room temperature, then, 1 N hydrochloric acid (25 g) and chloroform (50 ml) were added, then, the liquid was separated, and the liquid was separated and washed with ion exchanged water. The organic phase was concentrated, to obtain a crude product. The resultant crude product was recrystallized using a mixed solvent of tetrahydrofuran and heptane, to obtain a compound L (2.20 g). The HPLC area percentage was 99.7%.

¹H-NMR (CD₂Cl₂, 400 MHz): δ (ppm) = 0.89 (3H, t), 1.34-1.68 (12H, m), 2.72 (2H, m), 3.22 (12H, s), 3.40 (8H, m), 3.48-3.62 (24H, m), 3.84 (4H, m), 4.29 (8H, m), 6.95 (4H, d), 7.31-7.46 (16H, m), 7.55-7.71 (12H, m), 7.91 (6H, m), 8.09 (4H, d), 8.43 (2H, s), 10.92 (4H, s)

### <Synthesis of compound M>

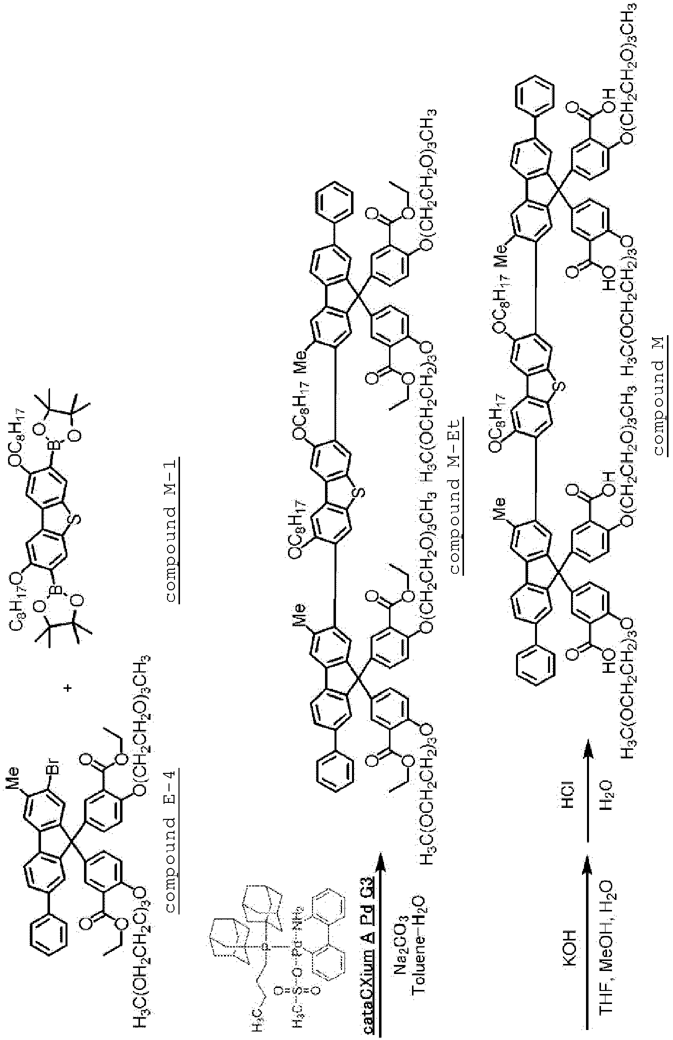

### <Example 31> (Synthesis of compound M-Et)

A compound M-1 was synthesized according to a method described in International Publication WO2006/11643.

An inert gas atmosphere was prepared in a reaction vessel, then, the compound M-1 (0.69 g), the compound E-4 (2.00 g), sodium carbonate (0.32 g), Aliquat336 (0.04 g), cataCXium A Pd G3 (manufactured by Sigma-Aldrich Inc.) (3.6 mg), toluene (10.3 g) and ion exchanged water (6.2 g) were added, and the mixture was stirred at 90°C for 2 hours. The reaction product was cooled, then, toluene was added and the liquid was separated, and the organic phase was liquid-separated and washed with ion exchanged water. After dehydration with magnesium sulfate, it was filtrated and concentrated, to obtain a crude product. The resultant crude product was purified by silica gel column chromatography (a mixed solvent of hexane, toluene and ethanol), and concentrated under reduced pressure, and dried, to obtain a compound M-Et (1.76 g). The HPLC area percentage was 99.3%.

¹H-NMR (CDCl₃, 400 MHz): δ (ppm) = 0.83 (6H, t), 1.14 to 1.30 (30H, m), 1.57 to 1.66 (6H, m), 2.26 (6H, brs), 3.33 (12H, s), 3.48 to 3.52 (8H, m), 3.59 to 3.65 (16H, m), 3.70 to 3.75 (8H, m), 3.81 to 3.87 (8H, m), 3.97 to 4.03 (4H, m), 4.08 to 4.17 (8H, m), 4.19 to 4.27 (8H, m), 6.74 to 6.89 (4H, m), 7.12 to 7.18 (4H, m), 7.26 to 7.35 (4H, m), 7.38 to 7.43 (4H, m), 7.53 to 7.70 (18H, m), 7.81 (2H, d)

### <Example 32> (Synthesis of compound M)

An inert gas atmosphere was prepared in a reaction vessel, then, the compound M-Et (1.61 g), potassium hydroxide (0.29 g), tetrahydrofuran (8.0 g), methanol (2.4 g) and ion exchanged water (2.4 g) were added, and the mixture was stirred at 60°C for 2 hours. The resultant reaction liquid was cooled down to room temperature, then, 1 N hydrochloric acid (6 g) and methyl isobutyl ketone (20 ml) were added, then, the liquid was separated, and the liquid was separated and washed with ion exchanged water. The organic phase was concentrated, to obtain a crude product. The resultant crude product was recrystallized using a mixed solvent of butyl acetate and heptane, to obtain a compound M (1.26 g). The HPLC area percentage was 99.6%. The detection wavelength was 300 nm.

¹H-NMR (CDCl₃, 400 MHz): δ (ppm) = 0.85 (6H, t), 1.16 to 1.29 (20H, m), 1.55 to 1.67 (4H, m), 2.28 (6H, s), 3.34 (12H, s), 3.49 to 3.52 (8H, m), 3.59 to 3.63 (8H, m), 3.64 to 3.68 (8H, m), 3.69 to 3.73 (8H, m), 3.86 to 3.91 (8H, m), 4.01 (4H, t), 4.27 to 4.35 (8H, m), 6.81 to 6.95 (4H, m), 7.21 to 7.24 (2H, m), 7.29 to 7.37 (6H, m), 7.38 to 7.44 (4H, m), 7.51 to 7.58 (10H, m), 7.61 to 7.65 (2H, m), 7.69 (2H, s), 7.83 (2H, d), 8.09 to 8.20 (4H, m), 11.00 (4H, s)

### <Synthesis of compound N>

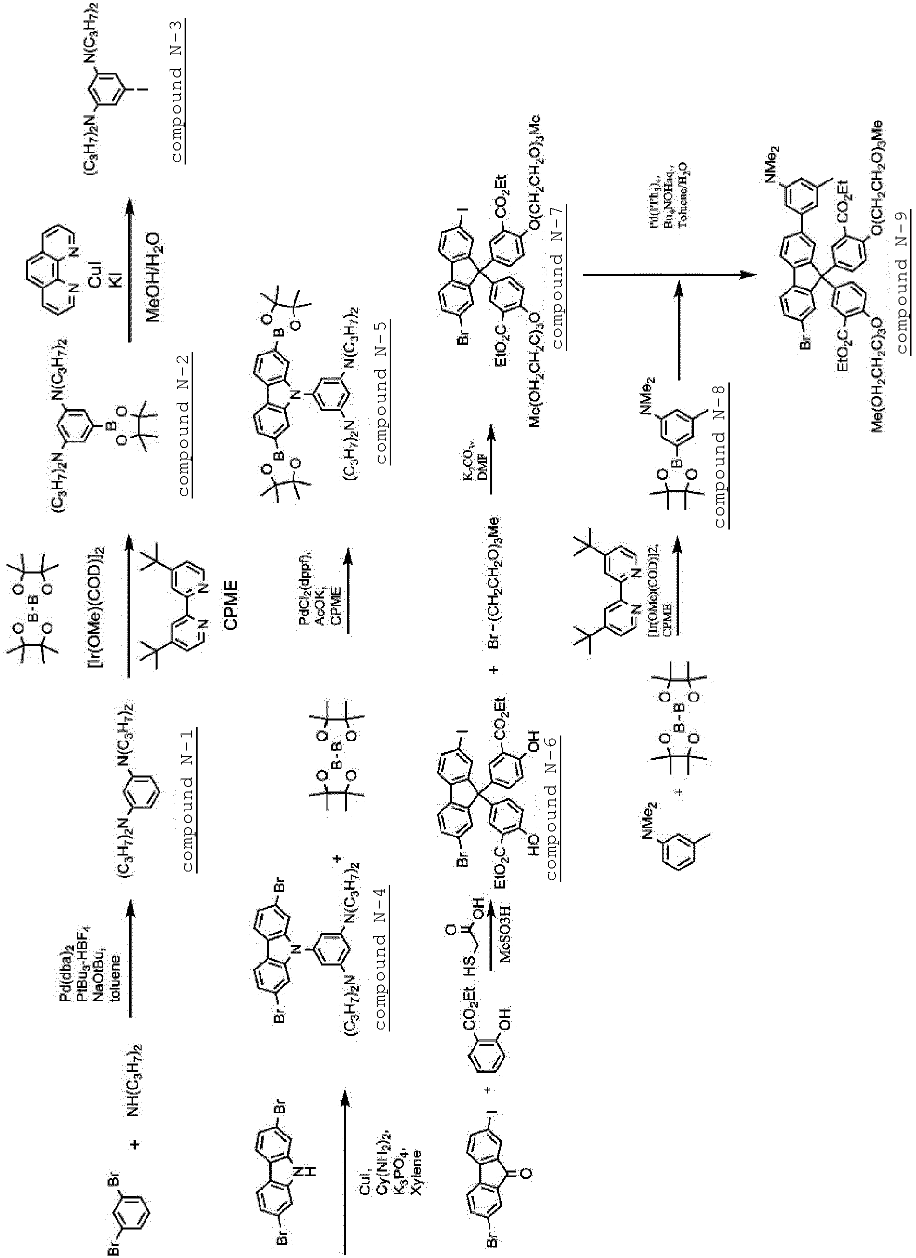

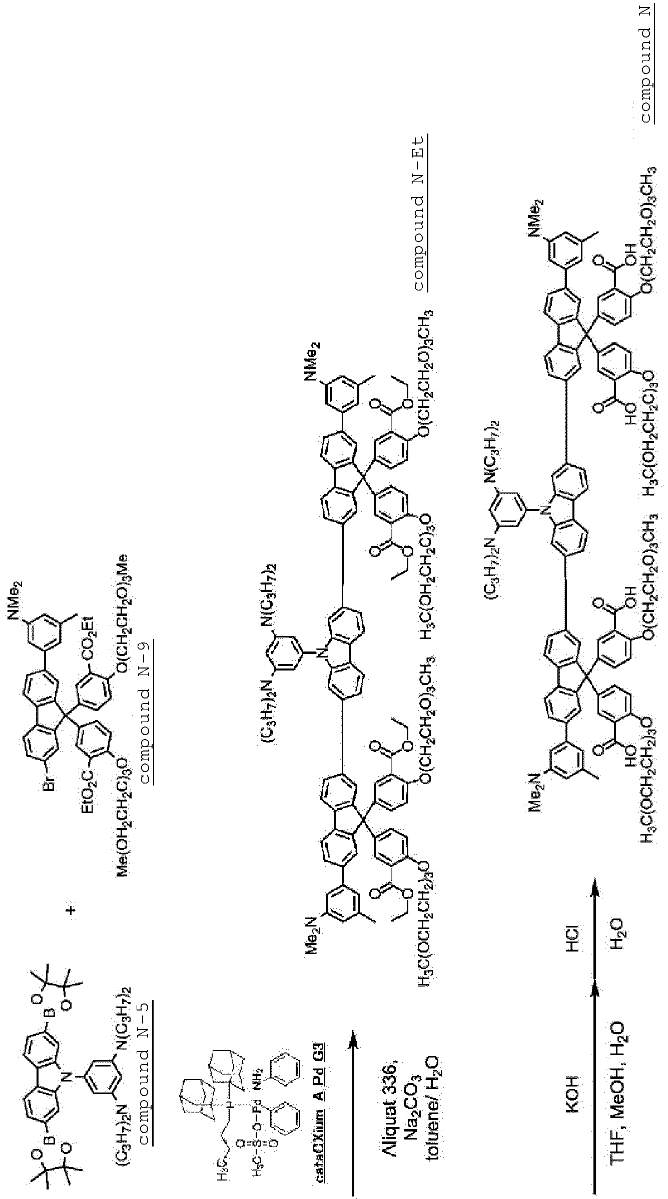

### <Synthesis Example 6> (Synthesis of compound N-1)

An inert gas atmosphere was prepared in a reaction vessel, then, 1,3-dibromobenzene (37.76 g), dipropylamine (35.52 g), tris(dibenzylideneacetone)dipalladium(0) (1.46 g), tri-tert-butylphosphonium tetrafluoroborate (1.85 g), sodium tert-butoxide (45.91 g) and dehydrated toluene (380 ml) were added, and the mixture was stirred at 80°C for 3 hours. The resultant reaction liquid was cooled down to room temperature, then, water was added and the liquid was separated and washed, then, dehydrated with magnesium sulfate. Magnesium sulfate was removed by filtration, then, the liquid was concentrated under reduced pressure at 50°C, and dried, to obtain a crude product of a compound N-1.

After purifying by silica gel column chromatography (a mixed solvent of hexane and ethyl acetate), it was concentrated under reduced pressure at 50°C and dried, to obtain a compound N-1 (34.13 g). The GC area percentage was 99.8%.

TLC-MS (DART): m/z = 277.37 ([M+H]⁺)

Exact Mass: 276.26

### <Synthesis Example 7> (Synthesis of compound N-2)

An inert gas atmosphere was prepared in a reaction vessel, then, the compound N-1 (27.82 g), bis(pinacolato)diboron (50.97 g), (1,5-cyclooctadiene)(methoxy)iridium(I) dimer (1.33 g), 4,4'-di-tert-butyl-2,2'-bipyridyl (1.07 g) and dehydrated cyclopentyl methyl ether (280 ml) were added, and the mixture was stirred at the reflux temperature for 16 hours. The resultant reaction liquid was cooled down to room temperature, then, the reaction liquid was dropped into a reaction vessel containing water (560 ml) added. After liquid separation, further, water was added and the liquid was separated and washed, then, dehydrated with magnesium sulfate. Magnesium sulfate was removed by filtration, then, the liquid was concentrated under reduced pressure at 50°C, and dried, to obtain a crude product of a compound N-2.

The resultant crude product was dissolved in toluene, and the solution was filtrated through silica gel. The resultant filtrate was concentrated under reduced pressure at 50°C and dried, to obtain a compound N-2 (36.30 g). The GC area percentage was 88.3%.

¹H-NMR (CDCl₃, 400 MHz): δ (ppm) = 0.889 to 0.926 (12H, m), 1.30 (122H, s), 1.55 to 1.65 (8H, m), 3.16 to 3.25 (8H, q), 6.02 (1H, t), 6.48 (2H, d)

### <Synthesis Example 8> (Synthesis of compound N-3)

An inert gas atmosphere was prepared in a reaction vessel, then, the compound N-2 (25.00 g), copper iodide (I) (1.05 g), 1,10-phenanthroline (2.00 g), potassium iodide (13.78 g), methanol (250 ml) and water (62.5 ml) were added, and the mixture was stirred at 70°C for 28 hours. Toluene was added and the liquid was separated, and the organic phase was further liquid-separated and washed with water and dehydrated with magnesium sulfate. Magnesium sulfate was removed by filtration, then, the liquid was concentrated under reduced pressure at 50°C, and dried, to obtain a crude product of a compound N-3.

The resultant crude product was purified by silica gel column chromatography (a mixed solvent of hexane and ethyl acetate), then, concentrated under reduced pressure at 50°C and dried, to obtain a compound N-3 (9.54 g). The GC area percentage was 99.9%.

### <Synthesis Example 9> (Synthesis of compound N-4)

An inert gas atmosphere was prepared in a reaction vessel, then, the compound N-3 (1.73 g), 2,7-dibromocarbazole (3.06 g), trans-1,2-cyclohexanediamine (1.73 g), copper iodide (I) (2.40 g) and dehydrated xylene (70 ml) were added, and the mixture was stirred at 85°C for 1.5 hours. Heating was stopped once, and 2,7-dibromocarbazole (1.55 g) was additionally added, and the mixture was further stirred at 85°C for 3 hours. After cooling down to room temperature, toluene (25 ml) was added to the reaction mass, and it was filtrated through a Kiriyama funnel paved with Celite. It was concentrated under reduced pressure at 50°C, and dried, to obtain a crude product of a compound N-4.

The resultant crude product was purified by silica gel column chromatography (a mixed solvent of hexane and toluene), then, Recrystallization was performed with an acetonitrile solvent, to obtain a compound N-4 (3.56 g). The GC area percentage was 97.8%.

TLC-MS (DART): m/z = 598.09 ([M+H]⁺)

Exact Mass: 597.14

### <Synthesis Example 10> (Synthesis of compound N-5)

An inert gas atmosphere was prepared in a reaction vessel, then, the compound N-4 (2.95 g), bis(pinacolato)diboron (2.75 g), potassium acetate (2.84 g), [1,1'-bis(diphenylphosphino)ferrocene]palladium(II) dichloride dichloromethane adduct (0.20 g) and dehydrated cyclopentyl methyl ether (30 ml) were added, and the mixture was stirred at 100°C for 8 hours. After allowing to cool to room temperature, it was filtrated through a Kiriyama funnel paved with Celite, concentrated under reduced pressure at 50°C, and dried, to obtain a crude product of a compound N-5.

The resultant crude product was dissolved in toluene, activated carbon was added and the mixture was stirred for 30 minutes, then, it was filtrated through a Kiriyama funnel paved with Celite, concentrated under reduced pressure at 50°C, and dried. Recrystallization was performed with an acetonitrile solvent, to obtain a compound N-5 (2.37 g). The HPLC area percentage was 99.2%.

¹H-NMR (CDCl₃, 400 MHz): δ (ppm) = 0.93 (12H, t), 1.34 (24H, s), 1.65 to 1.75 (8H, m), 3.25 (8H, t), 5.93 to 5.95 (1H, m), 6.18 (2H, d), 7.69 (2H, dd), 9.13 (2H, d), 8.15 (2H, d)

### <Synthesis Example 11> (Synthesis of compound N-6)

An inert gas atmosphere was prepared in a reaction vessel, then, 2-bromo-7-iodo-9H-fluoren-9-one (100.12 g), ethyl salicylate (259.62 g), mercaptoacetic acid (2.40 g) and methanesulfonic acid (249.90 g) were added, and the mixture was stirred at 65°C for 6.5 hours. After allowing to cool to room temperature, chloroform (1.50 L) was added to dissolve the solid, then, methanol (7.50 L) was added, and the solution was dropped into a vessel cooled to 0°C, to cause reprecipitation thereof. After filtration, it was dried under reduced pressure at 50°C, to obtain 155.54 g of a crude product of a compound N-6.

Recrystallization was performed from a mixed solvent of toluene and acetonitrile, to obtain a compound N-6 (119.08 g). The HPLC area percentage was 99.5%.

### <Synthesis Example 12> (Synthesis of compound N-7)

An inert gas atmosphere was prepared in a reaction vessel, then, the compound N-6 (94.50 g), diethylene glycol 2-bromoethyl methyl ether (70.63 g), potassium carbonate (56.03 g) and N,N-dimethylformamide (500 ml) were added, and the mixture was stirred at 70°C for 9 hours. After cooling down to room temperature, toluene and water were added and the liquid was separated and washed, and dehydrated with magnesium sulfate. Magnesium sulfate was removed by filtration, then, the liquid was concentrated under reduced pressure at 50°C, and dried, to obtain a crude product of a compound N-7.

The resultant crude product was purified by silica gel column chromatography (ethyl acetate solvent), then, concentrated under reduced pressure at 50°C and dried, to obtain a compound N-7 (67.70 g).

The HPLC area percentage was 99.6%.

LC-MS (ESI positive): m/z = 991.2 ([M+H]⁺)

Exact Mass: 990.2

### <Synthesis Example 13> (Synthesis of compound N-8)

An inert gas atmosphere was prepared in a reaction vessel, then, 3-dimethylaminotoluene (5.00 g), bis(pinacolato)diboron (11.22 g), (1,5-cyclooctadiene)(methoxy)iridium(I) dimer (0.29 g), 4,4'-di-tert-butyl-2,2'-bipyridyl (0.24 g) and dehydrated cyclopentyl methyl ether (50 ml) were added, and the mixture was stirred at the reflux temperature for 17 hours. The reaction liquid was cooled down to 0°C, then, methanol (25 ml) was dropped, and activated white earth was added and the mixture was stirred, then, filtrated. The filtrate was concentrated under reduced pressure at 50°C and dried, to obtain a crude product of a compound N-8.

The resultant crude product was dissolved in toluene, and the solution was washed with toluene through a Kiriyama funnel paved with silica gel. It was concentrated under reduced pressure at 50°C, and dried, then, recrystallization was performed with a methanol solvent, to obtain a compound N-8 (5.51 g).

The GC area percentage was 98.2%.

TLC-MS (DART): m/z = 262.16 ([M+H]⁺)

Exact Mass: 261.19

¹H-NMR (CDCl₃, 400 MHz): δ (ppm) = 1.34 (12H, s), 2.32 (3H, s), 2.95 (6H, s), 6.68 (1H, s), 7.00 to 7.03

### (2H, m)

### <Example 33> (Synthesis of compound N-9)

An inert gas atmosphere was prepared in a reaction vessel, then, the compound N-7 (10.85 g), the compound N-8 (2.90 g), tetrakis(triphenylphosphine)palladium(0) (0.25 g), a 40% tetrabutylammonium hydroxide aqueous solution (17.7 g), water (53.0 g) and toluene (160 ml) were added, and the mixture was stirred at 70°C for 12 hours. The reaction liquid was allowed to cool to room temperature, then, the liquid was separated, and the organic phase was further liquid-separated and washed with water. Thereafter, activated carbon was added to the organic phase and the mixture was stirred for 1 hour, then, washed with toluene through a Kiriyama funnel paved with silica gel. The filtrate was concentrated under reduced pressure at 50°C, and dried, to obtain a crude product of a compound N-9.

After purifying by reverse phase silica gel chromatography (a mixed solvent of water and acetonitrile), it was concentrated under reduced pressure at 50°C and dried, to obtain a compound N-9 (4.57 g). The HPLC area percentage was 99.5%.

TLC-MS (DART): m/z = 998.23 ([M+H]⁺)

Exact Mass: 997.36

¹H-NMR (CDCl₃, 400 MHz): δ (ppm) = 1.28 (6H, t), 2.35 (3H, s), 2.96 (6H, s), 3.35 (6H, s), 3.51 to 3.75 (4H, m), 3.61 to 3.66 (8H, m), 3.72 to 4.75 (4H, m), 3, 84 to 3.88 (4H, m), 4.13 to 4.17 (4H, m), 4.25 (4H, q), 6.54 (1H, brs), 6.68 (2H, d), 6.85 (2H, d), 7.24 (2H, dd), 7.45 to 7.51 (3H, m), 7.56 to 7.60 (3H, m), 7.62 (1H, d), 7.74 (1H, d)

### <Example 34> (Synthesis of compound N-Et)

An inert gas atmosphere was prepared in a reaction vessel, then, the compound N-5 (0.98 g), the compound N-9 (2.88 g), toluene (15 ml), Aliquat336 (0.057 g), sodium carbonate (0.45 g), water (7.87 g) and cataCXium A Pd G3 (manufactured by Sigma-Aldrich Inc.) (5.2 mg) were added, and the mixture was stirred at 80°C for 8 hours. The reaction liquid was cooled, then, toluene was added and the liquid was separated, and further the liquid was separated and washed with ion exchanged water, then, magnesium sulfate was added to the organic layer for dehydration. It was filtrated, concentrated under reduced pressure at 50°C, and dried, to obtain a crude product of a compound N-Et.

After purifying by reverse phase silica gel chromatography (a mixed solvent of acetonitrile and ethyl acetate), it was concentrated under reduced pressure at 50°C and dried, to obtain a compound N-Et (1.28 g). The HPLC area percentage was 98.3%.

¹H-NMR (CDCl₃, 400 MHz): δ (ppm) = 0.88 (12H, t), 1.25 (12H, t), 1.62 to 1.72 (8H, m), 2.36 (6H, s), 2.97 (12H, s), 3.21 to 3.27 (8H, m), 3.34 (12H, s), 3.49 to 3.53 (4H, m), 3.60 to 3.65 (16H, m), 3.71 to 3.75 (8H, m), 3.83 to 3.87 (8H, m), 4.11 to 4.15 (8H, m), 4.23 (8H, q), 5.94 (1H, s), 6.20 to 6.22 (2H, m), 6.55 (2H, s), 6.71 (4H, d), 6.83 (4H, d), 7.32 (4H, dd), 7.42 (2H, dd), 7.55 (2H, brs), 7.60 (2H, dd), 7.64 to 7.69 (8H, m), 7.77 to 7.81 (6H, m), 8.12 (2H, d)

### <Example 35> (Synthesis of compound N)

An inert gas atmosphere was prepared in a reaction vessel, then, the compound N-Et (1.22 g), potassium hydroxide (0.21 g), THF (6.1 g), methanol (1.8 g) and water (1.8 g) were added, and the mixture was stirred at 60°C for 12 hours. The reaction liquid was cooled down to 0°C, then, methyl isobutyl ketone (12.2 g) was added, and 1 N hydrochloric acid (4.4 g) was dropped. The liquid was separated, the organic phase was liquid-separated and washed with ion exchanged water, then, dehydrated with magnesium sulfate added. It was filtrated, concentrated at 40°C under reduced pressure, and dried, to obtain a crude product of a compound N.

Recrystallization was performed with a mixed solvent of methyl isobutyl ketone and hexane, to obtain a compound N (0.73 g). The HPLC area percentage was 97.2%.

¹H-NMR (CDCl₃, 400 MHz): δ (ppm) = 0.88 (12H, t), 1.62 to 1.72 (8H, m), 2.36 (6H, s), 2.97 (12H, s), 3.22 to 3.28 (8H, m), 3.33 (12H, s), 3.49 to 3.52 (4H, m), 3.59 to 3.63 (8H, m), 3.64 to 3.68 (8H, m), 3.69 to 3.72 (8H, m), 3.86 to 3.90 (8H, m), 4.28 to 4.32 (8H, m), 5.94 (1H, s), 6.20 (2H, brs), 6.54 (2H, s), 6.70 (4H, d), 6.86 (4H, d), 7.32 (4H, dd), 7.40 (2H, dd), 7.56 (2H, brs), 7.60 to 7.64 (4H, m), 7.77 to 7.82 (6H, m), 8.12 (2H, d), 8.21 (4H, d), 10.98 (4H, brs)

### <Synthesis of compound O>

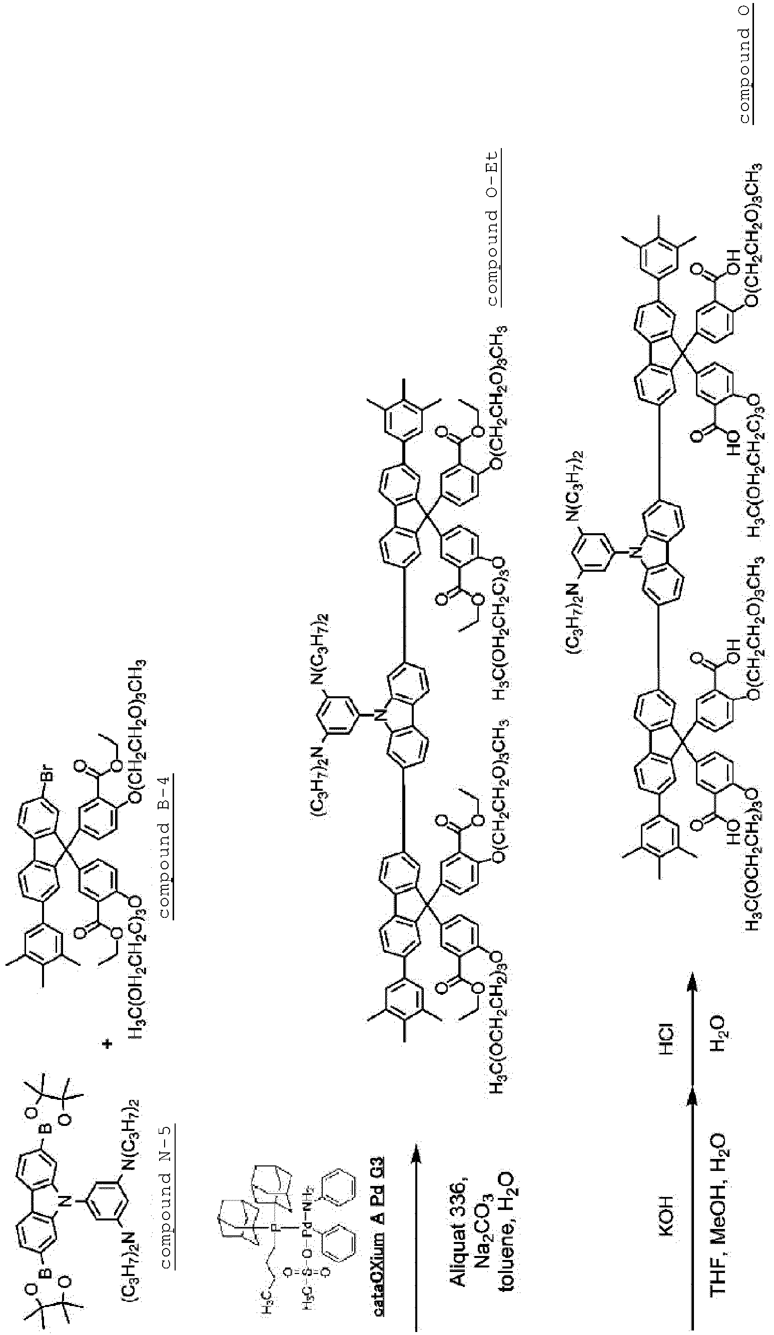

### <Example 36> (Synthesis of compound O-Et)

An inert gas atmosphere was prepared in a reaction vessel, then, the compound N-5 (0.47 g), the compound B-4 (1.45 g), cataCXium A Pd G3 (manufactured by Sigma-Aldrich Inc.) (2.5 mg), Aliquat336 (0.028 g), sodium carbonate (0.22 g), ion exchanged water (3.78 g) and toluene (6.6 g) were added, and the mixture was stirred at 80°C for 6 hours. After cooling down to room temperature, toluene (2.8 g) and ion exchanged water (4.0 g) were added, and the liquid was separated. The organic phase was further liquid-separated and washed with ion exchanged water, and magnesium sulfate and activated carbon were added, and the mixture was stirred for 1 hour. It was filtrated, and concentrated under reduced pressure at 50°C, and dried, to obtain a crude product of a compound O-Et.

After purifying by silica gel chromatography (a mixed solvent of hexane, toluene and ethanol), it was concentrated under reduced pressure at 50°C and dried, to obtain a compound O-Et (1.08 g). The HPLC area percentage was 98.9%.

¹H-NMR (CDCl₃, 400 MHz): δ (ppm) = 0.88 (12H, t), 1.25 (12H, t), 1.61 to 1.72 (8H, m), 2.20 (6H, s), 2.34 (12H, s), 3.21 to 3.27 (8H, m), 3.33 (12H, s), 3.49 to 3.52 (8H, m), 3.59 to 3.65 (16H, m), 3.71 to 3.74 (8H, m), 3.83 to 3.87 (8H, m), 4.12 to 4.15 (8H, m), 4.23 (8H, q), 5.94 (1H, brs), 6.21 (2H, brs), 6.83 (4H, d), 7.16 to 7.20 (4H, m), 7.32 (4H, dd), 7.41 (2H, dd), 7.52 (2H, d), 7.58 (2H, dd), 7.64 to 7.69 (8H, m), 7.77 to 7.81 (6H, m), 8.12 (2H, d)

### <Example 37> (Synthesis of compound O)

An inert gas atmosphere was prepared in a reaction vessel, then, the compound O-Et (0.90 g), potassium hydroxide (0.16 g), THF (4.5 g), methanol (1.4 g) and water (1.4 g) were added, and the mixture was stirred at 60°C for 3 hours. The reaction liquid was cooled down to 0°C, then, methyl isobutyl ketone (9.0 g) was added, and 1 N hydrochloric acid (3.3 g) was dropped. The liquid was separated, and the organic phase was further liquid-separated and washed with ion exchanged water, then, dehydrated with magnesium sulfate added. It was filtrated, and concentrated at 40°C under reduced pressure, and dried, to obtain a crude product of a compound O.

The resultant crude product was dissolved in THF, and the solution was dropped into hexane to cause reprecipitation thereof, to obtain a compound O (0.73 g). The HPLC area percentage was 99.1%.

LC-MS (ESI positive): m/z = 2135.0[M+H]⁺

Exact Mass: 2134.0

¹H-NMR (CDCl₃, 400 MHz): δ (ppm) = 0.88 (12H, t), 1.61 to 1.72 (8H, m), 2.19 (6H, s), 2.33 (12H, s), 3.22 to 3.27 (8H, m), 3.33 (12H, s), 3.49 to 3.52 (8H, m), 3.59 to 3.62 (8H, m), 3.63 to 3.67 (8H, m), 3.68 to 3.72 (8H, m), 3.86 to 3.89 (8H, m), 4.28 to 4.32 (8H, m), 5.94 (1H, brs), 6.20 (2H, brs), 6.87 (4H, d), 7.22 to 7.28 (4H, m), 7.32 (4H, dd), 7.40 (2H, dd), 7.51 (2H, brs), 7.59 (2H, dd), 7.62 (2H, brs), 7.67 (2H, dd), 7.77 to 7.82 (6H, m), 8.11 (2H, d), 8.20 (4H, d), 10.94 (4H, brs)

### <Synthesis of compound P>

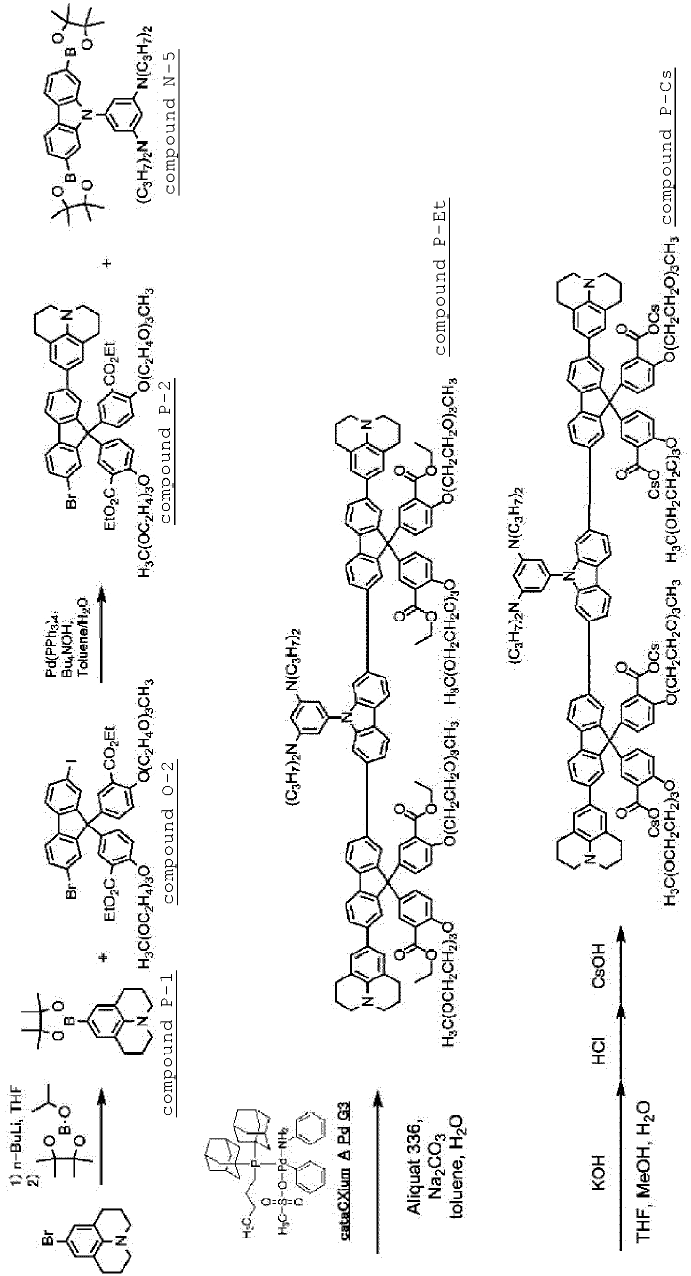

### <Synthesis Example 14> (Synthesis of compound P-1)

An inert gas atmosphere was prepared in a reaction vessel, then, 4-bromojulolidine (9.33 g) and THF (84 g) were added, and the mixture was cooled down to -70°C, and a hexane solution of n-butyllithium (1.55 M) (25.0 ml) was dropped. After stirring for 2 hours, 2-isopropoxy-4,4,5,5-tetramethyl-1,3,2-dioxaborolane (12.2 ml) was dropped, and the mixture was stirred for 1 hour, then, the temperature was raised to 0°C. 2-Propanol (9.3 ml) was dropped, and the liquid was separated and washed with ion exchanged water and chloroform added, and the organic phase was concentrated at 40°C under reduced pressure, and dried, to obtain a crude product of a compound P-1.

The resultant crude product was purified by silica gel column chromatography (toluene solvent), then, concentrated at 40°C under reduced pressure, and dried, to obtain a compound P-1 (11.53 g). The GC area percentage was 94.1%.

TLC-MS (DART): m/z = 300.20 ([M+H]⁺)

Exact Mass: 299.21

### <Example 38> (Synthesis of compound P-2)

An inert gas atmosphere was prepared in a reaction vessel, then, the compound O-2 (15.15 g), the compound P-1 (5.17 g), tetrakis(triphenylphosphine)palladium(0) (0.35 g), a 40% tetrabutylammonium hydroxide aqueous solution (24.6 g), water (73.8 g) and toluene (230 ml) were added, and the mixture was stirred at 60°C for 15 hours. The reaction liquid was allowed to cool to room temperature, then, the liquid was separated, and the organic phase was further liquid-separated and washed with water, concentrated under reduced pressure at 50°C, and dried, to obtain a crude product of a compound P-2.

The resultant crude product was purified by reverse phase silica gel column chromatography (a mixed solvent of water and acetonitrile), then, concentrated at 40°C under reduced pressure, and dried, to obtain a compound P-2 (10.30 g). The HPLC area percentage was 99.3%.

TLC-MS (DART): m/z = 1036.3 ([M+H]⁺)

Exact Mass: 1035.38

¹H-NMR (CDCl₃, 400 MHz): δ (ppm) = 1.29 (6H, t), 1.94 to 2.01 (4H, m), 2.78 (4H, t), 3.16 (4H, t), 3.35 (6H, s), 3.51 to 3.54 (4H, m), 3.61 to 3.66 (8H, m), 3.72 to 3.75 (4H, m), 3.86 (4H, t), 4.15 (4H, t), 4.25 (4H, q), 6.85 (2H, d), 6.97 (2H, s), 7.23 (2H, dd), 7.40 to 7.44 (2H, m), 7.45 to 7.51 (2H, m), 7.56 to 7.60 (3H, m), 7.68 (1H, d)

### <Example 39> (Synthesis of compound P-Et)

An inert gas atmosphere was prepared in a reaction vessel, then, the compound N-5 (1.21 g), the compound P-2 (3.67 g), cataCXium A Pd G3 (manufactured by Sigma-Aldrich Inc.) (6.3 mg), Aliquat336 (0.070 g), sodium carbonate (0.55 g), ion exchanged water (9.7 g) and toluene (16.9 g) were added, and the mixture was stirred at 80°C for 15 hours. After cooling down to room temperature, the liquid was separated, the organic phase was further liquid-separated and washed with ion exchanged water, magnesium sulfate was added, and the mixture was stirred for 1 hour. It was filtrated, concentrated at 40°C under reduced pressure, and dried, to obtain a crude product of a compound P-Et.

The resultant crude product was purified by reverse phase silica gel column chromatography (a mixed solvent of acetonitrile and ethyl acetate), then, concentrated at 40°C under reduced pressure, and dried, to obtain a compound P-Et (2.60 g). The HPLC area percentage was 98.1%.

¹H-NMR (CDCl₃, 400 MHz): δ (ppm) = 0.88 (12H, t), 1.25 (12H, t), 1.61 to 1.73 (8H, m), 1.95 to 2.02 (8H, m), 2.80 (8H, t), 3.16 (8H, t), 3.21 to 3.26 (8H, m), 3.34 (12H, s), 3.49 to 3.53 (8H, m), 3.60 to 3.65 (16H, m), 3.71 to 3.75 (8H, m), 3.85 (8H, t), 4.13 (8H, t), 4.23 (8H, q), 5.94 (1H, s), 6.19 to 6.21 (2H, m), 6.82 (4H, d), 6.99 (4H, s), 7.31 (4H, dd), 7.39 to 7.42 (2H, m), 7.44 to 7.46 (2H, m), 7.49 to 7.53 (2H, m), 7.61 to 7.65 (4H, m), 7.68 (4H, d), 7.74 (4H, dd), 7.79 (2H, s), 8.10 (2H, d)

### <Example 40> (Synthesis of compound P-Cs)

An inert gas atmosphere was prepared in a reaction vessel, then, the compound P-Et (2.15 g), potassium hydroxide (0.36 g), THF (10.8 g), methanol (3.2 g) and water (3.2 g) were added, and the mixture was stirred at 60°C for 2 hours. The reaction liquid was cooled down to 0°C, then, chloroform (21.5 ml) was added, and 1 N hydrochloric acid (7.5 g) was dropped, and the mixture was stirred for 0.5 hours. The liquid was separated, and the organic phase was liquid-separated and washed with ion exchanged water, then, a solution prepared by dissolving cesium hydroxide mono-hydrate (0.74 g) in 2.2 ml of ion exchanged water and 15.1 ml of methanol was dropped at 0°C, and the mixture was stirred for 0.5 hours. The reaction liquid was dropped into acetonitrile, to cause reprecipitation thereof. The resultant solid was filtrated, and dried at 40°C under reduced pressure, to obtain a compound P-Cs (2.10 g). The HPLC area percentage was 97.0%.

¹H-NMR (CDCl₃, 400 MHz): δ (ppm) = 0.86 (12H, t), 1.61 to 1.72 (8H, m), 1.90 to 1.98 (8H, m), 2.73 (8H, t), 3.11 (8H, t), 3.24 (12H, s), 3.27 to 3.29 (8H, m), 3.42 to 3.45 (8H, m), 3.50 to 3.56 (16H, m), 3.59 to 3.62 (8H, m), 3.72 to 3.76 (8H, m), 4.08 to 4.12 (8H, m), 5.98 (1H, s), 6.16 (2H, s), 6.89 (4H, d), 6.93 (4H, s), 7.16 (4H, d), 7.32 (4H, dd), 7.40 (2H, d), 7.44 (2H, s), 7.46 (2H, d), 7.58 (2H, d), 7.62 (2H, s), 7.67 (2H, s), 7.73 to 7.79 (4H, m), 8.08 (2H, d)

### <Synthesis of compound Z>

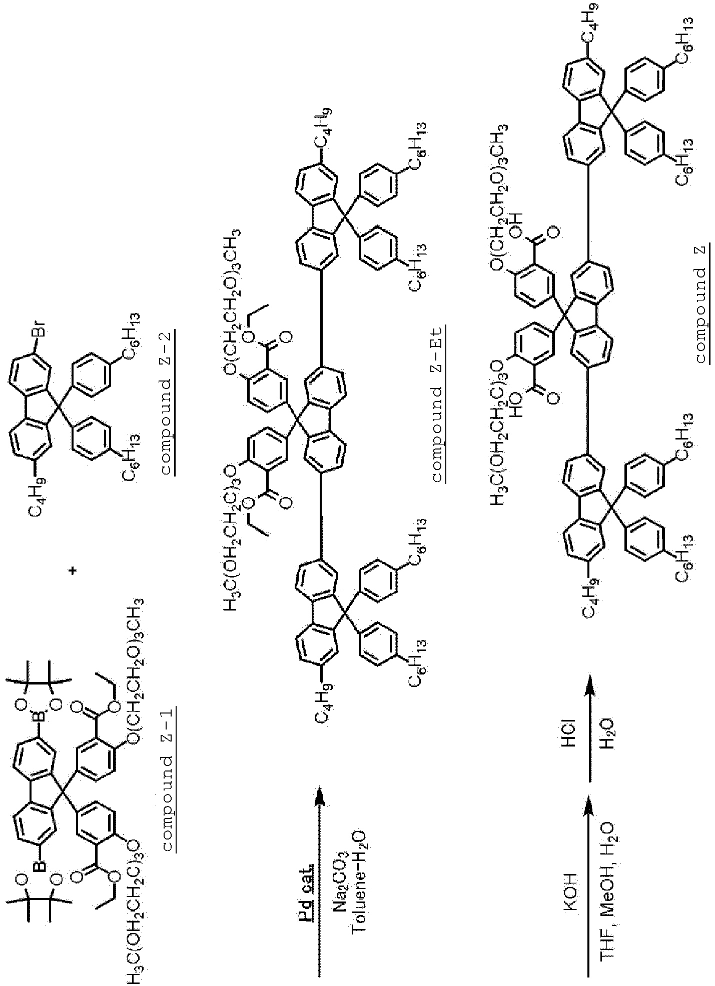

### <Synthesis Example 15> (Synthesis of compound Z-Et)

A compound Z-1 was synthesized according to a method described in International Publication WO2010/11705.

A compound Z-2 was synthesized according to a method described in JP-A No. 2016-176063.

An inert gas atmosphere was prepared in a reaction vessel, then, the compound Z-1 (2.00 g), the compound Z-2 (2.54 g), toluene (20 ml), Aliquat336 (0.02 g), a 10% by weight Na₂CO₃ aqueous solution (6.1 g) and dichlorobis[tris(2-methoxyphenyl)phosphine]palladium(II) (3.4 mg) were added, and the mixture was heated at 90°C and reacted for 8 hours. The reaction liquid was cooled, then, toluene was added and the liquid was separated, the liquid was separated and washed with ion exchanged water, then, magnesium sulfate was added to the organic layer and the mixture was stirred for 1 hour, then, filtrated, and concentrated under reduced pressure, to obtain a crude product.

The crude product was purified by a silica gel column (a mixed solvent of hexane, ethyl acetate and methanol), then, concentrated and dried, to obtain a compound Z-Et (1.85 g). The HPLC area percentage value was 99.7%.

¹H-NMR (CDCl₃, 400 MHz): δ (ppm) 0.83 to 0.92 (18H, m), 1.22 (6H, t), 1.24 to 1.37 (28H, m), 1.51 to 1.61 (12H, m), 2.52 (8H, t), 2.59 (4H, t), 3.33 (6H, s), 3.48 to 3.52 (4H, m), 3.59 to 3.64 (8H, m), 3.71 to 3.74 (4H, m), 3.82 to 3.86 (4H, m), 4.10 to 4.14 (4H, m), 4.20 (4H, q), 6.83 (2H, d), 7.02 (8H, d), 7.12 (8H, d), 7.12 to 7.19 (4H, m), 7.27 (2H, dd), 7.45 to 7.54 (6H, m), 7.57 (2H, brs), 7.62 to 7.65 (4H, m), 7.68 to 7.74 (4H, m)

### <Synthesis Example 16> (Synthesis of compound Z)

An inert gas atmosphere was prepared in a reaction vessel, then, the compound Z-Et (1.75 g), potassium hydroxide (0.16 g), tetrahydrofuran (13 mL), methanol (4.5 mL) and ion exchanged water (1.44 g) were added, and the mixture was heated at 70°C and reacted for 2 hours.

The resultant reaction liquid was cooled down to room temperature, then, chloroform was added, and the liquid was separated and washed with 1 N hydrochloric acid and ion exchanged water, and the organic layer was dried over magnesium sulfate added, and filtrated. The filtrate was concentrated under reduced pressure to obtain a compound Z (1.54 g).

¹H-NMR (CDCl₃, 400 MHz): δ (ppm) 0.82 to 0.91 (18H, m), 1.23 to 1.36 (28H, m), 1.51 to 1.60 (12H, m), 2.52 (8H, t), 2.58 (4H, t), 3.32 (6H, s), 3.47 to 3.50 (4H, m), 3.58 to 3.61 (4H, m), 3.62 to 3.66 (4H, m), 3.67 to 3.71 (4H, m), 3.84 to 3.88 (4H, m), 4.26 to 4.30 (4H, m), 6.84 (2H, d), 7.01 (8H, d), 7.11 (8H, d), 7.12 to 7.18 (4H, m), 7.21 to 7.26 (2H, m), 7.45 to 7.48 (2H, m), 7.50 to 7.57 (6H, m), 7.63 (2H, d), 7.69 (2H, d), 7.73 (2H, d), 8.18 (2H, d), 10.95 (2H, brs)

### <Synthesis of compound Y>

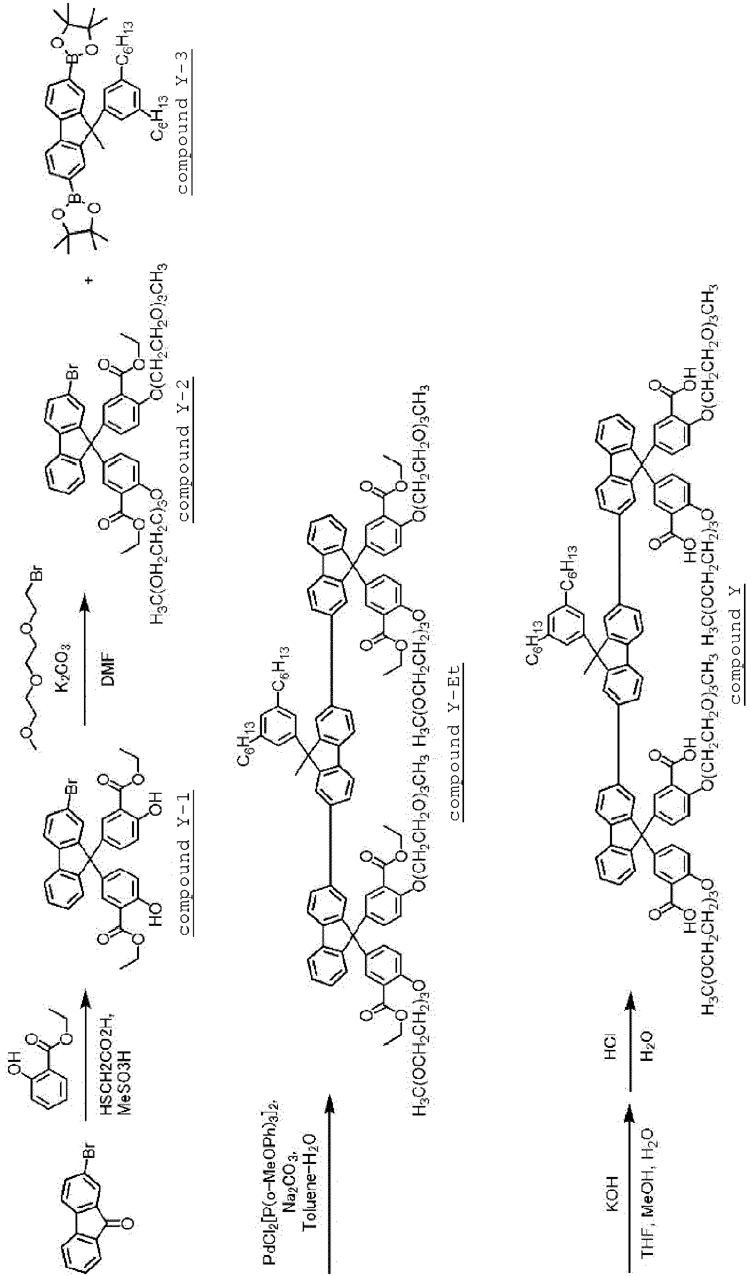

### <Synthesis Example 17> (Synthesis of compound Y-1)

A nitrogen gas atmosphere was prepared in a light-shielded reaction vessel, then, 2-bromo-9-fluorenone (19.99 g), ethyl salicylate (77.00 g), mercaptoacetic acid (0.65 g) and methanesulfonic acid (200 ml) were added, and the mixture was stirred at 65°C for 7.5 hours. The resultant reaction liquid was dropped into ice-cooled ion exchanged water (500 ml), and the aqueous phase was removed by decantation. Ion exchanged water (100 ml) was added and the mixture was stirred, and the aqueous phase was removed by decantation. The resultant viscous oil was dissolved in toluene (300 ml), dried over magnesium sulfate added, then, filtrated and concentrated, to obtain a crude product.

The resultant crude product was recrystallized using a mixed solvent of toluene and acetonitrile, to obtain 11.4 g of a compound Y-1. The HPLC area percentage value of the compound Y-1 was 99.2%.

TLC-MS (DART positive): m/z = 572[M]⁺

### <Synthesis Example 18> (Synthesis of compound Y-2)

A nitrogen gas atmosphere was prepared in a light-shielded reaction vessel, then, the compound Y-1 (11.00 g), diethylene glycol 2-bromoethyl methyl ether (10.59 g), potassium carbonate (7.32 g) and N,N-dimethylformamide (55 mL) were added, and the mixture was stirred at 90°C for 18 hours. The resultant reaction liquid was cooled down to room temperature, then, toluene was added and the mixture was stirred, and washed with ion exchanged water. To the resultant organic layer was added magnesium sulfate and they were stirred, and the resultant mixture was filtrated, and the resultant filtrate was concentrated under reduced pressure to obtain a crude product (17.85 g). The resultant crude product was purified by silica gel column chromatography (a mixed solvent of toluene and ethyl acetate), and dried under reduced pressure, to obtain a compound Y-2 (8.4 g). The HPLC area percentage value of the compound Y-2 was 99.3%.

### <Synthesis Example 19> (Synthesis of compound Y-Et)

A compound Y-3 was synthesized according to a method described in International Publication WO2012/086670.

A nitrogen gas atmosphere was prepared in a light-shielded reaction vessel, then, the compound Y-2 (1.96 g), the compound Y-3 (0.70 g), dichlorobis[tris(o-methoxyphenyl)phosphine]palladium (9.9 mg), Aliquat336 (0.040 g), sodium carbonate (0.34 g), ion exchanged water (2.8 g) and toluene (5.7 g) were added, and the mixture was stirred at 80°C for 9 hours. The resultant reaction liquid was cooled down to room temperature, then, toluene was added and the mixture was stirred, and the liquid was separated and washed with ion exchanged water. To the resultant organic layer was added magnesium sulfate and they were stirred, and the resultant mixture was filtrated, and the resultant filtrate was concentrated under reduced pressure to obtain a crude product. The resultant crude product was purified by silica gel column chromatography (a mixed solvent of hexane, toluene and ethanol), and dried under reduced pressure, to obtain a compound Y-Et (1.05 g). The HPLC area percentage value of the compound Y-Et was 98.3%.

¹H-NMR (CD₂Cl₂, 400 MHz): δ (ppm) 7.85-7.79 (m, 6H), 7.63-7.59 (m, 4H), 7.57-7.53 (m, 6H), 7.50 (d, 2H), 7.44-7.36 (m, 4H), 7.35-7.29 (m, 6H), 6.91-6.86 (m, 6H), 6.84 (s, 1H), 4.22 (q, 8H), 4.15-4.10 (m, 8H), 3, 84-3.79 (m, 8H), 3.70-3.65 (m, 8H), 3.61-3.55 (m, 16H), 3.50-3.46 (m, 8H), 3.31 (s, 12H), 2.48 (t, 4H), 1.94 (s, 3H), 1.55-1.46 (m, 4H), 1.29-1.18 (m, 24H), 0.85-0.79 (m, 6H)

### <Synthesis Example 20> (Synthesis of compound Y)

An inert gas atmosphere was prepared in a reaction vessel, then, the compound Y-Et (0.85 g), potassium hydroxide (0.15 g), tetrahydrofuran (4.4 g), methanol (1.3 g) and water (1.35 g) were added, and the mixture was heated at 60°C and reacted for 4 hours.

The resultant reaction liquid was cooled down to room temperature, then, methyl isobutyl ketone was added, and the liquid was separated and washed with 1 N hydrochloric acid and ion exchanged water, and the organic layer was dried with magnesium sulfate added, and filtrated. The filtrate was concentrated under reduced pressure, to obtain 0.68 g of a compound Y. The HPLC area percentage value of the compound Y was 97.6%.

¹H-NMR (CD₂Cl₂, 400 MHz): δ (ppm) 10.87 (br, 4H), 7.97 (d, 4H), 7.83-7.77 (m, 6H), 7.61-7.55 (m, 4H), 7.51 (dd, 2H), 7.45 (d, 2H), 7.43-7.26 (m, 10H), 6.95-6.90 (m, 4H), 6.85-6.78 (m, 3H), 4.32-4.26 (m, 8H), 3, 87-3.81 (m, 8H), 3.66-3.62 (m, 8H), 3.60-3.55 (m, 8H), 3.54-3.50 (m, 8H), 3.45-3.40 (m, 8H), 3.26 (s, 12H), 2.43 (t, 4H), 1.90 (s, 3H), 1.51-1.41 (m, 4H), 1.18 (br, 12H), 0.81-0.74 (m, 6H).

### <Synthesis of polymer compound PA>

A polymer compound PA, which is an alternating copolymer having a constitutional unit represented by the following formula, was synthesized according to a method described in International Publication WO2015/159932.

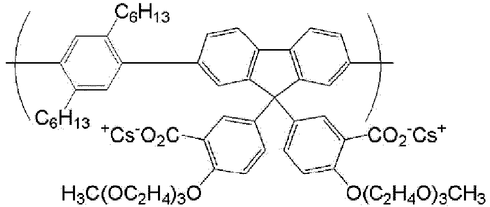

### <Synthesis of polymer compound PB>

A polymer compound PB represented by the following formula was synthesized according to a method described in International Publication WO2013/58160.

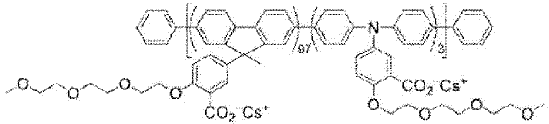

### <Synthesis of polymer compound PC>

A polymer compound PC represented by the following formula was synthesized according to a method described in JP-A No. 2012-33845.

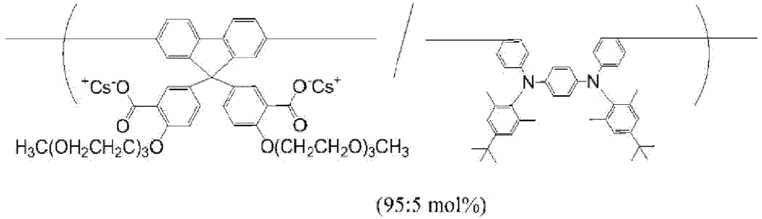

### <Synthesis of polymer compound PD>

A polymer compound PD represented by the following formula was synthesized according to a method described in JP-A No. 2013-168825.

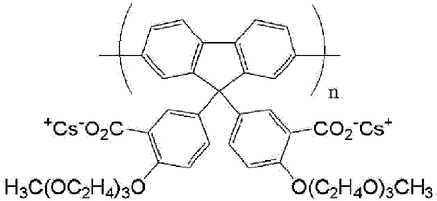

### <Synthesis of monomers CM1 to CM4>

Monomers CM1 to CM4 were synthesized according to methods described in the following literatures, and those showing HPLC area percentage values of 99.5% or more were used.

Monomers CM1 to CM3 were synthesized according to a method described in International Publication WO2013/146806.

A monomer CM4 was synthesized according to a method described in International Publication WO2009/157424.

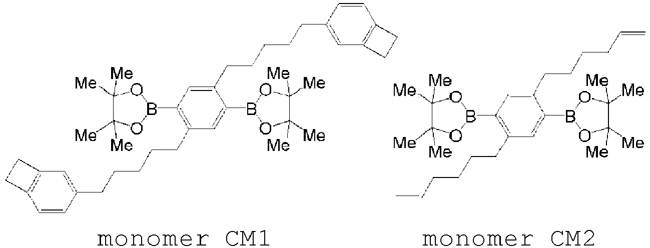

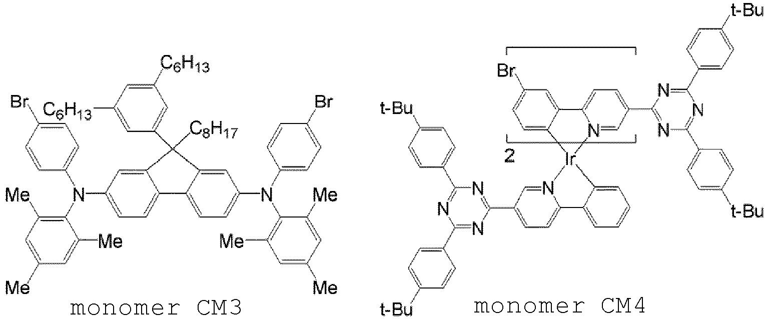

### <Synthesis of polymer compound 1>

An inert gas atmosphere was prepared in a reaction vessel, then, the monomer CM1 (2.52 g), the monomer CM2 (0.47 g), the monomer CM3 (4.90 g), the monomer CM4 (0.53 g) and toluene (158 mL) were added, and the mixture was heated at 95°C. To the reaction liquid were added a 20% by weight tetraethylammonium hydroxide aqueous solution (16 mL) and dichlorobis(tris-o-methoxyphenylphosphine)palladium (4.2 mg), and the liquid was refluxed for 8 hours. After the reaction, to this were added phenylboronic acid (0.12 g), a 20% by weight tetraethylammonium hydroxide aqueous solution (16 mL) and dichlorobis(tris-o-methoxyphenylphosphine)palladium (4.2 mg), and the liquid was refluxed for 15 hours. Thereafter, to this was added a sodium diethyldithiacarbamate aqueous solution, and the mixture was stirred at 85°C for 2 hours. After cooling, the reaction liquid was washed with a 3.6% by weight hydrochloric acid aqueous solution twice, with a 2.5% by weight ammonia aqueous solution twice, and with ion exchanged water four times, and the resultant solution was dropped into methanol, to generate a precipitate. The precipitate was dissolved in toluene, and purified by passing the solution through an alumina column and a silica gel column in this order. The resultant solution was dropped into methanol, and stirred, then, the resultant precipitate was collected by filtration, and dried, to obtain 6.02 g of a polymer compound 1. The polymer compound 1 had an Mn of 3.8×10⁴ and an Mw of 4.5×10⁵.

The polymer compound 1 is a copolymer constituted of a constitutional unit derived from the monomer CM1, a constitutional unit derived from the monomer CM2, a constitutional unit derived from the monomer CM3 and a constitutional unit derived from the monomer CM4 at a molar ratio of 40:10:47:3 according to the theoretical values calculated from the amounts of the charged raw materials.

### <Synthesis of phosphorescent compounds 1 and 2>

Phosphorescent compounds 1 and 2 were synthesized according to methods described in the following literatures, and those showing HPLC area percentage values of 99.5% or more were used.

The phosphorescent compound 1 was synthesized with reference to methods described in International Publication WO2006/121811 and JP-A No. 2013-048190.

The phosphorescent compound 2 was synthesized according to a method described in International Publication WO2009/131255.

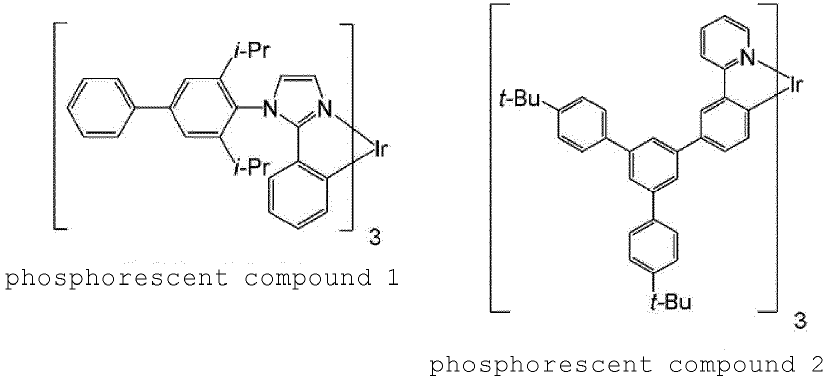

### <Example D1> Fabrication of light emitting device D1 (Formation of anode and hole injection layer)

An ITO film was attached with a thickness of 45 nm onto a glass substrate by a sputtering method, to form an anode. On the anode, a hole injection material ND-3202 manufactured by Nissan Chemical Corporation was spin-coated to form a film with a thickness of 35 nm, and under an air environment where ozone had been removed, the film was heated by a hot plate at 50°C for 3 minutes to volatilize the solvent, and subsequently, heated by a hot plate at 240°C for 15 minutes, to form a hole injection layer.

### (Formation of hole transporting layer)

The polymer compound 1 was dissolved at a concentration of 0.65% by weight in xylene. The resultant xylene solution was spin-coated on the hole injection layer to form a film with a thickness of 20 nm, and under a nitrogen gas atmosphere, the film was heated by a hot plate at 180°C for 60 minutes, to form a hole transporting layer.

### (Formation of light emitting layer)

A low-molecular host 1 (LT-N4013 manufactured by Luminescence Technology Corp.) represented by the following formula:

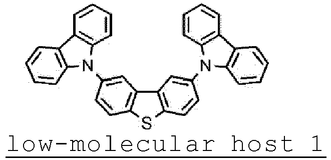

, the phosphorescent compound 1 and the phosphorescent compound 2 (weight ratio: low-molecular host 1/phosphorescent compound 1/phosphorescent compound 2 = 79/20/1) were dissolved in toluene at a concentration of 2.0% by weight, to prepare a toluene solution. This toluene solution was spin-coated on the hole transporting layer to form a film with a thickness of 75 nm, and under a nitrogen gas atmosphere, the film was heated at 130°C for 10 minutes, tor form a light emitting layer.

### (Formation of electron transporting layer)

The compound A-Cs was dissolved at a concentration of 0.25% by weight in 1H,1H,5H-octafluoropentanol, to prepare a 0.25% by weight 1H,1H,5H-octafluoropentanol solution of the compound A-Cs. This solution was spin-coated on the light emitting layer to form a film with a thickness of 10 nm, and under a nitrogen gas atmosphere, the film was heated at 130°C for 10 minutes, to form an electron transporting layer.

### (Formation of cathode and electron injection layer)

The substrate carrying the electron transporting layer formed thereon was placed in a vapor deposition machine, and the internal pressure was reduced to 1.0×10⁻⁴ Pa or less, then, as the cathode, sodium fluoride was vapor-deposited with a thickness of about 4 nm on the electron transporting layer, then, aluminum was vapor-deposited with a thickness of about 100 nm on this. Thereafter, sealing was performed using a glass substrate, to fabricate a light emitting device D1.

### <Example D2> Fabrication of light emitting device D2

A light emitting device D2 was fabricated in the same manner as in Example D1, except that an electron transporting layer was formed using a 1H,1H,5H-octafluoropentanol solution of a compound B-Cs prepared by dissolving the compound B at a concentration of 0.25% by weight and cesium hydroxide mono-hydrate at a concentration of 0.08% by weight in 1H,1H,5H-octafluoropentanol and stirring the solution at room temperature for 1 hour, instead of the 0.25% by weight 1H,1H,5H-octafluoropentanol solution of the compound A-Cs. The compound B-Cs is estimated to have the following structure.

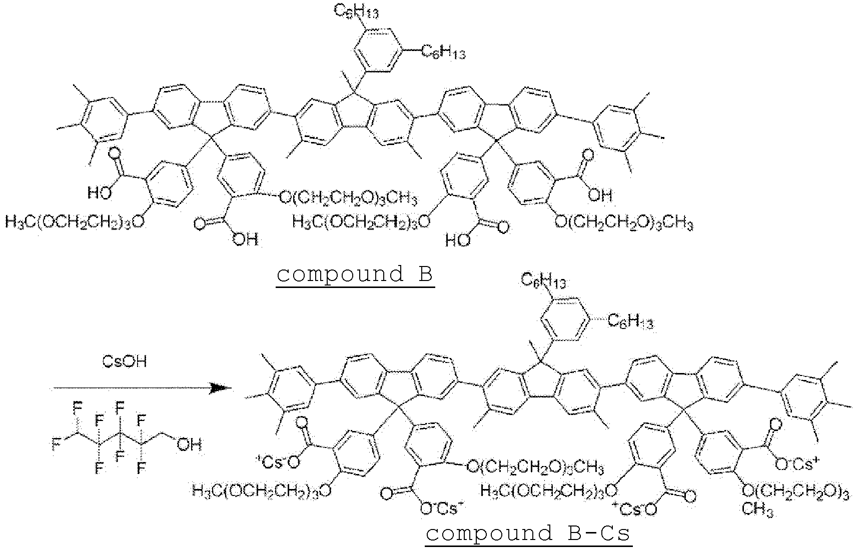

### <Example D3> Fabrication of light emitting device D3

A light emitting device D3 was fabricated in the same manner as in Example D1, except that an electron transporting layer was formed using a 1H,1H,5H-octafluoropentanol solution of a compound C-Cs prepared by dissolving the compound C at a concentration of 0.25% by weight and cesium hydroxide mono-hydrate at a concentration of 0.09% by weight in 1H,1H,5H-octafluoropentanol and stirring the solution at room temperature for 1 hour, instead of the 0.25% by weight 1H,1H,5H-octafluoropentanol solution of the compound A-Cs. The compound C-Cs is estimated to have the following structure.

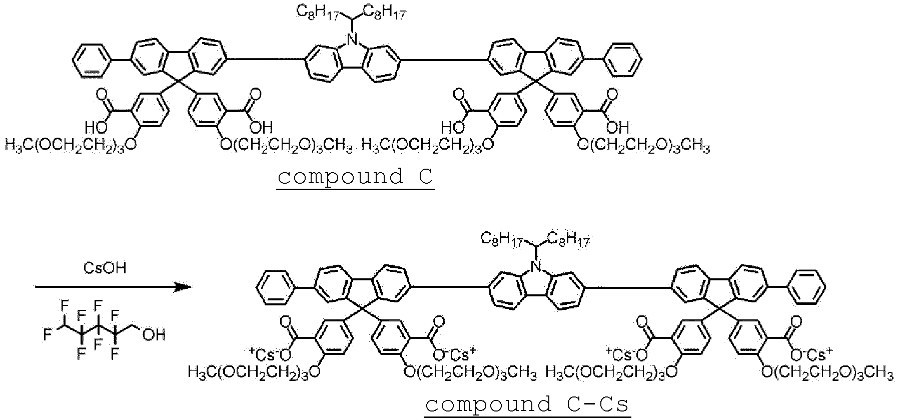

### <Example D4> Fabrication of light emitting device D4

A light emitting device D4 was fabricated in the same manner as in Example D1, except that an electron transporting layer was formed using a 1H,1H,5H-octafluoropentanol solution of a compound D-Cs prepared by dissolving the compound D at a concentration of 0.25% by weight and cesium hydroxide mono-hydrate at a concentration of 0.07% by weight in 1H,1H,5H-octafluoropentanol and stirring the solution at room temperature for 1 hour, instead of the 0.25% by weight 1H,1H,5H-octafluoropentanol solution of the compound A-Cs. The compound D-Cs is estimated to have the following structure.

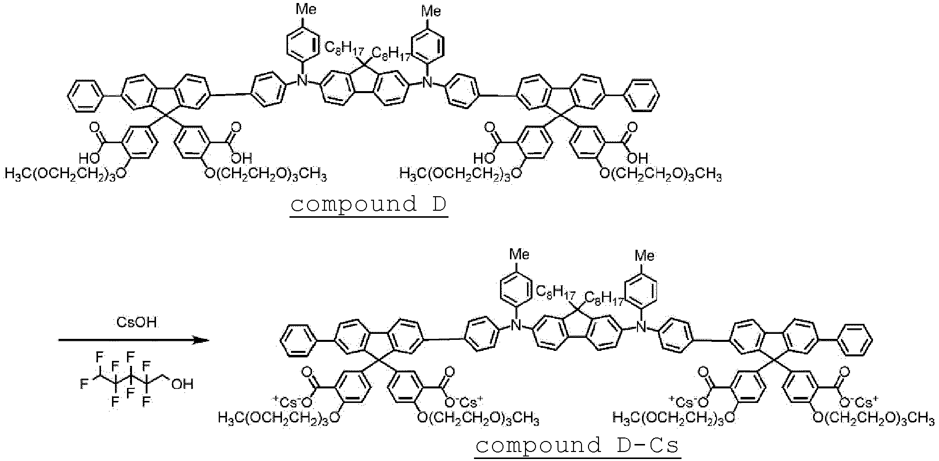

### <Example D5> Fabrication of light emitting device D5

A light emitting device D5 was fabricated in the same manner as in Example D1, except that an electron transporting layer was formed using a 1H,1H,5H-octafluoropentanol solution of a compound E-Cs prepared by dissolving the compound E at a concentration of 0.25% by weight and cesium hydroxide mono-hydrate at a concentration of 0.08% by weight in 1H,1H,5H-octafluoropentanol and stirring the solution at room temperature for 1 hour, instead of the 0.25% by weight 1H,1H,5H-octafluoropentanol solution of the compound A-Cs. The compound E-Cs is estimated to have the following structure.

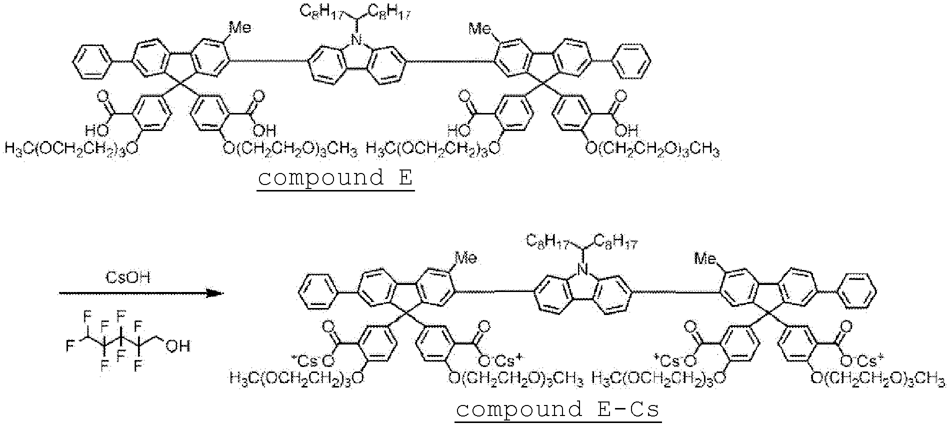

### <Example D6> Fabrication of light emitting device D6

A light emitting device D6 was fabricated in the same manner as in Example D1, except that an electron transporting layer was formed using a 1H,1H,5H-octafluoropentanol solution of a compound F-Cs prepared by dissolving the compound F at a concentration of 0.25% by weight and cesium hydroxide mono-hydrate at a concentration of 0.08% by weight in 1H,1H,5H-octafluoropentanol and stirring the solution at room temperature for 1 hour, instead of the 0.25% by weight 1H,1H,5H-octafluoropentanol solution of the compound A-Cs. The compound F-Cs is estimated to have the following structure.

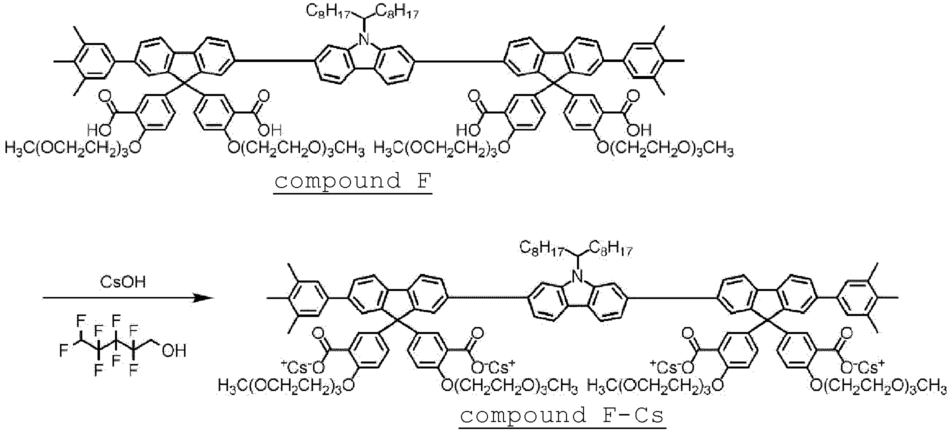

### <Example D7> Fabrication of light emitting device D7

A light emitting device D7 was fabricated in the same manner as in Example D1, except that an electron transporting layer was formed using a 1H,1H,5H-octafluoropentanol solution of a compound G-Cs prepared by dissolving the compound G at a concentration of 0.25% by weight and cesium hydroxide mono-hydrate at a concentration of 0.11% by weight in 1H,1H,5H-octafluoropentanol and stirring the solution at room temperature for 1 hour, instead of the 0.25% by weight 1H,1H,5H-octafluoropentanol solution of the compound A-Cs. The compound G-Cs is estimated to have the following structure.

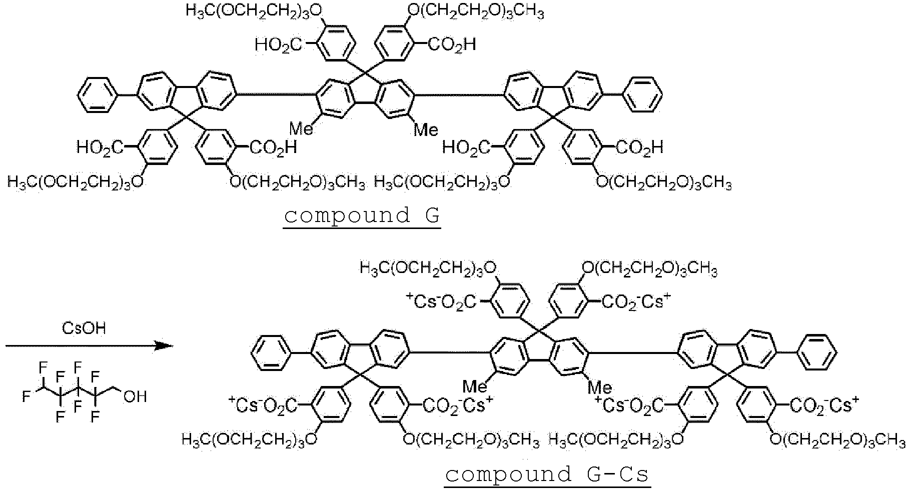

### <Example D8> Fabrication of light emitting device D8

A light emitting device D8 was fabricated in the same manner as in Example D1, except that an electron transporting layer was formed using a 1H, 1H, 5H-octafluoropentanol solution of a compound I-Cs prepared by dissolving the compound I at a concentration of 0.25% by weight and cesium hydroxide mono-hydrate at a concentration of 0.08% by weight in 1H,1H,5H-octafluoropentanol and stirring the solution at room temperature for 1 hour, instead of the 0.25% by weight 1H,1H,5H-octafluoropentanol solution of the compound A-Cs. The compound I-Cs is estimated to have the following structure.

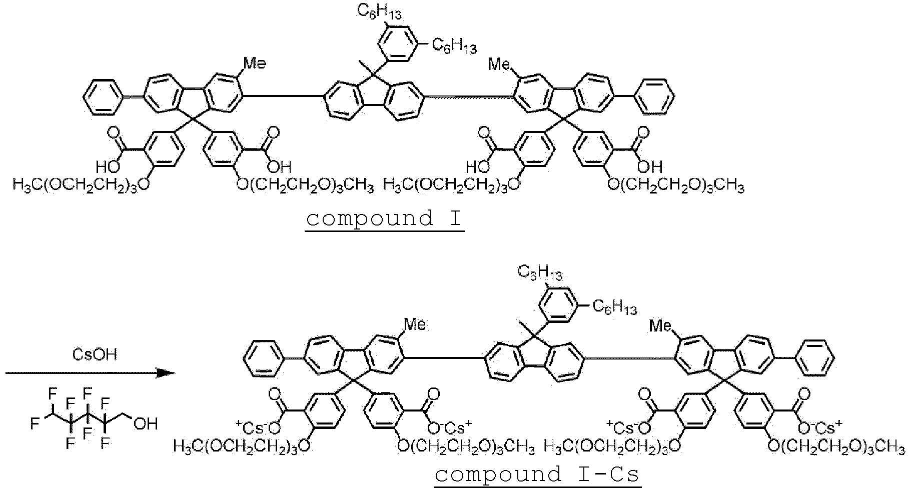

### <Example D9> Fabrication of light emitting device D9

A light emitting device D9 was fabricated in the same manner as in Example D1, except that an electron transporting layer was formed using a 1H,1H,5H-octafluoropentanol solution of a compound L-Cs prepared by dissolving the compound L at a concentration of 0.25% by weight and cesium hydroxide mono-hydrate at a concentration of 0.09% by weight in 1H,1H,5H-octafluoropentanol and stirring the solution at room temperature for 1 hour, instead of the 0.25% by weight 1H,1H,5H-octafluoropentanol solution of the compound A-Cs. The compound L-Cs is estimated to have the following structure.

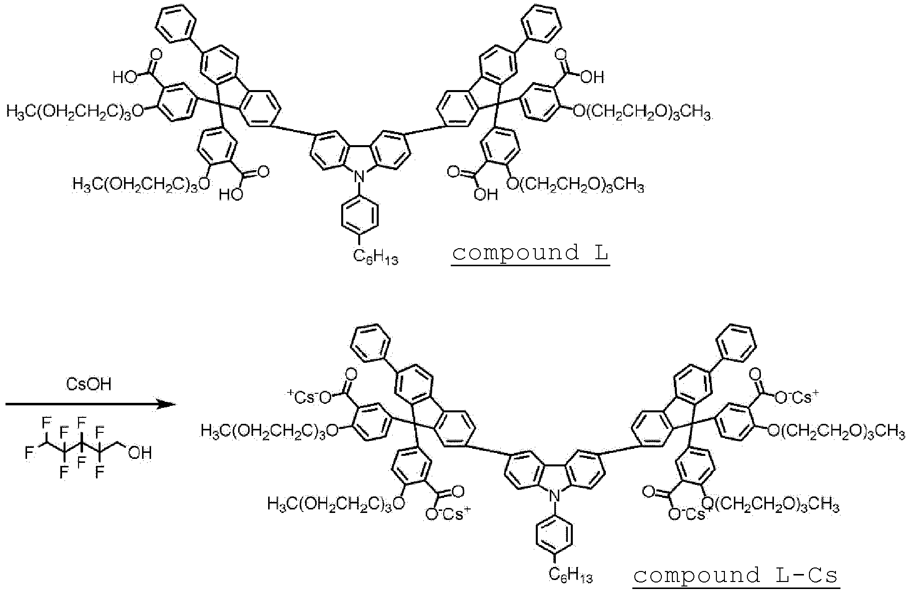

### <Example D10> Fabrication of light emitting device D10

A light emitting device D10 was fabricated in the same manner as in Example D1, except that an electron transporting layer was formed using a 1H,1H,5H-octafluoropentanol solution of a compound M-Cs prepared by dissolving the compound M at a concentration of 0.25% by weight and cesium hydroxide mono-hydrate at a concentration of 0.08% by weight in 1H,1H,5H-octafluoropentanol and stirring the solution at room temperature for 1 hour, instead of the 0.25% by weight 1H, 1H, 5H-octafluoropentanol solution of the compound A-Cs. The compound M-Cs is estimated to have the following structure.

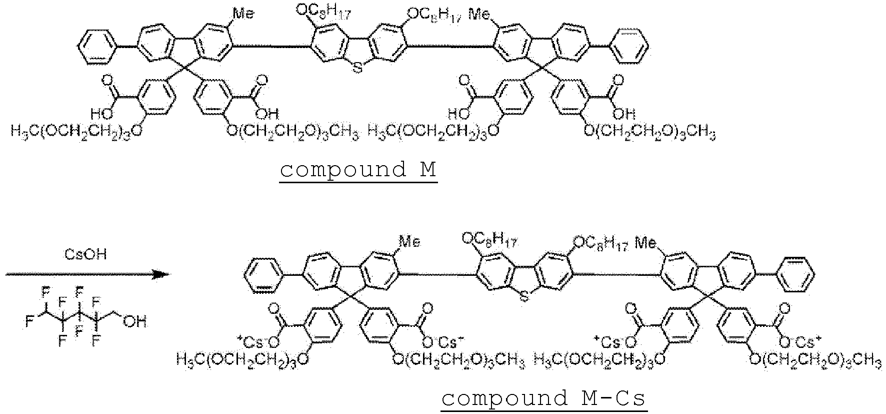

### <Example D11> Fabrication of light emitting device D11

A light emitting device D11 was fabricated in the same manner as in Example D1, except that an electron transporting layer was formed using a 1H,1H,5H-octafluoropentanol solution of a compound N-Cs prepared by dissolving the compound N at a concentration of 0.25% by weight and cesium hydroxide mono-hydrate at a concentration of 0.08% by weight in 1H,1H,5H-octafluoropentanol and stirring the solution at room temperature for 1 hour, instead of the 0.25% by weight 1H,1H,5H-octafluoropentanol solution of the compound A-Cs. The compound N-Cs is estimated to have the following structure.

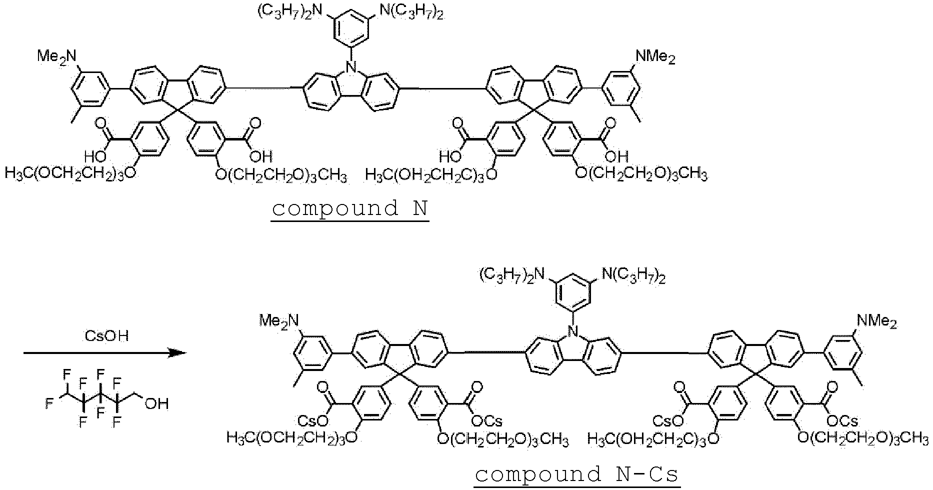

### <Example D12> Fabrication of light emitting device D12

A light emitting device D12 was fabricated in the same manner as in Example D1, except that an electron transporting layer was formed using a 1H,1H,5H-octafluoropentanol solution of a compound H-Cs prepared by dissolving the compound N at a concentration of 0.25% by weight and cesium hydroxide mono-hydrate at a concentration of 0.08% by weight in 1H,1H,5H-octafluoropentanol and stirring the solution at room temperature for 1 hour, instead of the 0.25% by weight 1H,1H,5H-octafluoropentanol solution of the compound A-Cs. The compound H-Cs is estimated to have the following structure.

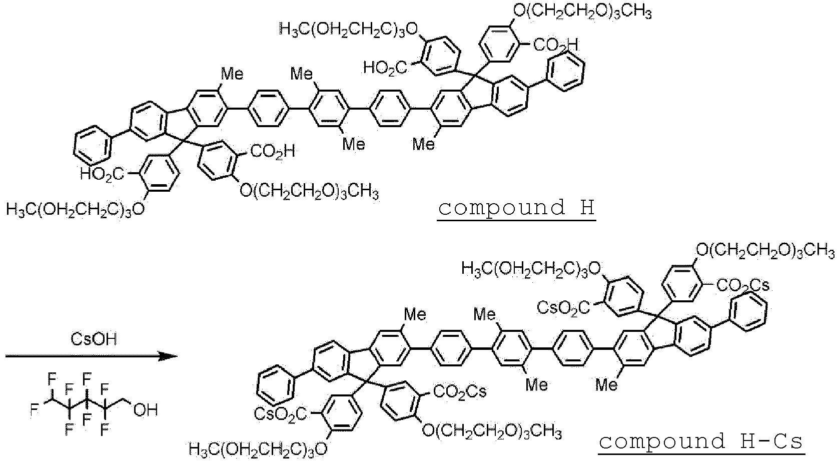

### <Example D13> Fabrication of light emitting device D13

A light emitting device D13 was fabricated in the same manner as in Example D1, except that an electron transporting layer was formed using a 1H,1H,5H-octafluoropentanol solution of a compound O-Cs prepared by dissolving the compound N at a concentration of 0.25% by weight and cesium hydroxide mono-hydrate at a concentration of 0.08% by weight in 1H,1H,5H-octafluoropentanol and stirring the solution at room temperature for 1 hour, instead of the 0.25% by weight 1H,1H,5H-octafluoropentanol solution of the compound A-Cs. The compound O-Cs is estimated to have the following structure.

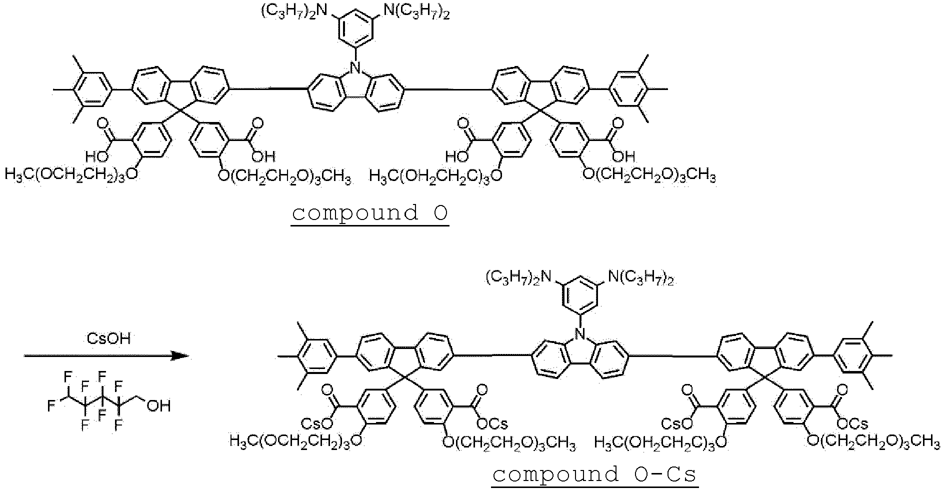

### <Example D14> Fabrication of light emitting device D14

A light emitting device D14 was fabricated in the same manner as in Example D1, except that an electron transporting layer was formed using the compound P-Cs instead of the compound A-Cs.

### <Comparative Example CD1> Fabrication of light emitting device CD1

A light emitting device CD1 was fabricated in the same manner as in Example D1, except that an electron transporting layer was formed using a 1H,1H,5H-octafluoropentanol solution of a compound Z-Cs prepared by dissolving the compound Z at a concentration of 0.25% by weight and cesium hydroxide mono-hydrate at a concentration of 0.09% by weight in 1H,1H,5H-octafluoropentanol and stirring the solution at room temperature for 1 hour, instead of the 0.25% by weight 1H,1H,5H-octafluoropentanol solution of the compound A-Cs. The compound Z-Cs is estimated to have the following structure.

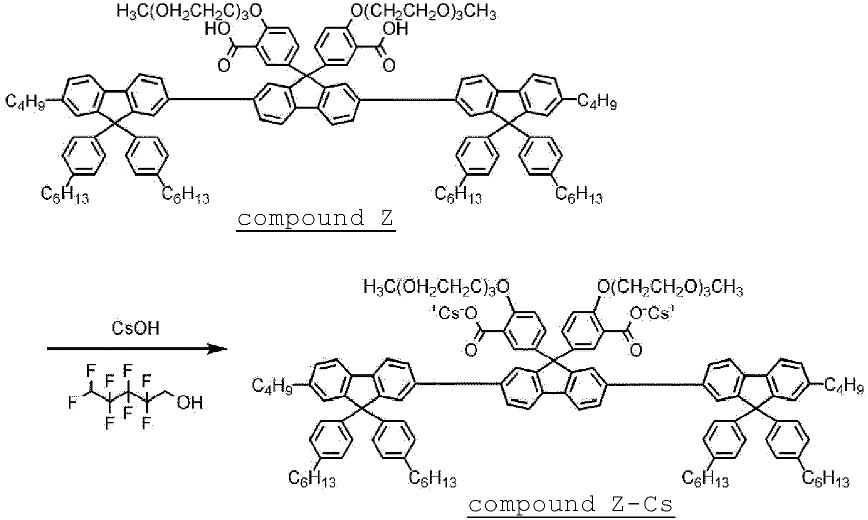

### <Comparative Example CD2> Fabrication of light emitting device CD2

A light emitting device CD2 was fabricated in the same manner as in Example 1, except that the polymer compound PA was used instead of the compound A-Cs.

### <Comparative Example CD3> Fabrication of light emitting device CD3

A light emitting device CD3 was fabricated in the same manner as in Example 1, except that the polymer compound PB was used instead of the compound A-Cs.

### <Comparative Example CD4> Fabrication of light emitting device CD4

A light emitting device CD4 was fabricated in the same manner as in Example 1, except that the polymer compound PC was used instead of the compound A-Cs.

### <Comparative Example CD5> Fabrication of light emitting device CD5

A light emitting device CD5 was fabricated in the same manner as in Example 1, except that the polymer compound PD was used instead of the compound A-Cs.

### <Comparative Example CD6> Fabrication of light emitting device CD6

A light emitting device CD6 was fabricated in the same manner as in Example 1, except that an electron transporting layer was formed using a 1H,1H,5H-octafluoropentanol solution of a compound Y-Cs prepared by dissolving the compound Y at a concentration of 0.25% by weight and cesium hydroxide mono-hydrate at a concentration of 0.09% by weight in 1H,1H,5H-octafluoropentanol and stirring the solution at room temperature for 1 hour, instead of the 0.25% by weight 1H,1H,5H-octafluoropentanol solution of the compound A-Cs. The compound Y-Cs is estimated to have the following structure.

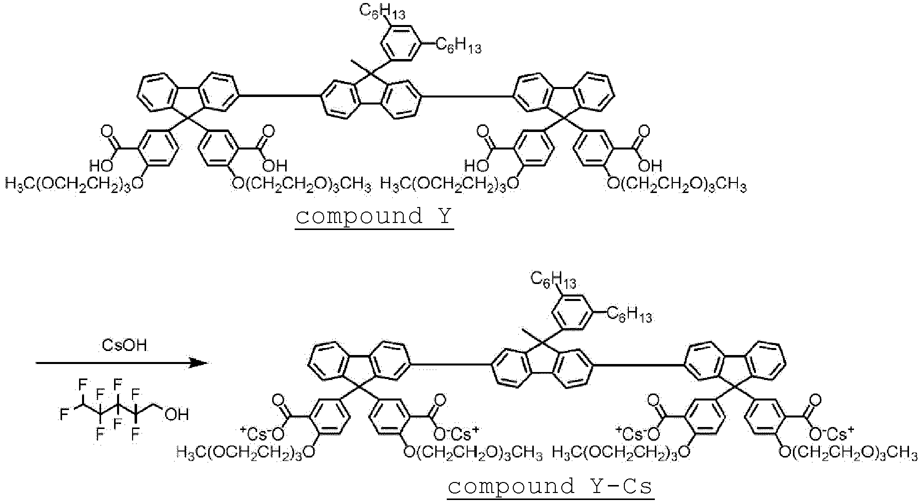

### (Measurement of luminance life of light emitting device)

For the light emitting devices D1 to D11 and CD1 to CD6, the time until the luminance reached 80% of the initial luminance set at 6000 cd/m² was measured. The relative value when the luminance life of the light emitting device CD2 was taken as 1.0 was determined. The results are shown in Table 1.

**[Table 1]**

| | light emitting device | electron transporting layer | luminance life (relative value) |
|---|---|---|---|
| Example D1 | D1 | compound A-Cs | 1.7 |
| Example D2 | D2 | compound B-Cs | 1.6 |
| Example D3 | D3 | compound C-Cs | 1.6 |
| Example D4 | D4 | compound D-Cs | 1.5 |
| Example D5 | D5 | compound E-Cs | 1.7 |
| Example D6 | D6 | compound F-Cs | 1.7 |
| Example D7 | D7 | compound G-Cs | 1.5 |
| Example D8 | D8 | compound I-Cs | 1.8 |
| Example D9 | D9 | compound L-Cs | 1.7 |
| Example D10 | D10 | compound M-Cs | 1.8 |
| Example D11 | D11 | compound N-Cs | 1.6 |
| Comparative Example CD1 | CD1 | compound Z-Cs | 0.9 |
| Comparative Example CD2 | CD2 | polymer compound PA | 1.0 |
| Comparative Example CD3 | CD3 | polymer compound PB | 1.0 |
| Comparative Example CD4 | CD4 | polymer compound PC | 1.2 |
| Comparative Example CD5 | CD5 | polymer compound PD | 0.9 |
| Comparative Example CD6 | CD6 | compound Y-Cs | 1.2 |

For the light emitting devices D12 to D14 and CD2, the time until the luminance reached 70% of the initial luminance set at 6000 cd/m² was measured. The relative value when the luminance life of the light emitting device CD2 was taken as 1.0 was determined. The results are shown in Table 2.

**[Table 2]**

| | light emitting device | electron transporting layer | luminance life (relative value) |
|---|---|---|---|
| Example D12 | D12 | compound H-Cs | 1.6 |
| Example D13 | D13 | compound O-Cs | 1.5 |
| Example D14 | D14 | compound P-Cs | 1.6 |
| Comparative Example CD2 | CD2 | polymer compound PA | 1.0 |

As understood from Table 1 and Table 2, a light emitting device containing the compound of the present invention is excellent in luminance life as compared with a light emitting device not containing the compound of the present invention.

### Industrial Applicability

According to the present invention, it is possible to provide a compound which gives a light emitting device excellent in luminance life, a composition containing the compound and a light emitting device containing the same, and an intermediate compound which is useful for production of the compound.

## Claims

1. A compound represented by the formula (1): 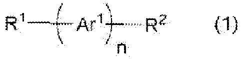 wherein,
n represents an integer of 2 or more and 20 or less,
R¹ and R² each independently represent a hydrogen atom, an alkyl group, a cycloalkyl group, an alkoxy group, a cycloalkoxy group or a halogen atom, and these groups optionally have a substituent,
Ar¹ represents a mono-cyclic or condensed-cyclic arylene group, a mono-cyclic or condensed-cyclic divalent heterocyclic group or a group represented by - N(R^{X1})-, and these groups optionally have a substituent, a plurality of Ar¹ may be the same or different, R^{X1} represents a hydrogen atom, an alkyl group, a cycloalkyl group, an aryl group or a monovalent heterocyclic group, and these groups optionally have a substituent,
at least two of Ar¹ represent groups represented by the formula (2), a plurality of the groups represented by the formula (2) may be the same or different, 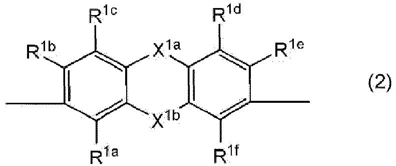
wherein,
X^{1a} and X^{1b} each independently represent a single bond, an oxygen atom, a sulfur atom, a group represented by -S(=O)-, a group represented by -S(=O)₂-, a group represented by -C(=O)-, a group represented by -C(R^{1g})₂-, a group represented by -Si(R^{1g})₂-, a group represented by -NR^{1g}- or a group represented by C(R^{1g})₂-C(R^{1g})₂-, at least one of X^{1a} and X^{1b} represents a group represented by -C(R^{1g})₂- or a group represented by -NR^{1g}-,
R^{1g} represents a hydrogen atom, an alkyl group, a cycloalkyl group, an aryl group or a monovalent heterocyclic group, and these groups optionally have a substituent, when a plurality of R^{1g} are present, they may be the same or different and may be combined together to form a ring,
R^{1a}, R^{1b}, R^{1c}, R^{1d}, R^{1e} and R^{1f} each independently represent a hydrogen atom, an alkyl group, a cycloalkyl group, an alkoxy group, a cycloalkoxy group, an aryl group, an aryloxy group, a monovalent heterocyclic group, an amino group or a halogen atom, and these groups optionally have a substituent, R^{1a} and R^{1g}, R^{1b} and R^{1c}, R^{1c} and R^{1g}, R^{1g} and R^{1d}, R^{1d} and R^{1e}, and R^{1f} and R^{1g} each may be combined together to form a ring together with atoms to which they are attached,
in at least two groups represented by said formula (2) in said formula (1), at least one of R^{1g} is a group represented by the formula (2-1) or a group represented by the formula (2-2),
in said formula (1), if all Ar¹ are groups represented by said formula (2) and all X^{1a} in all the groups represented by the formula (2) are single bonds and all X^{1b} are groups represented by -C(R^{1g})₂-, then, at least one of R^{1a}, R^{1b}, R^{1e} and R^{1f} represents at least one group selected from the group consisting of an alkyl group, a cycloalkyl group, an alkoxy group, a cycloalkoxy group, an aryl group, an aryloxy group, a monovalent heterocyclic group, an amino group and a halogen atom (the alkyl group, the cycloalkyl group, the alkoxy group, the cycloalkoxy group, the aryl group, the aryloxy group, the monovalent heterocyclic group or the amino group optionally has a substituent), in at least one of all the groups represented by the formula (2),
—R³—{(Q¹)ₙ₁-Y¹(M¹)ₐ₁(Z¹)_{b1}}ₘ₂ (2-1)
wherein,
R³ represents an aromatic hydrocarbon group or a heterocyclic group, and these groups optionally have a substituent,
n1, a1 and b1 each independently represent an integer of 0 or more, m2 represents an integer of 1 or more, and a1 and b1 are selected so that the charge of the group represented by said formula (2-1) is 0, when a plurality of n1, a1 and b1 are present, they may be the same or different at each occurrence,
Q¹ represents an alkylene group, a cycloalkylene group, an arylene group, an oxygen atom or a sulfur atom, and these groups optionally have a substituent, when a plurality of Q¹ are present, they may be the same or different,
Y¹ represents -CO₂⁻, -SO₃⁻, -SO₂⁻, -PO₃²⁻, -CO₂Y¹', -SO₃Y¹', -SO₂Y¹', -P(=O)(-OY¹')(-O⁻) or -P(=O)(-OY¹')₂, when a plurality of Y¹ are present, they may be the same or different, Y¹' represents a hydrocarbon group optionally having a substituent, a heterocyclic group optionally having a substituent, or a hydrogen atom, when Y¹ is -CO₂Y¹', -SO₃Y¹', -SO₂Y¹' or -P(=O)(-OY¹')₂, the subscript a1 for M¹ directly bonded to the Y¹ is 0 and the subscript b1 for Z¹ directly bonded to the M¹ is 0, when Y¹ is -CO₂⁻, -SO₃⁻, -SO₂⁻, -PO₃²⁻ or - P(=O)(-OY¹')(-O⁻), the subscript a1 for M¹ directly bonded to the Y¹ is an integer of 1 or more, when a plurality of Y¹' are present, they may be the same or different,
M¹ represents an alkali metal cation, an alkaline earth metal cation or an ammonium cation, and this ammonium cation optionally has substituent, when a plurality of M¹ are present, they may be the same or different,
Z¹ represents F⁻, Cl⁻, Br⁻, I⁻, OH⁻, B (R^{a})₄⁻, R^{a}SO₃⁻, R^{a}COO⁻, NO₃⁻, SO₄²⁻, HSO₄⁻, PO₄³⁻, HPO₄²⁻, H₂PO₄⁻, BF₄⁻ or PF₆⁻, R^{a} represents an alkyl group, a cycloalkyl group or an aryl group, and these groups optionally have a substituent, when a plurality of Z¹ are present, they may be the same or different,
—R⁴—{(Q²)ₙ₂-Y²(M²)ₐ₂(Z²)_{b2}}ₘ₃ (2-2)
wherein,
n2 and b2 each independently represent an integer of 0 or more and a2 and m3 each independently represent an integer of 1 or more, but a2 and b2 are selected so that the charge of the group represented by said formula (2-2) is 0, when a plurality of n2, a2 and b2 are present, they may be the same or different at each occurrence,
R⁴ represents an aromatic hydrocarbon group or a heterocyclic group, and these groups optionally have a substituent,
Q² represents an alkylene group, a cycloalkylene group, an arylene group, an oxygen atom or a sulfur atom, and these groups optionally have a substituent, when a plurality of Q² are present, they may be the same or different,
Y² represents -C⁺R^{c}₂, -N⁺R^{c}₃, -P⁺R^{c}₃, -S⁺R^{c}₂ or - I⁺R^{c}₂, R^{c} represents an alkyl group, a cycloalkyl group or an aryl group, and these groups optionally have a substituent, a plurality of R^{c} may be the same or different, when a plurality of Y² are present, they may be the same or different,
M² represents F⁻, Cl⁻, Br⁻, I⁻, OH⁻, B (R^{b})₄⁻, R^{b}SO₃⁻, R^{b}COO⁻, BF₄⁻, SbCl₆⁻ or SbF₆⁻, R^{b} represents an alkyl group, a cycloalkyl group or an aryl group, and these groups optionally have a substituent, when a plurality of R^{b} are present, they may be the same or different, when a plurality of M² are present, they may be the same or different,
Z² represents an alkali metal cation or an alkaline earth metal cation, when a plurality of Z² are present, they may be the same or different.

2. The compound according to Claim 1, wherein at least one of said Ar¹ is a mono-cyclic or condensed-cyclic arylene group other than a group represented by said formula (2), a mono-cyclic or condensed-cyclic divalent heterocyclic group other than a group represented by said formula (2) or a group represented by -N(R^{X1})- (R^{X1} represents the same meaning as described above).

3. The compound according to Claim 1 or 2, wherein the compound represented by said formula (1) is a compound represented by the formula (1-1): 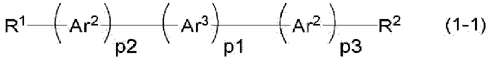 wherein,
p1 represents an integer of 2 or more, p2 and p3 each independently represent an integer of 1 or more, the sum of p1, p2 and p3 is 4 or more and 20 or less,
Ar² represents a mono-cyclic or condensed-cyclic arylene group other than a group represented by said formula (2) or a mono-cyclic or condensed-cyclic divalent heterocyclic group other than a group represented by said formula (2), and these groups optionally have a substituent, a plurality of Ar² may be the same or different,
Ar³ represents a mono-cyclic or condensed-cyclic arylene group, a mono-cyclic or condensed-cyclic divalent heterocyclic group or a group represented by - N(R^{X1})-, and these groups optionally have a substituent, a plurality of Ar³ may be the same or different,
at least two of Ar³ are groups represented by said formula (2),
R¹, R² and R^{x1} represent the same meaning as described above.

4. The compound according to Claim 3, wherein the compound represented by said formula (1-1) is a compound represented by the formula (1A): 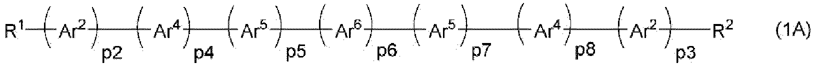 wherein,
Ar², p2 and p3 represent the same meaning as described above,
p4 and p8 each independently represent an integer of 1 or more, p5, p6 and p7 each independently represent an integer of 0 or more, the sum of p2, p3, p4, p5, p6, p7 and p8 is an integer of 4 or more and 20 or less,
Ar⁴ represents a group represented by the formula (2A), a plurality of Ar⁴ may be the same or different,
Ar⁶ represents a group represented by said formula (2), when a plurality of Ar⁶ are present, they may be the same or different,
Ar⁵ represents a mono-cyclic or condensed-cyclic arylene group other than a group represented by said formula (2), a mono-cyclic or condensed-cyclic divalent heterocyclic group other than a group represented by said formula (2) or a group represented by -N(R^{X1})-, and these groups optionally have a substituent, when a plurality of Ar⁶ are present, they may be the same or different, R¹, R² and R^{X1} represent the same meaning as described above, 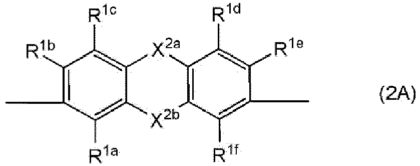
wherein,
X^{2a} and X^{2b} each independently represent a single bond, a group represented by -C(R^{1g})₂- or a group represented by -NR^{1g}-, one of X^{2a} and X^{2b} is a single bond and the other of X^{2a} and X^{2b} is a group represented by -C(R^{1g})₂- or a group represented by - NR^{1g}-,
R^{1a}, R^{1b}, R^{1c}, R^{1d}, R^{1e}, R^{1f} and R^{1g} represent the same meaning as described above.

5. The compound according to Claim 4, wherein p6 is an integer of 1 or more, and in at least one Ar⁶, one of X^{1a} and X^{1b} in said formula (2) is a single bond.

6. The compound according to any one of Claims 1 to 5, wherein at least two groups represented by said formula (2) are groups represented by the formula (2A): 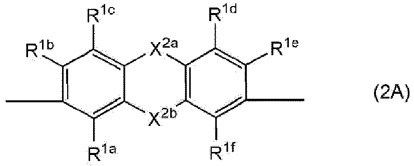 wherein,
X^{2a} and X^{2b} each independently represent a single bond, a group represented by -C(R^{1g})₂- or a group represented by -NR^{1g}-, one of X^{2a} and X^{2b} is a single bond and the other of X^{2a} and X^{2b} is a group represented by -C(R^{1g})₂- or a group represented by - NR^{1g}-,
R^{1a}, R^{1b}, R^{1c}, R^{1d}, R^{1e}, R^{1f} and R^{1g} represent the same meaning as described above.

7. The compound according to Claim 6, wherein at least two groups represented by said formula (2A) are groups represented by the formula (2A'): 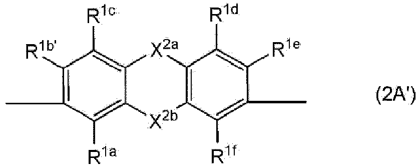 wherein,
R^{1a}, R^{1c}, R^{1d}, R^{1e}, R^{1f}, x^{2a} and X^{2b} represent the same meaning as described above,
R^{1b'} represents an alkyl group, a cycloalkyl group, an alkoxy group, a cycloalkoxy group, an aryl group, an aryloxy group, a monovalent heterocyclic group, an amino group or a halogen atom, and these groups optionally have a substituent, R^{1b'} and R^{1c} may be combined together to form a ring together with carbon atoms to which they are attached.

8. The compound according to Claim 6 or 7, wherein at least two of X^{2a} are single bonds.

9. The compound according to any one of Claims 1 to 8, wherein at least one of R^{1g} is a group represented by said formula (2-1).

10. The compound according to Claim 9, wherein the group represented by said formula (2-1) is a group represented by the formula (2-3): 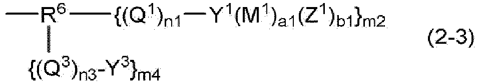 wherein,
n1, a1, b1, m2, Q¹, Y¹, M¹ and Z¹ represent the same meaning as described above,
n3 represents an integer of 0 or more, and m4 represents an integer of 1 or more, when a plurality of n3 are present, they may be the same or different,
R⁶ represents an aromatic hydrocarbon group or a heterocyclic group, and these groups optionally have a substituent,
Q³ represents an alkylene group, a cycloalkylene group, an arylene group, an oxygen atom or a sulfur atom, and these groups optionally have a substituent, when a plurality of Q³ are present, they may be the same or different,
Y³ represents a group represented by the formula (5) or the formula (6), when a plurality of Y³ are present, they may be the same or different,
-O- (R'O)ₐ₃-R" (5) 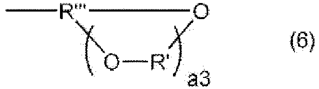
wherein,
a3 represents an integer of 1 or more,
R' represents an alkylene group, a cycloalkylene group or an arylene group, and these groups optionally have a substituent, when a plurality of R' are present, they may be the same or different,
R" represents a hydrogen atom, an alkyl group, a cycloalkyl group or an aryl group, and these groups optionally have a substituent,
R‴ represents a hydrocarbon group, and this hydrocarbon group optionally has a substituent.

11. The compound according to any one of Claims 1 to 10, wherein at least one of Ar¹ is a mono-cyclic or condensed-cyclic arylene group other than a group represented by said formula (2) or a mono-cyclic or condensed-cyclic divalent heterocyclic group other than a group represented by said formula (2), which is a group obtained by removing 2 hydrogen atoms constituting the ring from a benzene ring or an aromatic hydrocarbon ring in which only 2 or more and 10 or less benzene rings are condensed (the group optionally has a substituent) or a group represented by the formula (4): 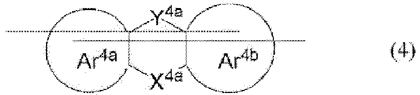 wherein,
Ar^{4a} and Ar^{4b} each independently represent an aromatic hydrocarbon group or a heterocyclic group, and these groups optionally have a substituent, when a plurality of said substituents are present, they may be combined together to form a ring together with atoms to which they are attached,
X^{4a} and Y^{4a} each independently represent a single bond, an oxygen atom, a sulfur atom, a group represented by -S(=O)-, a group represented by -S(=O)₂-, a group represented by -C(=O)-, a group represented by -SiR₂- or a group represented by -CR₂-CR₂-, R represents a hydrogen atom, an alkyl group, a cycloalkyl group, an aryl group or a monovalent heterocyclic group, and these groups optionally have a substituent, a plurality of R may be the same or different and may be combined together to form a ring,
the substituent which Ar^{4a} optionally has and R, and the substituent which Ar^{4b} optionally has and R each may be combined together to form a ring together with atoms to which they are attached.

12. The compound according to Claim 11, wherein the group represented by said formula (4) is a group represented by the formula (4A): 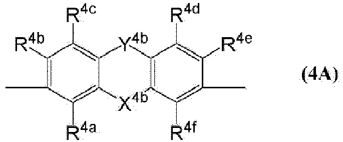 wherein,
X^{4b} and Y^{4b} each independently represent a single bond, an oxygen atom, a sulfur atom or a group represented by -CR₂-CR₂-, one of X^{4b} and Y^{4b} represents a single bond, R represents the same meaning as described above,
R^{4a}, R^{4b}, R^{4c}, R^{4d}, R^{4e} and R^{4f} each independently represent a hydrogen atom, an alkyl group, a cycloalkyl group, an alkoxy group, a cycloalkoxy group, an aryl group, an aryloxy group, a monovalent heterocyclic group, an amino group or a halogen atom, and these groups optionally have a substituent, R^{4b} and R^{4c}, R^{4c} and R, R^{4d} and R, R^{4a} and R, R^{4f} and R, and R^{4d} and R^{4e} each may be combined together to form a ring together with carbon atoms to which they are attached.

13. The compound according to any one of Claims 1 to 12, wherein in said formula (1), Ar¹ is composed only of groups selected from the group consisting of a group represented by said formula (2), a group obtained by removing 2 hydrogen atoms constituting the ring from a benzene ring or an aromatic hydrocarbon ring in which only 2 or more and 10 or less benzene rings are condensed (the group optionally has a substituent), a group represented by the formula (4), and a group represented by -N(R^{X1})- (R^{X1} represents the same meaning as described above): 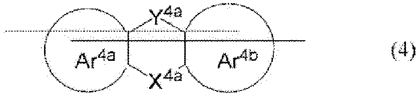 wherein,
Ar^{4a} and Ar^{4b} each independently represent an aromatic hydrocarbon group or a heterocyclic group, and these groups optionally have a substituent, when a plurality of said substituents are present, they may be combined together to form a ring together with atoms to which they are attached,
X^{4a} and Y^{4a} each independently represent a single bond, an oxygen atom, a sulfur atom, a group represented by -S(=O)-, a group represented by -S(=O)₂-, a group represented by -C(=O)-, a group represented by -SiR₂- or a group represented by -CR₂-CR₂-,
R represents a hydrogen atom, an alkyl group, a cycloalkyl group, an aryl group or a monovalent heterocyclic group, and these groups optionally have a substituent, a plurality of R may be the same or different and may be combined together to form a ring,
the substituent which Ar^{4a} optionally has and R, and the substituent which Ar^{4b} optionally has and R each may be combined together to form a ring together with atoms to which they are attached.

14. A composition comprising at least one compound selected from the group consisting of a hole transporting material, a hole injection material, an electron transporting material, an electron injection material, a light emitting material, an antioxidant and a solvent, and the compound according to any one of Claims 1 to 13.

15. A light emitting device comprising the compound according to any one of Claims 1 to 13.

16. A compound represented by the formula (11), the formula (12) or the formula (13): 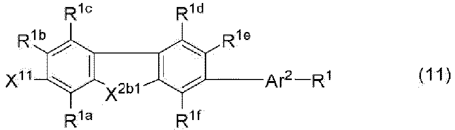 wherein,
R^{1a}, R^{1b}, R^{1c}, R^{1d}, R^{1e} and R^{1f} each independently represent a hydrogen atom, an alkyl group, a cycloalkyl group, an alkoxy group, a cycloalkoxy group, an aryl group, an aryloxy group, a monovalent heterocyclic group, an amino group or a halogen atom, and these groups optionally have a substituent, R^{1a} and R^{1g}, R^{1b} and R^{1c}, R^{1d} and R^{1e}, and R^{1f} and R^{1g} each may be combined together to form a ring structure together with atoms to which they are attached,
X^{2b1} represents a group represented by -C(R^{1g})₂- or a group represented by -NR^{1g}-,
R^{1g} represents a hydrogen atom, an alkyl group, a cycloalkyl group, an aryl group or a monovalent heterocyclic group, and these groups optionally have a substituent, when a plurality of R^{1g} are present, they may be the same or different and may be combined together to form a ring, at least one of R^{1g} is a group represented by the formula (2-1) or a group represented by the formula (2-2),
X¹¹ represents a halogen atom or B(OR^{C2})₂ (wherein, R^{C2} represents a hydrogen atom, an alkyl group, a cycloalkyl group or an aryl group, and these groups optionally have a substituent, a plurality of R^{C2} may be the same or different and may be combined together to form a ring structure together with oxygen atoms to which they are attached),
R¹ represents a hydrogen atom, an alkyl group, a cycloalkyl group, an alkoxy group, a cycloalkoxy group or a halogen atom, and these groups optionally have a substituent,
Ar² represents a mono-cyclic or condensed-cyclic arylene group other than the group represented by the formula (2) or a mono-cyclic or condensed-cyclic divalent heterocyclic group other than the group represented by the formula (2), and these groups optionally have a substituent,
—R³—{(Q¹)ₙ₁-Y¹M¹)ₐ₁(Z¹)_{b1}}ₘ₂ (2-1)
wherein,
R³ represents an aromatic hydrocarbon group or a heterocyclic group, and these groups optionally have a substituent,
n1, a1 and b1 each independently represent an integer of 0 or more, and m2 represents an integer of 1 or more, but a1 and b1 are selected so that the charge of the group represented by the formula (2-1) is 0, when a plurality of n1, a1 and b1 are present, they may be the same or different at each occurrence,
Q¹ represents an alkylene group, a cycloalkylene group, an arylene group, an oxygen atom or a sulfur atom, and these groups optionally have a substituent, when a plurality of Q¹ are present, they may be the same or different,
Y¹ represents -CO₂⁻, -SO₃⁻, -SO₂⁻, -PO₃²⁻, -CO₂Y¹', -SO₃Y¹', -SO₂Y¹', -P(=O)(-OY¹')(-O⁻) or -P(=O)(-OY¹')₂, when a plurality of Y¹ are present, they may be the same or different, Y¹' represents a hydrocarbon group optionally having a substituent, a heterocyclic group optionally having a substituent, or a hydrogen atom, when Y¹ is -CO₂Y¹', -SO₃Y¹', -SO₂Y¹' or -P(=O)(-OY¹')₂, the subscript a1 for M¹ directly bonded to the Y¹ is 0 and the subscript b1 for Z¹ directly bonded to the M¹ is 0, when Y¹ is -CO₂⁻, -SO₃⁻, -SO₂⁻, -PO₃²⁻ or - P(=O)(-OY¹')(-O⁻), the subscript a1 for M¹ directly bonded to the Y¹ is an integer of 1 or more, when a plurality of Y¹' are present, they may be the same or different,
M¹ represents an alkali metal cation, an alkaline earth metal cation or an ammonium cation, and this ammonium cation optionally has substituent, when a plurality of M¹ are present, they may be the same or different,
Z¹ represents F⁻, Cl⁻, Br⁻, I⁻, OH⁻, B (R^{a})₄⁻, R^{a}SO₃⁻, R^{a}COO⁻, NO₃⁻, SO₄²⁻, HSO₄⁻, PO₄³⁻, HPO₄²⁻, H₂PO₄⁻, BF₄⁻ or PF₆⁻, R^{a} represents an alkyl group, a cycloalkyl group or an aryl group, and these groups optionally have a substituent, when a plurality of Z¹ are present, they may be the same or different,
**―**R⁴―{(Q²)ₙ₂-Y²(M²)ₐ₂(Z²)_{b2}}ₘ₃ (2-2)
wherein,
n2 and b2 each independently represent an integer of 0 or more, and a2 and m3 each independently represent an integer of 1 or more, but a2 and b2 are selected so that the charge of the group represented by said formula (2-2) is 0, when a plurality of n2, a2 and b2 are present, they may be the same or different at each occurrence,
R⁴ represents an aromatic hydrocarbon group or a heterocyclic group, and these groups optionally have a substituent,
Q² represents an alkylene group, a cycloalkylene group, an arylene group, an oxygen atom or a sulfur atom, and these groups optionally have a substituent, when a plurality of Q² are present, they may be the same or different,
Y² represents -C⁺R^{c}₂, -N⁺R^{c}₃, -P⁺R^{c}₃, -S⁺R^{c}₂ or - I⁺R^{c}₂, R^{c} represents an alkyl group, a cycloalkyl group or an aryl group, and these groups optionally have a substituent, a plurality of R^{c} may be the same or different, when a plurality of Y² are present, they may be the same or different,
M² represents F⁻, Cl⁻, Br⁻, I⁻, OH⁻, B (R^{b})₄⁻, R^{b}SO₃⁻, R^{b}COO⁻, BF₄⁻, SbCl₆⁻ or SbF₆⁻, R^{b} represents an alkyl group, a cycloalkyl group or an aryl group, and these groups optionally have a substituent, when a plurality of R^{b} are present, they may be the same or different, when a plurality of M² are present, they may be the same or different,
Z² represents an alkali metal cation or an alkaline earth metal cation, when a plurality of Z² are present, they may be the same or different, 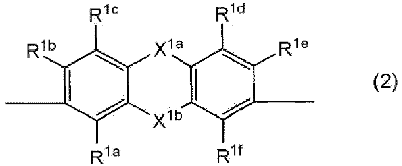
wherein,
X^{1a} and X^{1b} each independently represent a single bond, an oxygen atom, a sulfur atom, a group represented by -S(=O)-, a group represented by -S(=O)₂-, a group represented by -C(=O)-, a group represented by -C(R^{1g})₂-, a group represented by -Si(R^{1g})₂-, a group represented by -NR^{1g}- or a group represented by C(R^{1g})₂-C(R^{1g})₂-, at least one of X^{1a} and X^{1b} represents a group represented by -C(R^{1g})₂- or a group represented by -NR^{1g}-,
R^{1a}, R^{1b}, R^{1c}, R^{1d}, R^{1e}, R^{1f} and R^{1g} represent the same meaning as described above, 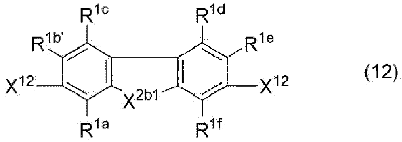
wherein,
R^{1a}, R^{1c}, R^{1d}, R^{1e}, R^{1f} and X^{2b1} represent the same meaning as described above,
R^{1b}' represents an alkyl group, a cycloalkyl group, an alkoxy group, a cycloalkoxy group, an aryl group, an aryloxy group, a monovalent heterocyclic group, an amino group or a halogen atom, and these groups optionally have a substituent, R^{1b}' and R^{1c} each may be combined together to form a ring structure together with atoms to which they are attached,
X¹² represents a halogen atom or a group represented by B(OR^{C2})₂ (wherein, R^{C2} represents the same meaning as described above), a plurality of X¹² may be the same or different, 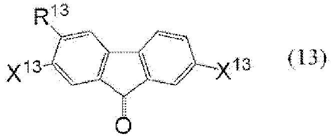
wherein,
R¹³ represents an alkyl group, a cycloalkyl group, an aryl group or a monovalent heterocyclic group, and these groups optionally have a substituent,
X¹³ represents a chlorine atom, a bromine atom, an iodine atom or a group represented by B(OR^{C2})₂ (wherein, R^{C2} represents the same meaning as described above, ), a plurality of X¹³ may be the same or different.
